# EUROPEAN PATENT SPECIFICATION

(11) **EP 3 935 047 B1**
(45) Date of publication and mention of the grant of the patent: **07.08.2024**
(21) Application number: 20707669.6
(22) Date of filing: 06.03.2020
(51) Int. Cl.: C07D 215/46, C07D 471/04

(54) **PROCESS FOR THE PREPARATION OF A SUBSTITUTED IMIDAZOQUINOLINE**
VERFAHREN ZUR HERSTELLUNG EINES SUBSTITUIERTEN IMIDAZOCHINOLINS
PROCÉDÉ DE PRÉPARATION D'UNE IMIDAZOQUINOLINE SUBSTITUÉE

(30) Priority: 07.03.2019 WO PCT/EP2019/055793
(43) Date of publication of application: 12.01.2022
(73) Proprietor: BioNTech SE, 55131 Mainz (DE)
(72) Inventor: HENRY, Christophe, 55131 Mainz (DE)
(74) Representative: Müller, Christian Stefan Gerd
(86) International application number: PCT/EP2020/056047
(87) International publication number: WO 2020/178434

(56) References cited:
- WO-A1-2019/048036
- WO-A1-2019/048353
- WO-A2-2009/118296

## Description

### Field of the Invention

The present invention relates to a process for synthesizing N-(4-(4-amino-2-(2-methoxyethyl)-1H-imidazo[4,5-c]quinolin-1-yl)butyl)-N-(tetrahydro-2H-pyran-4-yl)acetamide, an imidazoquinoline derivative useful as toll-like receptor agonist, in particular as an agonist of TLR7, which promotes induction of certain cytokines. Furthermore, the present invention also provides intermediates useful in the synthesis of N-(4-(4-amino-2-(2-methoxyethyl)-1H-imidazo[4,5-c]quinolin-l-yl)butyl)-N-(tetrahydro-2H-pyran-4-yl)acetamide.

### Background of the invention

The present disclosure relates generally to the field of organic synthetic methodology for the preparation of Toll-like receptor 7 agonists and their synthetic intermediates. Toll-like receptors (TLR) currently comprising a gene family of 13 receptors with different specificities, 11 of them found in humans, are part of the cellular pathogen pattern recognition system, which has evolved for defense against a variety of infections (bacteria, virus, fungi). Activation of TLRs leads to cytokine responses, e.g. with release of interferons and activation of specified immune cells. The functional expression of selected TLRs in tissues is highly different. Part of the receptors are located at the cell surface such as TLR4 (stimulated by *E.coli* lipopolysaccharide LPS), e.g. on epithelial cells, or TLR3, 7, 8 and 9 located at endosomal membranes in specified immune cells. The latter are all activated by nucleic acids, but recognize various types of them. For instance, TLR9 is activated by single-stranded DNA containing CpG subsequences, TLR7 and 8 are activated by single-stranded RNA, and TLR3 is activated by double-stranded RNA.

Some small-molecule (SMOL) TLR7 or TLR8 agonists have been identified. Those agonists can be grouped into purine-like molecules, such as 7-thia-8-oxoguanosine (TOG, isatoribine) or the imidazoquinoline-based compounds like imiquimod. Imiquimod is so far the only approved definitive TLR7 agonist, marketed as 5% cream (Aldara^{®}). It generates approx. 80% 5-year clearance of superficial basal cell carcinomas, which is the most frequent cancer worldwide. Imiquimod activates TLR7. The functional expression of TLR7 appears to be restricted to specific immune cells, i.e. in humans plasmacytoid dendritic cells, B-cells and probably eosinophils are known to be activated by TLR7 agonists.

For several years, strong efforts have been ongoing worldwide trying to exploit the strong immune activation induced by TLR7, 8 or 9 agonists for the treatment of cancer. Cancer immunotherapy, however, experienced a long history of failures. In recent years, though, the knowledge on cancer immune surveillance and the function of subsets of immune cells thereby has improved drastically. TLR7, TLR8 or TLR9 agonists are in clinical development for cancer mono- or combination therapies, or as vaccine adjuvants.

The TLR agonist approach for cancer immunotherapy is different from earlier efforts using, e.g. cytokines, interferons or monovalent vaccinations. TLR agonist mediated immune activation is pleiotropic via specified immune cells (primarily dendritic cells and B-cells, subsequently other cells), which generates an innate and adaptive immune response. Moreover, not only one interferon is induced, but rather the many different isoforms altogether, and not only type I (alpha, beta), but also (indirectly) type II (gamma) interferon. At least for local application, Aldara^{®} has delivered a remarkable proof-of-concept. This demonstrates that antigens are released by tumors, and that immune therapy in principle can work for cancer indications, even also in monotherapy. For a systemic administration route, though, the clinical proof-of-concept is pending for TLR7 agonists. For advanced cancers and systemic application (particularly s.c. or i.v. administration route) it appears to be clear that such TLR agonists might provide stronger, i.e. synergistic, efficacy in combination with other therapeutic interventions.

In case of earlier stages of cancer, the situation might be different. Tumor metastasis is a severe aspect of tumor development in patients, largely because tumors are detected too late when metastasis already has occurred. Established tumor therapies mostly include cytotoxic drugs with rather narrow therapeutic windows. Hence, for the treatment in earlier tumor stages, when the suppression of metastasis spread might still be possible, the need is high for new therapies with good tolerability and safety.

The activation of the immune system and, in particular, the activation of toll-like receptor (TLR) signaling offers new promising approaches. TLR9 agonistic CpG-ODN like H2006 or H1826, and TLR7 agonists like the guanosine derivative isatoribine or an Imiquimod derivative were tested in a murine Renca lung metastasis model. All tested molecules virtually completely suppressed the emergence of lung metastases with good tolerability. This provides a convincing rational for clinical development of such molecules for suppression of cancer metastasis and points to the possibility of systemic application of such drugs. However, the SMOL type TLR7 agonists have the advantage of established and cost effective synthesis if compared to the nucleic acid type TLR9 agonists, and are well suited for topical as well as for systemic application.

US-B-6,573,273 describes imidazoquinoline and tetrahydroimidazoquinoline compounds that contain urea, thiourea, acylurea, sulfonylurea or carbamate functionality. The compounds are said to be useful as immunomodulators.

US-B-6,677,349 describes imidazoquinoline and tetrahydroimidazoquinoline compounds that contain sulfonamide functionality at the 1-position. The compounds are said to be useful as immunomodulators.

US-A-2003/0144283 and WO-A-00/76505 describe imidazoquinoline and tetrahydroimidazoquinoline compounds that contain amide functionality at the 1-position. The compounds are said to be useful as immunomodulators.

WO-A-2005/051324 describes imidazoquinoline, pyridine and naphthyridine ring systems substituted in 1-position with oxime or a special N-oxide functionality. The compounds are said to be useful as immunomodulators.

WO-A-2009/118296 describes imidazoquinoline compounds. The compounds are described as toll-like receptor agonist/TLR7 activators.

N-(4-(4-amino-2-(2-methoxyethyl)-lH-imidazo[4,5-c]quinolin-1-yl)butyl)-N-(tetrahydro-2H-pyran-4-yl)acetamide and a process for its preparation are disclosed in WO2019/048350.

The present disclosure provides a novel process for the preparation of N-(4-(4-amino-2-(2-methoxyethyl)-1H-imidazo[4,5-c]quinolin-1-yl)butyl)-N-(tetrahydro-2H-pyran-4-yl)acetamide or a solvate or salt thereof that employs novel intermediates.

### Summary of the Invention

A first aspect of the present invention provides a process for synthesizing N-(4-(4-amino-2-(2-methoxyethyl)-1H-imidazo[4,5-c]quinolin-1-yl)butyl)-N-(tetrahydro-2H-pyran-4-yl)acetamide, or a solvate or salt thereof, as further described in the following. Said compound is an agonist or activator for TLR7 and may serve as cytokine inducing substance. Said compound has the following chemical formula (1):

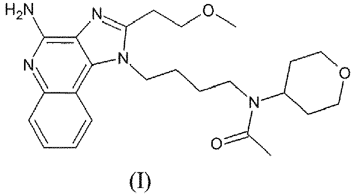

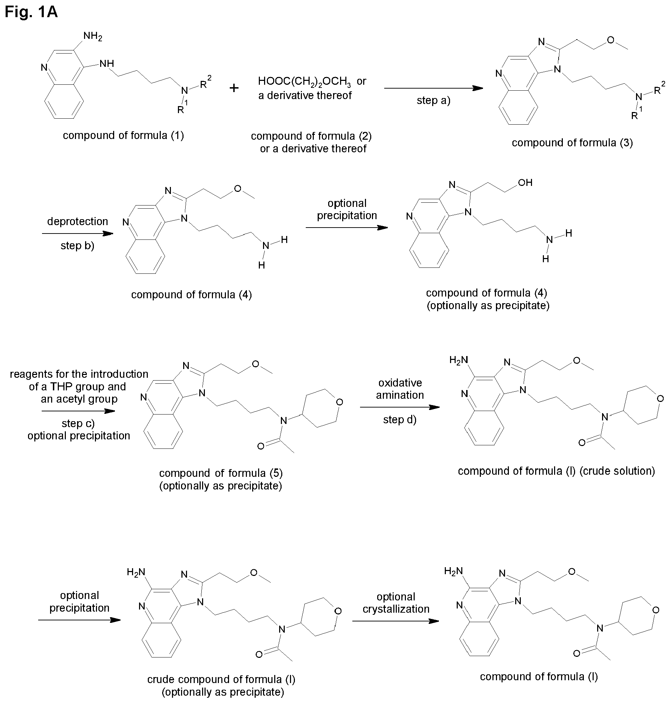

The process of the first aspect comprises the following steps:
a) providing a compound of formula (3): 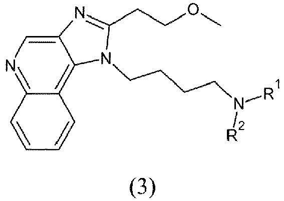 or a solvate or salt thereof, wherein each of R¹ and R² is independently selected from the group consisting of -H and an amino protecting group, and at least one of R¹ and R² is an amino protecting group;
b) reacting the compound of formula (3) or a solvate or salt thereof with one or more reagents to remove the amino protecting group(s) at position R¹ and R² to give a compound of formula (4): 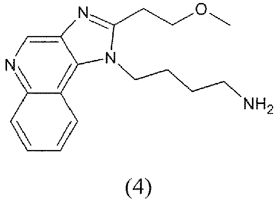 or a solvate or salt thereof;
c) reacting the compound of formula (4) or a solvate or salt thereof with one or more reagents for introducing (i) a tetrahydropyranyl group and (ii) an acetyl group to give a compound of formula (5): 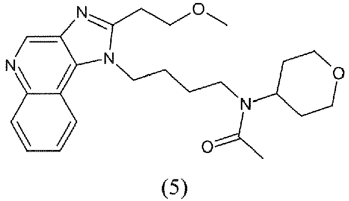 or a s solvate or salt thereof; and
d) subjecting the compound of formula (5) or a solvate or salt thereof to an oxidative amination reaction to give the compound of formula (1) or a solvate or salt thereof. Preferred embodiments of the process of the first aspect are specified below, in the claims, and in any one of Figures 1A to 1H, 2A to 2D, 3A to 3G, 4A to 4H, 5A to 5E, and 6A to 6D.

In a second aspect, the present invention provides a compound selected from the group consisting of: 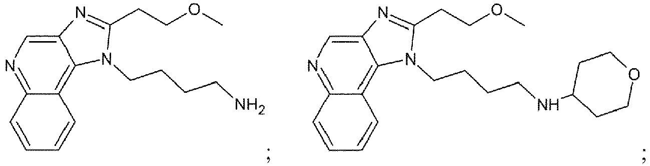 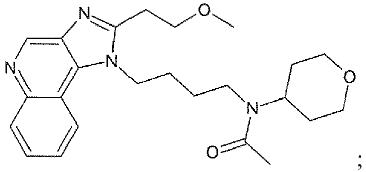 and a solvate or salt thereof. These compounds are intermediates useful in the process of the first aspect.

In a third aspect, the present invention provides a use of a compound of the second aspect for synthesizing a compound of formula (I).

### Brief description of the Figures

Figures 1A to 1H show preferred embodiments of the process of the present invention. Substituents (e.g., R¹, R², R¹⁰) and abbreviations (e.g., THP, PTSA) referred to in Figures 1A to 1H have the same meaning as specified in the description.
Figures 2A to 2D show preferred embodiments of step a) of the process of the present invention.
Figures 3A to 3G show preferred embodiments of step b) of the process of the present invention.
Figures 4A to 4H show preferred embodiments of step c) of the process of the present invention.
Figures 5A to 5E show preferred embodiments of step d) of the process of the present invention.
Figures 6A to 6D show preferred embodiments of steps (precipitation and/or crystallization) which may be performed after step d) of the process of the present invention.

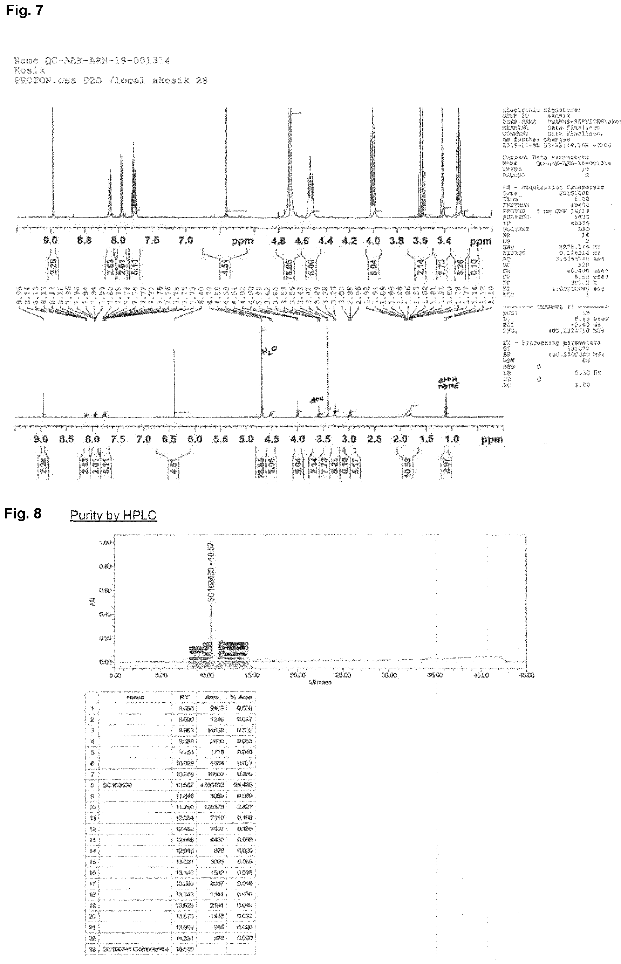

Figure 7 shows a spectrum (¹H NMR, D₂O, 400 MHz) for the compound of formula (4) as fumaric acid addition salt obtained by the process of the present invention.
Figure 8 shows a chromatogram (HPLC) for the compound of formula (4) as fumaric acid addition salt (designated as SC 103439) obtained by the process of the present invention.

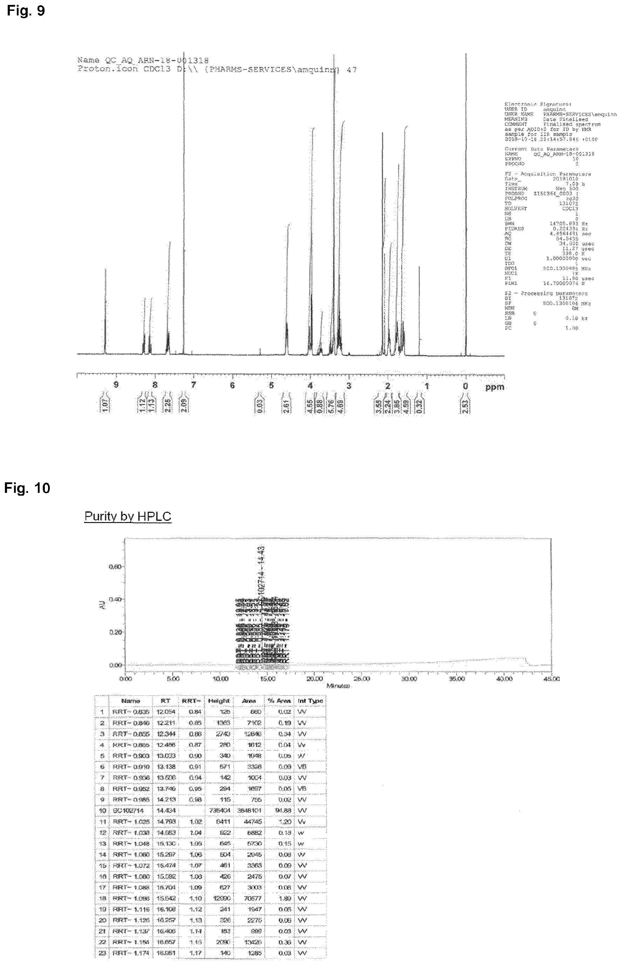

Figure 9 shows a spectrum (¹H NMR, CDCl₃, 500 MHz) for the compound of formula (5) obtained by the process of the present invention.
Figure 10 shows a chromatogram (HPLC) for the compound of formula (5) (designated as SC 102714) obtained by the process of the present invention.
Figure 11 shows a spectrum (¹H NMR, CDCl₃ 500 MHz) for the compound of formula (I) in crude form obtained immediately after step d) of the process of the present invention (i.e., after step d) the compound of formula (I) has not been subjected to a precipitation and/or crystallization step).

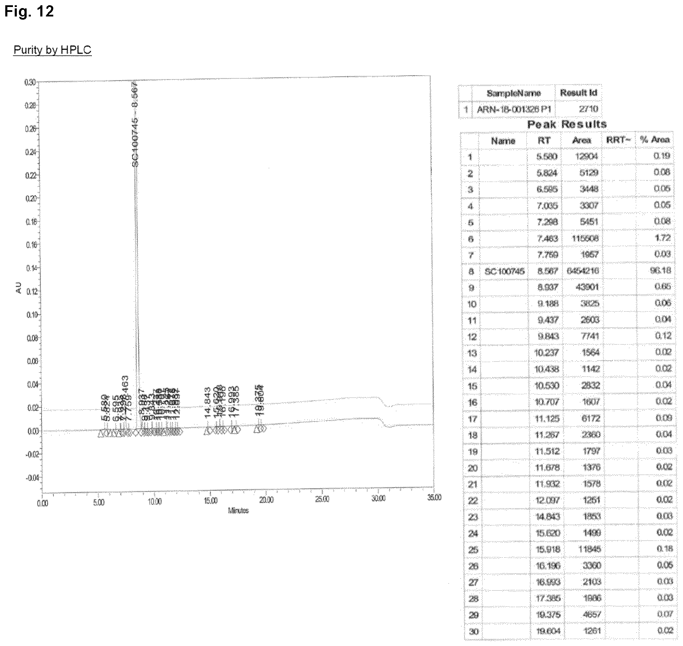

Figure 12 shows a chromatogram (HPLC) for the compound of formula (I) in crude form obtained immediately after step d) of the process of the present invention (i.e., after step d) the compound of formula (I) has not been subjected to a precipitation and/or crystallization step). The compound of formula (I) is designated as SC100745 in Figure 12.

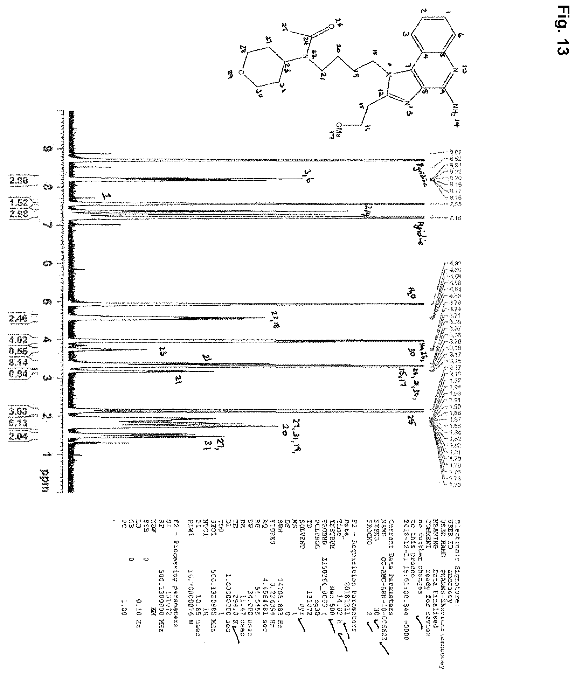

Figure 13 shows a spectrum (¹H NMR, pyridine-d5 500 MHz) for the compound of formula (I) obtained after the final crystallization step of the process of the present invention.

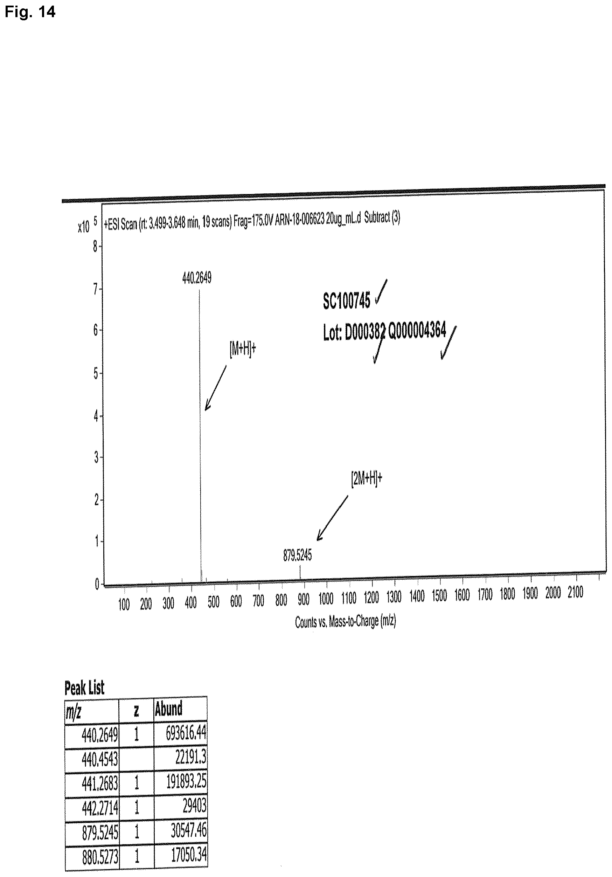

Figure 14 shows a mass spectrum (ESI) for the compound of formula (I) obtained after the final crystallization step of the process of the present invention.

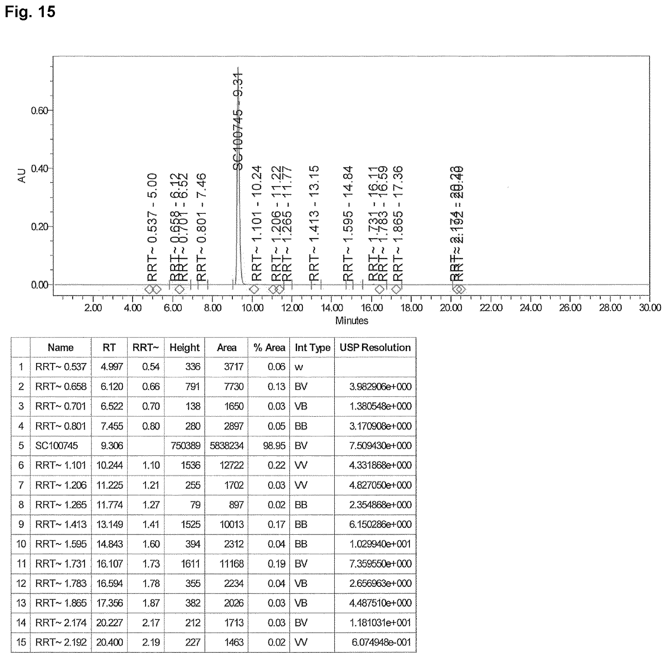

Figure 15 shows a spectrum (HPLC) for the compound of formula (I) obtained after the final crystallization step of the process of the present invention. The compound of formula (I) is designated as SC100745 in Figure 15.

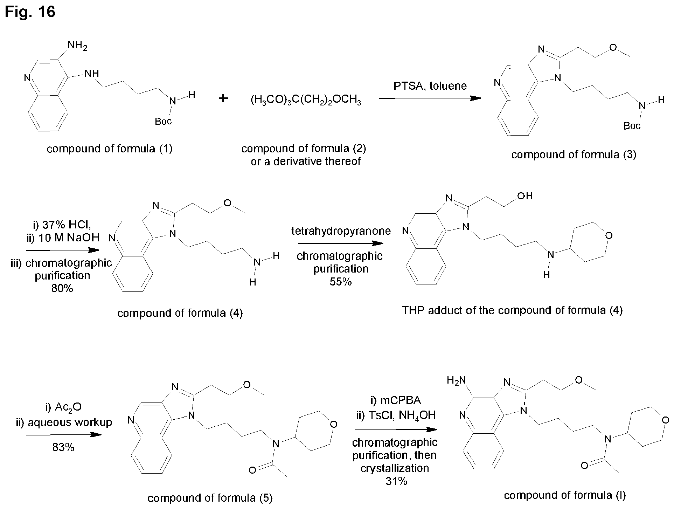

Figure 16 is an overview of a comparative process B for synthesizing N-(4-(4-amino-2-(2-methoxyethyl)-1H-imidazo[4,5-c]quinolin-1-yl)butyl)-N-(tetrahydro-2H-pyran-4-yl)acetamide using chromatographic purification steps.

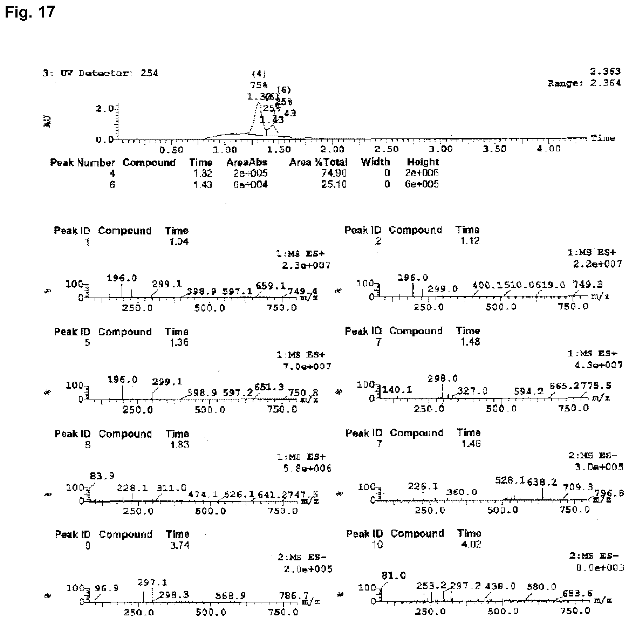

Figure 17 shows a chromatogram (HPLC) for the compound of formula (4) obtained by using the comparative process B.

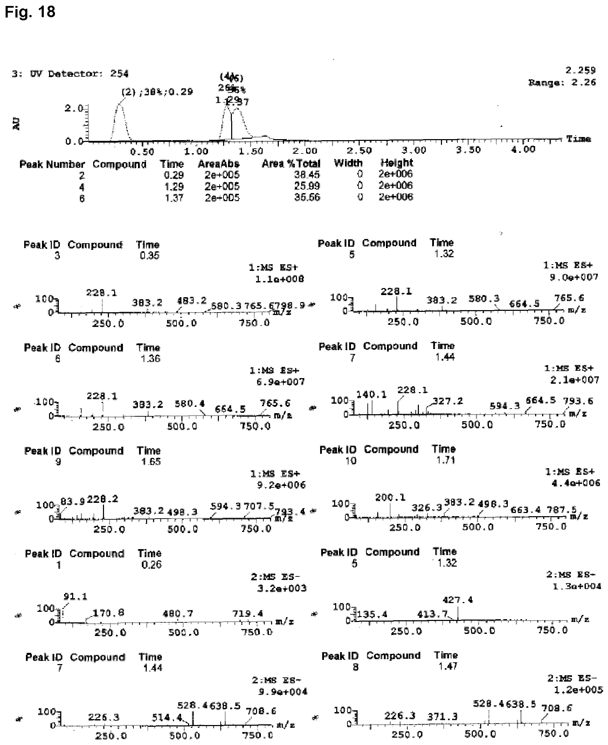

Figure 18 shows a chromatogram (HPLC) for the THP adduct of the compound of formula (4) obtained by using the comparative process B.

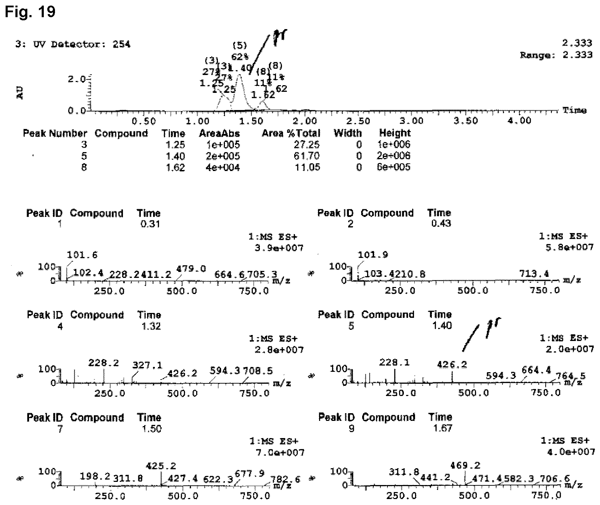

Figure 19 shows a chromatogram (HPLC) for the compound of formula (5) obtained by using the comparative process B.

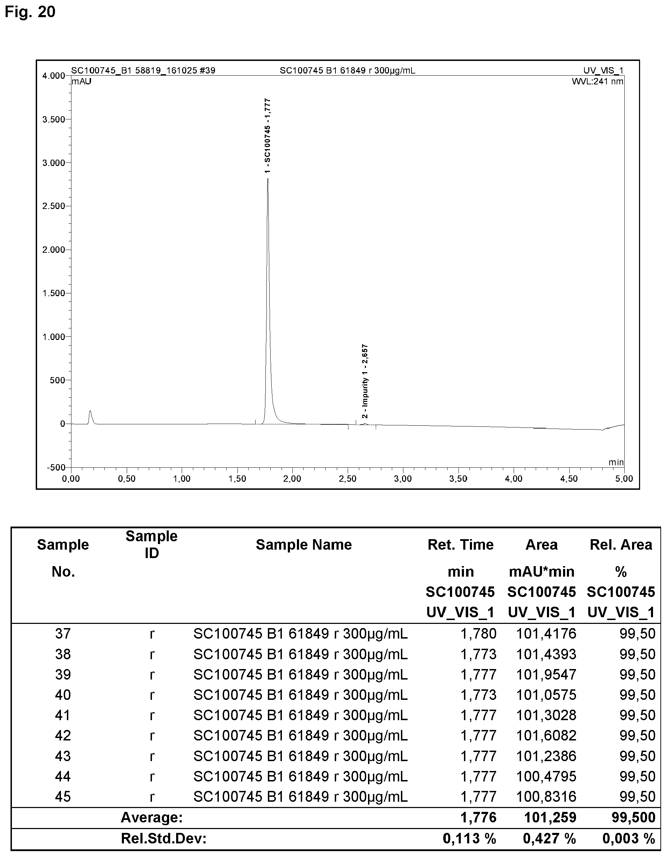

Figure 20 shows a chromatogram (HPLC) for the compound of formula (I) obtained by using the comparative process B. The compound of formula (I) is designated as SC100745 in Figure 20.

### Detailed Description of the Invention

Although the present invention is further described in more detail below, it is to be understood that this invention is not limited to the particular methodologies, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described in more detail. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise. For example, if in a preferred embodiment of the process of the present invention a solvent exchange is performed after step d) and in another preferred embodiment of the process of the present invention a crystallization step is performed after step d), then in a further preferred embodiment of the process of the present invention, a solvent exchange is performed after step d) and a crystallization step is performed after step d).

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

The practice of the present invention will employ, unless otherwise indicated, conventional chemistry methods which are explained in the literature in the field (cf., e.g., Organikum, Deutscher Verlag der Wissenschaften, Berlin 1990; Streitwieser/Heathcook, "Organische Chemie", VCH, 1990; Beyer/Walter, "Lehrbuch der Organischen Chemie", S. Hirzel Verlag Stuttgart, 1988; Carey/Sundberg, "Organische Chemie", VCH, 1995; March, "Advanced Organic Chemistry", John Wiley & Sons, 1985; Rompp Chemie Lexikon, Falbe/Regitz (Hrsg.), Georg Thieme Verlag Stuttgart, New York, 1989).

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. The term "consisting essentially of" means excluding other members, integers or steps of any essential significance. The term "comprising" encompasses the term "consisting essentially of" which, in turn, encompasses the term "consisting of". Thus, at each occurrence in the present application, the term "comprising" may be replaced with the term "consisting essentially of" or "consisting of". Likewise, at each occurrence in the present application, the term "consisting essentially of" may be replaced with the term "consisting of".

The terms "a", "an" and "the" and similar references used in the context of describing the invention (especially in the context of the claims) are to be construed to cover both the singular and the plural, unless otherwise indicated herein or clearly contradicted by the context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by the context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

Where used herein, "and/or" is to be taken as specific disclosure of each of the two specified features or components with or without the other. For example, "X and/or Y" is to be taken as specific disclosure of each of (i) X, (ii) Y, and (iii) X and Y, just as if each is set out individually herein.

In the context of the present invention, the terms "about" and "approximately" are used interchangeably and denote an interval of accuracy that the person of ordinary skill will understand to still ensure the technical effect of the feature in question. The term typically indicates deviation from the indicated numerical value by ±5%, ±4%, ±3%, ±2%, ±1%, ±0.9%, ±0.8%, ±0.7%, ±0.6%, ±0.5%, ±0.4%, ±0.3%, ±0.2%, ±0.1%, ±0.05%, and for example ±0.01%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect.

Recitation of ranges of values herein is merely intended to serve as a shorthand method of referring individually to each separate value falling within the range. Unless otherwise indicated herein, each individual value is incorporated into the specification as if it were individually recited herein.

The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

The term "alkyl" refers to a monoradical of a saturated straight or branched hydrocarbon. Preferably, the alkyl group comprises from 1 to 12 (such as 1 to 10) carbon atoms, i.e., 1, 2, 3, 4, 5, 6, 7, 8, 9, 10, 11, or 12 carbon atoms (such as 1, 2, 3, 4, 5, 6, 7, 8, 9, or 10 carbon atoms), more preferably 1 to 8 carbon atoms, such as 1 to 6 or 1 to 4 carbon atoms. Exemplary alkyl groups include methyl, ethyl, propyl, iso-propyl (also called 2-propyl or 1-methylethyl), butyl, iso-butyl, tert-butyl, n-pentyl, isopentyl, sec-pentyl, neo-pentyl, 1,2-dimethyl-propyl, iso-amyl, n-hexyl, iso-hexyl, sec-hexyl, n-heptyl, iso-heptyl, n-octyl, 2-ethyl-hexyl, n-nonyl, n-decyl, n-undecyl, n-dodecyl, and the like. A "substituted alkyl" means that one or more (such as 1 to the maximum number of hydrogen atoms bound to an alkyl group, e.g., 1, 2, 3, 4, 5, 6, 7, 8, 9, or up to 10, such as between 1 to 5, 1 to 4, or 1 to 3, or 1 or 2) hydrogen atoms of the alkyl group are replaced with a substituent other than hydrogen (when more than one hydrogen atom is replaced the substituents may be the same or different). Preferably, the substituent other than hydrogen is a 1^{st} level substituent, a 2^{nd} level substituent, or a 3^{rd} level substituent as specified herein, such as halogen, -OH, -NH₂, -NHCH₃, -N(CH₃)₂, -CN, -OCH₃, -OCF₃, or optionally substituted aryl. Examples of a substituted alkyl include trifluoromethyl, 2-hydroxyethyl, 2-aminoethyl, 2-(dimethylamino)ethyl, arylalkyl (also called "aralkyl", e.g., benzyl), or heteroarylalkyl (also called "heteroaralkyl").

The term "alkenyl" refers to a monoradical of an unsaturated straight or branched hydrocarbon having at least one carbon-carbon double bond. Generally, the maximal number of carbon-carbon double bonds in the alkenyl group can be equal to the integer which is calculated by dividing the number of carbon atoms in the alkenyl group by 2 and, if the number of carbon atoms in the alkenyl group is uneven, rounding the result of the division down to the next integer. For example, for an alkenyl group having 9 carbon atoms, the maximum number of carbon-carbon double bonds is 4. Preferably, the alkenyl group has 1 to 6 (such as 1 to 4), i.e., 1, 2, 3, 4, 5, or 6, carbon-carbon double bonds. Preferably, the alkenyl group comprises from 2 to 12 (such as 2 to 10) carbon atoms, i.e., 2, 3, 4, 5, 6, 7, 8, 9, 10, 11, or 12 carbon atoms (such as 2, 3, 4, 5, 6, 7, 8, 9, or 10 carbon atoms), more preferably 2 to 8 carbon atoms, such as 2 to 6 carbon atoms or 2 to 4 carbon atoms. Thus, in a preferred embodiment, the alkenyl group comprises from 2 to 12 (e.g., 2 to 10) carbon atoms and 1, 2, 3, 4, 5, or 6 (e.g., 1, 2, 3, 4, or 5) carbon-carbon double bonds, more preferably it comprises 2 to 8 carbon atoms and 1, 2, 3, or 4 carbon-carbon double bonds, such as 2 to 6 carbon atoms and 1, 2, or 3 carbon-carbon double bonds or 2 to 4 carbon atoms and 1 or 2 carbon-carbon double bonds. The carbon-carbon double bond(s) may be in cis (Z) or trans (E) configuration. Exemplary alkenyl groups include vinyl, 1-propenyl, 2-propenyl (i.e., allyl), 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, 5-hexenyl, 1-heptenyl, 2-heptenyl, 3-heptenyl, 4-heptenyl, 5-heptenyl, 6-heptenyl, 1-octenyl, 2-octenyl, 3-octenyl, 4-octenyl, 5-octenyl, 6-octenyl, 7-octenyl, 1-nonenyl, 2-nonenyl, 3-nonenyl, 4-nonenyl, 5-nonenyl, 6-nonenyl, 7-nonenyl, 8-nonenyl, 1-decenyl, 2-decenyl, 3-decenyl, 4-decenyl, 5-decenyl, 6-decenyl, 7-decenyl, 8-decenyl, 9-decenyl, 1-undecenyl, 2-undecenyl, 3-undecenyl, 4-undecenyl, 5-undecenyl, 6-undecenyl, 7-undecenyl, 8-undecenyl, 9-undecenyl, 10-undecenyl, 1-dodecenyl, 2-dodecenyl, 3-dodecenyl, 4-dodecenyl, 5-dodecenyl, 6-dodecenyl, 7-dodecenyl, 8-dodecenyl, 9-dodecenyl, 10-dodecenyl, 11-dodecenyl, and the like. If an alkenyl group is attached to a nitrogen atom, the double bond cannot be alpha to the nitrogen atom. A "substituted alkenyl" means that one or more (such as 1 to the maximum number of hydrogen atoms bound to an alkenyl group, e.g., 1, 2, 3, 4, 5, 6, 7, 8, 9, or up to 10, such as between 1 to 5, 1 to 4, or 1 to 3, or 1 or 2) hydrogen atoms of the alkenyl group are replaced with a substituent other than hydrogen (when more than one hydrogen atom is replaced the substituents may be the same or different). Preferably, the substituent other than hydrogen is a 1^{st} level substituent, a 2^{nd} level substituent, or a 3^{rd} level substituent as specified herein, such as halogen or optionally substituted aryl. An example of a substituted alkenyl is styryl (i.e., 2-phenylvinyl).

The term "alkynyl" refers to a monoradical of an unsaturated straight or branched hydrocarbon having at least one carbon-carbon triple bond. Generally, the maximal number of carbon-carbon triple bonds in the alkynyl group can be equal to the integer which is calculated by dividing the number of carbon atoms in the alkynyl group by 2 and, if the number of carbon atoms in the alkynyl group is uneven, rounding the result of the division down to the next integer. For example, for an alkynyl group having 9 carbon atoms, the maximum number of carbon-carbon triple bonds is 4. Preferably, the alkynyl group has 1 to 6 (such as 1 to 4), i.e., 1, 2, 3, 4, 5, or 6, more preferably 1 or 2 carbon-carbon triple bonds. Preferably, the alkynyl group comprises from 2 to 12 (such as 2 to 10) carbon atoms (such as 1, 2, 3, 4, 5, 6, 7, 8, 9, or 10 carbon atoms), i.e., 2, 3, 4, 5, 6, 7, 8, 9, 10, 11, or 12 carbon atoms, more preferably 2 to 8 carbon atoms, such as 2 to 6 carbon atoms or 2 to 4 carbon atoms. Thus, in a preferred embodiment, the alkynyl group comprises from 2 to 12 (such as 2 to 10) carbon atoms and 1, 2, 3, 4, 5, or 6 (such as 1, 2, 3, 4, or 5 (preferably 1, 2, or 3)) carbon-carbon triple bonds, more preferably it comprises 2 to 8 carbon atoms and 1, 2, 3, or 4 (preferably 1 or 2) carbon-carbon triple bonds, such as 2 to 6 carbon atoms and 1, 2 or 3 carbon-carbon triple bonds or 2 to 4 carbon atoms and 1 or 2 carbon-carbon triple bonds. Exemplary alkynyl groups include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 2-heptynyl, 3-heptynyl, 4-heptynyl, 5-heptynyl, 6-heptynyl, 1-octynyl, 2-octynyl, 3-octynyl, 4-octynyl, 5-octynyl, 6-octynyl, 7-octynyl, 1-nonylyl, 2-nonynyl, 3-nonynyl, 4-nonynyl, 5-nonynyl, 6-nonynyl, 7-nonynyl, 8-nonynyl, 1-decynyl, 2-decynyl, 3-decynyl, 4-decynyl, 5-decynyl, 6-decynyl, 7-decynyl, 8-decynyl, 9-decynyl, and the like. If an alkynyl group is attached to a nitrogen atom, the triple bond cannot be alpha to the nitrogen atom. A "substituted alkynyl" means that one or more (such as 1 to the maximum number of hydrogen atoms bound to an alkynyl group, e.g., 1, 2, 3, 4, 5, 6, 7, 8, 9, or up to 10, such as between 1 to 5, 1 to 4, or 1 to 3, or 1 or 2) hydrogen atoms of the alkynyl group are replaced with a substituent other than hydrogen (when more than one hydrogen atom is replaced the substituents may be the same or different). Preferably, the substituent other than hydrogen is a 1^{st} level substituent, a 2^{nd} level substituent, or a 3^{rd} level substituent as specified herein, such as halogen or optionally substituted aryl.

The term "aryl" or "aromatic ring" refers to a monoradical of an aromatic cyclic hydrocarbon. Preferably, the aryl group contains 3 to 14 (e.g., 5, 6, 7, 8, 9, or 10, such as 5, 6, or 10) carbon atoms which can be arranged in one ring (e.g., phenyl) or two or more condensed rings (e.g., naphthyl). Exemplary aryl groups include cyclopropenylium, cyclopentadienyl, phenyl, indenyl, naphthyl, azulenyl, fluorenyl, anthryl, and phenanthryl. Preferably, "aryl" refers to a monocyclic ring containing 6 carbon atoms or an aromatic bicyclic ring system containing 10 carbon atoms. Preferred examples are phenyl and naphthyl. Aryl does not encompass fullerenes. A "substituted aryl" means that one or more (such as 1 to the maximum number of hydrogen atoms bound to an aryl group, e.g., 1, 2, 3, 4, 5, 6, 7, 8, 9, or up to 10, such as between 1 to 5, 1 to 4, or 1 to 3, or 1 or 2) hydrogen atoms of the aryl group are replaced with a substituent other than hydrogen (when more than one hydrogen atom is replaced the substituents may be the same or different). Preferably, the substituent other than hydrogen is a 1^{st} level substituent, a 2^{nd} level substituent, or a 3^{rd} level substituent as specified herein, such as halogen, -OH, -NH₂, -NHCH₃, -N(CH₃)₂, -CN, -OCH₃, -OCF₃, nitro, alkyl (e.g., C₁₋₆ alkyl), alkenyl (e.g., C₂₋₆ alkenyl), and alkynyl (e.g., C₂₋₆ alkynyl). Examples of a substituted aryl include biphenyl, 2-fluorophenyl, 2-chloro-6-methylphenyl, anilinyl, 3-nitrophenyl, 4-hydroxyphenyl, methoxyphenyl (i.e., 2-, 3-, or 4-methoxyphenyl), and 4-ethoxyphenyl.

The term "heteroaryl" or "heteroaromatic ring" means an aryl group as defined above in which one or more carbon atoms in the aryl group are replaced by heteroatoms (such as O, S, or N). Preferably, heteroaryl refers to a five or six-membered aromatic monocyclic ring, wherein 1, 2, or 3 carbon atoms are replaced by the same or different heteroatoms of O, N, or S. Alternatively, it means an aromatic bicyclic or tricyclic ring system wherein 1, 2, 3, 4, or 5 carbon atoms are replaced with the same or different heteroatoms of O, N, or S. Preferably, in each ring of the heteroaryl group the maximum number of O atoms is 1, the maximum number of S atoms is 1, and the maximum total number of O and S atoms is 2. For example, 3- to 14-membered heteroaryl encompasses monocyclic heteroaryl (e.g., 5- or 6-membered), bicyclic heteroaryl (e.g., 9- or 10-membered), and tricyclic heteroaryl (e.g., 13- or 14-membered). Exemplary heteroaryl groups include furanyl, thienyl, oxazolyl, isoxazolyl, oxadiazolyl, pyrrolyl, imidazolyl, pyrazolyl, triazolyl, tetrazolyl, thiazolyl, isothiazolyl, thiadiazolyl, pyridyl (also called pyridinyl), pyrimidinyl, pyrazinyl, triazinyl, benzofuranyl, indolyl, isoindolyl, benzothienyl, 1H-indazolyl, benzimidazolyl, benzoxazolyl, indoxazinyl, benzisoxazolyl, benzothiazolyl, benzisothiazolyl, benzotriazolyl, quinolinyl, isoquinolinyl, benzodiazinyl, quinoxalinyl, quinazolinyl, benzotriazinyl, pyridazinyl, phenoxazinyl, thiazolopyridinyl, pyrrolothiazolyl, phenothiazinyl, isobenzofuranyl, chromenyl, xanthenyl, phenoxathiinyl, pyrrolizinyl, indolizinyl, indazolyl, purinyl, quinolizinyl, phthalazinyl, naphthyridinyl, cinnolinyl, pteridinyl, carbazolyl, phenanthridinyl, acridinyl, perimidinyl, phenanthrolinyl, phenazinyl, oxazolopyridinyl, isoxazolopyridinyl, pyrrolooxazolyl, and pyrrolopyrrolyl. Exemplary 5- or 6-membered heteroaryl groups include furanyl, thienyl, oxazolyl, isoxazolyl, oxadiazolyl, pyrrolyl, imidazolyl, pyrazolyl, triazolyl, thiazolyl, isothiazolyl, thiadiazolyl, pyridyl, pyrimidinyl, pyrazinyl, triazinyl, and pyridazinyl. A "substituted heteroaryl" means that one or more (such as 1 to the maximum number of hydrogen atoms bound to a heteroaryl group, e.g., 1, 2, 3, 4, 5, 6, 7, 8, 9, or up to 10, such as between 1 to 5, 1 to 4, or 1 to 3, or 1 or 2) hydrogen atoms of the heteroaryl group are replaced with a substituent other than hydrogen (when more than one hydrogen atom is replaced the substituents may be the same or different). Preferably, the substituent other than hydrogen is a 1^{st} level substituent, a 2^{nd} level substituent, or a 3^{rd} level substituent as specified herein, such as halogen, -OH, -NH₂, -NHCH₃, -N(CH₃)₂, -CN, -OCH₃, -OCF₃, alkyl (e.g., C₁₋₆ alkyl), alkenyl (e.g., C₂₋₆ alkenyl), and alkynyl (e.g., C₂₋₆ alkynyl). Examples of a substituted heteroaryl include 2,4-dimethylpyridin-3-yl, 2-methyl-4-bromopyridin-3-yl, 3-methyl-2-pyridin-2-yl, 3-chloro-5-methylpyridin-4-yl, 4-chloro-2-methylpyridin-3 -yl, 3,5 -dimethylpyridin-4-yl, 2-methylpyridin-3 -yl, 2-chloro-4-methyl-thien-3-yl, 1,3,5-trimethylpyrazol-4-yl, 3,5-dimethyl-1,2-dioxazol-4-yl, 1,2,4-trimethylpyrrol-3-yl, 3-phenylpyrrolyl, 2,3'-bifuryl, 4-methylpyridyl, 2-, or 3-ethylindolyl.

The term "cycloalkyl" or "cycloaliphatic" represents cyclic non-aromatic versions of "alkyl" and "alkenyl" with preferably 3 to 14 carbon atoms, such as 3 to 12 or 3 to 10 carbon atoms, i.e., 3, 4, 5, 6, 7, 8, 9, 10, 11, 12, 13, or 14 carbon atoms (such as 3, 4, 5, 6, 7, 8, 9, or 10 carbon atoms), more preferably 3 to 7 carbon atoms. Exemplary cycloalkyl groups include cyclopropyl, cyclopropenyl, cyclobutyl, cyclobutenyl, cyclopentyl, cyclopentenyl, cyclohexyl, cyclohexenyl, cycloheptyl, cycloheptenyl, cyclooctyl, cyclooctenyl, cyclononyl, cyclononenyl, cylcodecyl, cylcodecenyl, and adamantyl. The term "cycloalkyl" is also meant to include bicyclic and tricyclic versions thereof. If bicyclic rings are formed it is preferred that the respective rings are connected to each other at two adjacent carbon atoms, however, alternatively the two rings are connected via the same carbon atom, i.e., they form a spiro ring system or they form "bridged" ring systems. Preferred examples of cycloalkyl include C₃₋₈-cycloalkyl, in particular cyclopropyl, cyclobutyl, cyclopentyl, cyclohexyl, cycloheptyl, cyclooctyl, spiro[3,3]heptyl, spiro[3,4]octyl, spiro[4,3]octyl, bicyclo[4.1.0]heptyl, bicyclo[3.2.0]heptyl, bicyclo[2.2.1]heptyl, bicyclo[2.2.2]octyl, bicyclo[5.1.0]octyl, and bicyclo[4.2.0]octyl. Cycloalkyl does not encompass fullerenes. A "substituted cycloalkyl" means that one or more (such as 1 to the maximum number of hydrogen atoms bound to a cycloalkyl group, e.g., 1, 2, 3, 4, 5, 6, 7, 8, 9, or up to 10, such as between 1 to 5, 1 to 4, or 1 to 3, or 1 or 2) hydrogen atoms of the cycloalkyl group are replaced with a substituent other than hydrogen (when more than one hydrogen atom is replaced the substituents may be the same or different). Preferably, the substituent other than hydrogen is a 1^{st} level substituent, a 2^{nd} level substituent, or a 3^{rd} level substituent as specified herein, such as halogen, -OH, -NH₂, -NHCH₃, -N(CH₃)₂, -CN, -OCH₃, -OCF₃, =O, =S, =NH, alkyl (e.g., C₁₋₆ alkyl), alkenyl (e.g., C₂₋₆ alkenyl), and alkynyl (e.g., C₂₋₆ alkynyl). Examples of a substituted cycloalkyl include oxocyclohexyl, oxocyclopentyl, fluorocyclohexyl, and oxocyclohexenyl.

The term "heterocyclyl" or "heterocyclic ring" means a cycloalkyl group as defined above in which from 1, 2, 3, or 4 ring carbon atoms in the cycloalkyl group are replaced by heteroatoms (such as those selected from the group consisting of O, S, S(O), S(O)₂, N, B, Si, and P, preferably selected from the group consisting of O, S, S(O)₂, and N, more preferably selected from the group consisting of O, S, and N). If a ring of the heterocyclyl group only contains one type of heteroatom, the maximum number of said heteroatom in the ring of said heterocyclyl group may be as follows: 2 O atoms (preferably 1 O atom); 2 S atoms (preferably 1 S atom); 4 N atoms (such as 1, 2, or 3 N atoms); 2 B atoms (preferably 1 B atom); 1 Si atom; and/or 1 P atom. If a ring of the heterocyclyl group contains two or more types of heteroatoms, the maximum number of said heteroatoms in the ring of said heterocyclyl group may be as follows: 1 O atom; 1 S atom; 2 N atoms (preferably 1 N atom); 1 B atom; 1 Si atom; and/or 1 P atom, wherein the maximum total number of heteroatoms in the ring of said heterocyclyl group is 4 and the maximum total number of each heteroatom in the ring of said heterocyclyl group is as follows: 1 O atom; 1 S atom; 1 or 2 N atoms; 1 B atom (preferably 0 B atom); 1 Si atom (preferably 0 Si atom); and/or 1 P atom (preferably 0 P atom). In one embodiment, the heteroatoms of the heterocyclyl group are selected from the group consisting of O, S, and N. In this embodiment, preferably, in each ring of the heterocyclyl group the maximum number of O atoms is 1, the maximum number of S atoms is 1, and the maximum total number of O and S atoms is 2. For example, 3- to 14-membered heterocyclyl encompasses monocyclic heterocyclyl (e.g., 3-, 4-, 5-, 6-, or 7-membered, preferably 4- to 7-membered), bicyclic heterocyclyl (e.g., 8-, 9-, or 10-membered), and tricyclic heterocyclyl (e.g., 12-, 13-, or 14-membered). If a heterocyclyl group comprises two or more rings, these rings either are fused (such as in quinolinyl or purinyl), are a spiro moiety, are a bridged structure, are linked via a double bond, or are a combination thereof. In other words, an unsubstituted heterocyclyl group does not encompass two heterocyclyl groups linked via a single bond. The term "heterocyclyl" is also meant to encompass partially or completely hydrogenated forms (such as dihydro, tetrahydro, hexahydro, octahydro, decahydro, dodecahydro, etc., or perhydro forms) of the above-mentioned heteroaryl groups. Exemplary heterocyclyl groups include azetidinyl, morpholino, isochromanyl, chromanyl, pyrrolidinyl, imidazolidinyl, pyrazolidinyl, piperidinyl, piperazinyl, indolinyl, isoindolinyl, triazininanyl; dihydro forms of pyrrolyl, imidazolyl, and pyrazolyl; di- and tetrahydro forms of furanyl, thienyl, oxazolyl, isoxazolyl, oxadiazolyl, triazolyl, thiazolyl, thiazolyl, thiadiazolyl, pyridyl, pyrazinyl, and triazinyl; di-, tetra- and hexahydro forms of pyrimidinyl, pyridazinyl, pyrrolothiazolyl, and pyrrolizinyl. A "substituted heterocyclyl" means that one or more (such as 1 to the maximum number of hydrogen atoms bound to a heterocyclyl group, e.g., 1, 2, 3, 4, 5, 6, 7, 8, 9, or up to 10, such as between 1 to 5, 1 to 4, or 1 to 3, or 1 or 2) hydrogen atoms of the heterocyclyl group are replaced with a substituent other than hydrogen (when more than one hydrogen atom is replaced the substituents may be the same or different). Preferably, the substituent other than hydrogen is a 1^{st} level substituent, a 2^{nd} level substituent, or a 3^{rd} level substituent as specified herein, such as halogen, -OH, -NH₂, -NHCH₃, -N(CH₃)₂, -CN, -OCH₃, -OCF₃, =O, =S, =NH, alkyl (e.g., C₁₋₆ alkyl), alkenyl (e.g., C₂₋₆ alkenyl), and alkynyl (e.g., C₂₋₆ alkynyl).

The expression "partially hydrogenated form" of an unsaturated compound or group as used herein means that part of the unsaturation has been removed by formally adding hydrogen to the initially unsaturated compound or group without removing all unsaturated moieties. The phrase "completely hydrogenated form" of an unsaturated compound or group is used herein interchangeably with the term "perhydro" and means that all unsaturation has been removed by formally adding hydrogen to the initially unsaturated compound or group. For example, partially hydrogenated forms of a 5-membered heteroaryl group (containing 2 double bonds in the ring, such as furan) include dihydro forms of said 5-membered heteroaryl group (such as 2,3-dihydrofuran or 2,5-dihydrofuran), whereas the tetrahydro form of said 5-membered heteroaryl group (e.g., tetrahydrofuran, i.e., THF) is a completely hydrogenated (or perhydro) form of said 5-membered heteroaryl group. Likewise, for a 6-membered heteroaryl group having 3 double bonds in the ring (such as pyridyl), partially hydrogenated forms include di- and tetrahydro forms (such as di- and tetrahydropyridyl), whereas the hexahydro form (such as piperidinyl in case of the heteroaryl pyridyl) is the completely hydrogenated (or perhydro) derivative of said 6-membered heteroaryl group. Consequently, a hexahydro form of an aryl or heteroaryl can only be considered a partially hydrogenated form according to the present invention if the aryl or heteroaryl contains at least 4 unsaturated moieties consisting of double and triple bonds between ring atoms.

The term "aromatic" as used in the context of hydrocarbons means that the whole molecule has to be aromatic. For example, if a monocyclic aryl is hydrogenated (either partially or completely) the resulting hydrogenated cyclic structure is classified as cycloalkyl for the purposes of the present invention. Likewise, if a bi- or polycyclic aryl (such as naphthyl) is hydrogenated the resulting hydrogenated bi- or polycyclic structure (such as 1,2-dihydronaphthyl) is classified as cycloalkyl for the purposes of the present invention (even if one ring, such as in 1,2-dihydronaphthyl, is still aromatic). A similar distinction is made within the present application between heteroaryl and heterocyclyl. For example, indolinyl, i.e., a dihydro variant of indolyl, is classified as heterocyclyl for the purposes of the present invention, since only one ring of the bicyclic structure is aromatic and one of the ring atoms is a heteroatom.

The term "polycyclic" as used herein means that the structure has two or more (such as 2, 3, 4, 5, 6, 7, 8, 9, or 10), preferably, 2, 3, 4, or 5, more preferably, 2, 3, or 4, rings. Therefore, according to the invention, the term "polycyclic" does not encompass monocyclic structures, wherein the structures only contain one ring. Examples of polycyclic groups are fused structures (such as naphthyl or anthryl), spiro compounds, rings that are linked via single or double bonds (such as biphenyl), and bridged structures (such as bornyl). Exemplary polycyclic structures are those aryl, heteroaryl, cycloalkyl, and heterocyclyl groups specified above which have at least two rings.

The term "halogen" or "halo" means fluoro, chloro, bromo, or iodo.

The term "azido" means -N₃.

The term "N-oxide" means an amine oxide or amine-N-oxide which is a chemical compound containing the functional group (Rⁿ)₃N⁺-O⁻ or (Rⁿ)₃N⁺-OH, i.e., an N-O coordinate covalent bond, wherein Rⁿ is independently selected from the group consisting of hydrogen, alkyl, alkenyl, alkynyl, aryl, heteroaryl, cycloalkyl, and heterocyclyl, wherein each of the alkyl, alkenyl, alkynyl, aryl, heteroaryl, cycloalkyl, and heterocyclyl groups is optionally substituted with one or more (such as 1 to the maximum number of hydrogen atoms bound to the alkyl, alkenyl, alkynyl, aryl, heteroaryl, cycloalkyl, or heterocyclyl group, e.g., 1, 2, 3, 4, 5, 6, 7, 8, 9, or up to 10, such as between 1 to 5, 1 to 4, or 1 to 3, or 1 or 2) independently selected R²⁰, the R²⁰ preferably being a 1^{st} level substituent, a 2^{nd} level substituent, or a 3^{rd} level substituent as specified herein.

The term "optionally substituted" indicates that one or more (such as 1 to the maximum number of hydrogen atoms bound to a group, e.g., 1, 2, 3, 4, 5, 6, 7, 8, 9, or up to 10, such as between 1 to 5, 1 to 4, or 1 to 3, or 1 or 2) hydrogen atom(s) may be replaced with a group (i.e., a 1^{st} level substituent) different from hydrogen such as alkyl (preferably, C₁₋₆ alkyl), alkenyl (preferably, C₂₋₆ alkenyl), alkynyl (preferably, C₂₋₆ alkynyl), aryl (preferably, 6- to 14-membered aryl), heteroaryl (preferably, 3-to 14-membered heteroaryl), cycloalkyl (preferably, 3- to 14-membered cycloalkyl), heterocyclyl (preferably, 3- to 14-membered heterocyclyl), halogen, -CN, azido, -NO₂, -OR⁷¹, -N(R⁷²)(R⁷³), -S(O)₀₋₂R⁷¹, -S(O)₁₋₂OR⁷¹, -OS(O)₁₋₂R⁷¹, -OS(O)₁₋₂OR⁷¹, -S(O)₁₋₂N(R⁷²)(R⁷³), -OS(O)₁₋₂N(R⁷²)(R⁷³), -N(R⁷¹)S(O)₁₋₂R⁷¹, -NR⁷¹S(O)₁₋₂OR⁷¹, -NR⁷¹S(O)₁₋₂N(R⁷²)(R⁷³), -OP(O)(OR⁷¹)₂, -C(=X¹)R⁷¹, -C(=X¹)X¹R⁷¹, -X¹C(=X¹)R⁷¹, and -X¹C(=X¹)X¹R⁷¹, and/or any two 1^{st} level substituents which are bound to the same carbon atom of a cycloalkyl or heterocyclyl group may join together to form =X¹, wherein each of the alkyl, alkenyl, alkynyl, aryl, heteroaryl, cycloalkyl, and heterocyclyl groups of the 1^{st} level substituent may themselves be substituted by one or more (e.g., one, two or three) substituents (i.e., a 2^{nd} level substituent) selected from the group consisting of C₁₋₆ alkyl, C₂₋₆ alkenyl, C₂₋₆ alkynyl, 6- to 14-membered aryl, 3- to 14-membered heteroaryl, 3- to 14-membered cycloalkyl, 3- to 14-membered heterocyclyl, halogen, -CF₃, -CN, azido, -NO₂, -OR⁸¹, -N(R⁸²)(R⁸³), -S(O)₀₋₂R⁸¹, -S(O)₁₋₂OR⁸¹, -OS(O)₁₋₂R⁸¹, -OS(O)₁₋₂OR⁸¹, -S(O)₁₋₂N(R⁸²)(R⁸³), -OS(O)₁₋₂N(R⁸²)(R⁸³), -N(R⁸¹)S(O)₁₋₂R⁸¹, -NR⁸¹S(O)₁₋₂OR⁸¹, -NR⁸¹S(O)₁₋₂N(R⁸²)(R⁸³), -OP(O)(OR⁸¹)₂, -C(=X²)R⁸¹, -C(=X²)X²R⁸¹, -X²C(=X²)R⁸¹, and -X²C(=X²)X²R⁸¹, and/or any two 2^{nd} level substituents which are bound to the same carbon atom of a cycloalkyl or heterocyclyl group being a 1^{st} level substituent may join together to form =X², wherein each of the C₁₋₆ alkyl, C₂₋₆ alkenyl, C₂₋₆ alkynyl, 6- to 14-membered aryl, 3- to 14-membered heteroaryl, 3- to 14-membered cycloalkyl, 3- to 14-membered heterocyclyl groups of the 2^{nd} level substituent is optionally substituted with one or more (e.g., one, two or three) substituents (i.e., a 3^{rd} level substituent) independently selected from the group consisting of C₁₋₃ alkyl, halogen, -CF₃, -CN, azido, -NO₂, -OH, -O(C₁₋₃ alkyl), -OCF₃, -S(C₁₋₃ alkyl), -NH₂, -NH(C₁₋₃ alkyl), -N(C₁₋₃ alkyl)₂, -NHS(O)₂(C₁₋₃ alkyl), -S(O)₂NH_{2-z}(C₁₋₃ alkyl)_{z}, -C(=O)OH, -C(=O)O(C₁₋₃ alkyl), -C(=O)NH_{2-z}(C₁₋₃ alkyl)_{z}, -NHC(=O)(C₁₋₃ alkyl), -NHC(=NH)NH_{z-2}(C₁₋₃ alkyl)_{z}, and -N(C₁₋₃ alkyl)C(=NH)NH_{2-z}(C₁₋₃ alkyl)_{z}, wherein each z is independently 0, 1, or 2 and each C₁₋₃ alkyl is independently methyl, ethyl, propyl or isopropyl, and/or any two 3^{rd} level substituents which are bound to the same carbon atom of a 3- to 14-membered cycloalkyl or heterocyclyl group being a 2^{nd} level substituent may join together to form =O, =S, =NH, or =N(C₁₋₃ alkyl);
wherein
each of R⁷¹, R⁷², and R⁷³ is independently selected from the group consisting of H, C₁₋₆ alkyl, C₂₋₆ alkenyl, C₂₋₆ alkynyl, 3- to 7-membered cycloalkyl, 5- or 6-membered aryl, 5- or 6-membered heteroaryl, and 3- to 7-membered heterocyclyl, wherein each of the C₁₋₆ alkyl, C₂₋₆ alkenyl, C₂₋₆ alkynyl, 3- to 7-membered cycloalkyl, 5- or 6-membered aryl, 5- or 6-membered heteroaryl, and 3- to 7-membered heterocyclyl groups is optionally substituted with one, two or three substituents independently selected from the group consisting of C₁₋₃ alkyl, halogen, -CF₃, -CN, azido, -NO₂, -OH, -O(C₁₋₃ alkyl), -OCF₃, =O, -S(C₁₋₃ alkyl), -NH₂, -NH(C₁₋₃ alkyl), -N(C₁₋₃ alkyl)₂, -NHS(O)₂(C₁₋₃ alkyl), -S(O)₂NH_{2-z}(C₁₋₃ alkyl)_{z}, -C(=O)(C₁₋₃ alkyl), -C(=O)OH, -C(=O)O(C₁₋₃ alkyl), -C(=O)NH_{2-z}(C₁₋₃ alkyl)_{z}, -NHC(=O)(C₁₋₃ alkyl), -NHC(=NH)NH_{z-2}(C₁₋₃ alkyl)_{z}, and -N(C₁₋₃ alkyl)C(=NH)NH_{2-z}(C₁₋₃ alkyl)_{z}, wherein each z is independently 0, 1, or 2 and each C₁₋₃ alkyl is independently methyl, ethyl, propyl or isopropyl;
each of R⁸¹, R⁸², and R⁸³ is independently selected from the group consisting of H, C₁₋₄ alkyl, C₂₋₄ alkenyl, C₂₋₄ alkynyl, 3- to 6-membered cycloalkyl, 5- or 6-membered aryl, 5- or 6-membered heteroaryl, and 3- to 6-membered heterocyclyl, wherein each of the C₁₋₄ alkyl, C₂₋₄ alkenyl, C₂₋₄ alkynyl, 3- to 6-membered cycloalkyl, 5- or 6-membered aryl, 5- or 6-membered heteroaryl, and 3- to 6-membered heterocyclyl groups is optionally substituted with one, two or three substituents independently selected from the group consisting of C₁₋₃ alkyl, halogen, -CF₃, -CN, azido, -NO₂, -OH, -O(C₁₋₃ alkyl), -OCF₃, =O, -S(C₁₋₃ alkyl), -NH₂, -NH(C₁₋₃ alkyl), -N(C₁₋₃ alkyl)₂, -NHS(O)₂(C₁₋₃ alkyl), -S(O)₂NH_{2-z}(C₁₋₃ alkyl)_{z}, -C(=O)(C₁₋₃ alkyl), -C(=O)OH, -C(=O)O(C₁₋₃ alkyl), -C(=O)NH_{2-z}(C₁₋₃ alkyl)_{z}, -NHC(=O)(C₁₋₃ alkyl), -NHC(=NH)NH_{z-2}(C₁₋₃ alkyl)_{z}, and -N(C₁₋₃ alkyl)C(=NH)NH_{2-z}(C₁₋₃ alkyl)_{z}, wherein each z is independently 0, 1, or 2 and each C₁₋₃ alkyl is independently methyl, ethyl, propyl or isopropyl; and
each of X¹ and X² is independently selected from O, S, and N(R⁸⁴), wherein R⁸⁴ is H or C₁₋₃ alkyl.

Typical 1^{st} level substituents are preferably selected from the group consisting of C₁₋₆ alkyl, C₂₋₆ alkenyl, C₂₋₆ alkynyl, 6- to 14-membered (such as 6- to 10-membered) aryl, 3- to 14-membered (such as 5- or 6-membered) heteroaryl, 3- to 14-membered (such as 3- to 7-membered) cycloalkyl, 3- to 14-membered (such as 3- to 7-membered) heterocyclyl, halogen, -CN, azido, -NO₂, -OR⁷¹, -N(R⁷²)(R⁷³), -S(O)₀₋₂R⁷¹, -S(O)₁₋₂OR⁷¹, -OS(O)₁₋₂R⁷¹, -OS(O)₁₋₂OR⁷¹, -S(O)₁₋₂N(R⁷²)(R⁷³), -OS(O)₁₋₂N(R⁷²)(R⁷³), -N(R⁷¹)S(O)₁₋₂R⁷¹, -NR⁷¹S(O)₁₋₂OR⁷¹, -C(=X¹)R⁷¹, -C(=X¹)X¹R⁷¹, -X¹C(=X¹)R⁷¹, and -X¹C(=X¹)X¹R⁷¹, such as C₁₋₄ alkyl, C₂₋₄ alkenyl, C₂₋₄ alkynyl, 6-membered aryl, 5- or 6-membered heteroaryl, 3- to 7-membered cycloalkyl, 3- to 7-membered (such as 5- or 6-membered) heterocyclyl, halogen, -CF₃, -CN, azido, -NO₂, -OH, -O(C₁₋₃ alkyl), -S(C₁₋₃ alkyl), -NH₂, -NH(C₁₋₃ alkyl), -N(C₁₋₃ alkyl)₂, -NHS(O)₂(C₁₋₃ alkyl), -S(O)₂NH_{2-z}(C₁₋₃ alkyl)_{z}, -C(=O)OH, -C(=O)O(C₁₋₃ alkyl), -C(=O)NH_{2-z}(C₁₋₃ alkyl)_{z}, -NHC(=O)(C₁₋₃ alkyl), -NHC(=NH)NH_{z-2}(C₁₋₃ alkyl)_{z}, and -N(C₁₋₃ alkyl)C(=NH)NH_{2-z}(C₁₋₃ alkyl)_{z}, wherein each z is independently 0, 1, or 2 and each C₁₋₃ alkyl is independently methyl, ethyl, propyl or isopropyl; wherein X¹ is independently selected from O, S, NH and N(CH₃); and each of R⁷¹, R⁷², and R⁷³ is as defined above or, preferably, is independently selected from the group consisting of H, C₁₋₄ alkyl, C₂₋₄ alkenyl, C₂₋₄ alkynyl, 5- or 6-membered cycloalkyl, 5- or 6-membered aryl, 5- or 6-membered heteroaryl, and 5- or 6-membered heterocyclyl, wherein each of the alkyl, alkenyl, alkynyl, cycloalkyl, aryl, heteroaryl, and heterocyclyl groups is optionally substituted with one, two or three substituents independently selected from the group consisting of C₁₋₃ alkyl, halogen, -CF₃, -CN, azido, -NO₂, -OH, -O(C₁₋₃ alkyl), -S(C₁₋₃ alkyl), -NH₂, -NH(C₁₋₃ alkyl), -N(C₁₋₃ alkyl)₂, -NHS(O)₂(C₁₋₃ alkyl), -S(O)₂NH_{2-z}(C₁₋₃ alkyl)_{z}, -C(=O)OH, -C(=O)O(C₁₋₃ alkyl), -C(=O)NH_{2-z}(C₁₋₃ alkyl)_{z}, -NHC(=O)(C₁₋₃ alkyl), -NHC(=NH)NH_{z-2}(C₁₋₃ alkyl)_{z}, and -N(C₁₋₃ alkyl)C(=NH)NH_{2-z}(C₁₋₃ alkyl)_{z}, wherein each z is independently 0, 1, or 2 and each C₁₋₃ alkyl is independently methyl, ethyl, propyl or isopropyl.

Particular examples of 1^{st} level substituents are independently selected from the group consisting of C₁₋₃ alkyl, phenyl, imidazolyl, thiazolyl, cyclopentyl, cyclohexyl, dihydrothiazolyl, thiazolidinyl, halogen, -CF₃, -CN, -OH, -O(C₁₋₃ alkyl), -S(C₁₋₃ alkyl), -NH₂, -NH(C₁₋₃ alkyl), -N(C₁₋₃ alkyl)₂, -NHS(O)₂(C₁₋₃ alkyl), -C(=O)OH, -C(=O)O(C₁₋₃ alkyl), -C(=O)NH_{2-z}(C₁₋₃ alkyl)_{z}, -NHC(=O)(C₁₋₃ alkyl), -NHC(=NH)NH_{z-2}(C₁₋₃ alkyl)_{z}, and -N(C₁₋₃ alkyl)C(=NH)NH_{2-z}(C₁₋₃ alkyl)_{z}, wherein each z is independently 0, 1, or 2 and each C₁₋₃ alkyl is independently methyl, ethyl, propyl or isopropyl. Particularly preferred 1^{st} level substituents are independently selected from the group consisting of C₁₋₃ alkyl, phenyl, thiazolidinyl, halogen (such as F, Cl, or Br), -NH₂, -NHS(O)₂(C₁₋₃ alkyl), -NHC(=O)(C₁₋₃ alkyl), and -NHC(=NH)NH_{z-2}(C₁₋₃ alkyl)_{z}, wherein z is 0, 1, or 2 and each C₁₋₃ alkyl is independently methyl, ethyl, propyl or isopropyl.

Typical 2^{nd} level substituents are preferably selected from the group consisting of C₁₋₄ alkyl, C₂₋₄ alkenyl, C₂₋₄ alkynyl, 6- or 10-membered aryl, 5- or 6-membered heteroaryl, 5- or 6-membered cycloalkyl, 5- or 6-membered heterocyclyl, halogen, =O, =S, -CF₃, -CN, azido, -NO₂, -OH, -O(C₁₋₃ alkyl), -S(C₁₋₃ alkyl), -NH₂, -NH(C₁₋₃ alkyl), -N(C₁₋₃ alkyl)₂, -NHS(O)₂(C₁₋₃ alkyl), -S(O)₂NH_{2-z}(C₁₋₃ alkyl)_{z}, -C(=O)OH, -C(=O)O(C₁₋₃ alkyl), -C(=O)NH_{2-z}(C₁₋₃ alkyl)_{z}, -NHC(=O)(C₁₋₃ alkyl), -NHC(=NH)NH_{z-2}(C₁₋₃ alkyl)_{z}, and -N(C₁₋₃ alkyl)C(=NH)NH_{2-z}(C₁₋₃ alkyl)_{z}, wherein each z is independently 0, 1, or 2 and each C₁₋₃ alkyl is independently methyl, ethyl, propyl or isopropyl. Particular examples of 2^{nd} level substituents are independently selected from the group consisting of C₁₋₃ alkyl, phenyl, 5- or 6-membered heteroaryl, 5- or 6-membered cycloalkyl, 5- or 6-membered heterocyclyl, halogen, =O, =S, -CF₃, -CN, -OH, -O(C₁₋₃ alkyl), -S(C₁₋₃ alkyl), -NH₂, -NH(C₁₋₃ alkyl), -N(C₁₋₃ alkyl)₂, -NHS(O)₂(C₁₋₃ alkyl), -C(=O)OH, -C(=O)O(C₁₋₃ alkyl), -C(=O)NH_{2-z}(C₁₋₃ alkyl)_{z}, -NHC(=O)(C₁₋₃ alkyl), -NHC(=NH)NH_{z-2}(C₁₋₃ alkyl)_{z}, and -N(C₁₋₃ alkyl)C(=NH)NH_{2-z}(C₁₋₃ alkyl)_{z}, wherein each z is independently 0, 1, or 2 and each C₁₋₃ alkyl is independently methyl, ethyl, propyl or isopropyl. Particularly preferred 2^{nd} level substituents are independently selected from the group consisting of methyl, ethyl, propyl, isopropyl, phenyl, =O, and =S.

Typical 3^{rd} level substituents are preferably selected from the group consisting of C₁₋₃ alkyl, phenyl, halogen, -CF₃, -OH, -OCH₃, -SCH₃, -NH_{2-z}(CH₃)_{z}, -C(=O)OH, and -C(=O)OCH₃, wherein z is 0, 1, or 2 and C₁₋₃ alkyl is methyl, ethyl, propyl or isopropyl. Particularly preferred 3^{rd} level substituents are selected from the group consisting of methyl, ethyl, propyl, isopropyl, halogen (such as F, Cl, or Br), and -CF₃, such as halogen (e.g., F, Cl, or Br), and -CF₃.

The term "optional" or "optionally" as used herein means that the subsequently described event, circumstance or condition may or may not occur, and that the description includes instances where said event, circumstance, or condition occurs and instances in which it does not occur.

"Isomers" are compounds having the same molecular formula but differ in structure ("structural isomers") or in the geometrical (spatial) positioning of the functional groups and/or atoms ("stereoisomers"). "Enantiomers" are a pair of stereoisomers which are non-superimposable mirror-images of each other. A "racemic mixture" or "racemate" contains a pair of enantiomers in equal amounts and is denoted by the prefix (±). "Diastereomers" are stereoisomers which are non-superimposable and which are not mirror-images of each other. "Tautomers" are structural isomers of the same chemical substance that spontaneously and reversibly interconvert into each other, even when pure, due to the migration of individual atoms or groups of atoms; i.e., the tautomers are in a dynamic chemical equilibrium with each other. Examples of tautomers are the isomers of the ketoenol-tautomerism. "Conformers" are stereoisomers that can be interconverted just by rotations about formally single bonds, and include - in particular - those leading to different 3-dimentional forms of (hetero)cyclic rings, such as chair, half-chair, boat, and twist-boat forms of cyclohexane.

"Polymorphism" as referred to herein means that a solid material (such as a compound) is able to exist in more than one form or crystalline structure, i.e., "polymorphic modifications" or "polymorphic forms". The terms "polymorphic modifications", "polymorphic forms", and "polymorphs" are used interchangeable in the present invention. According to the present invention, these "polymorphic modifications" include crystalline forms, amorphous forms, solvates, and hydrates. Mainly, the reason for the existence of different polymorphic forms lies in the use of different conditions during the crystallization process, such as the following:
- solvent effects (the packing of crystal may be different in polar and nonpolar solvents);
- certain impurities inhibiting growth pattern and favor the growth of a metastable polymorphs;
- the level of supersaturation from which material is crystallized (in which generally the higher the concentration above the solubility, the more likelihood of metastable formation);
- temperature at which crystallization is carried out;
- geometry of covalent bonds (differences leading to conformational polymorphism);
- change in stirring conditions.

Polymorphic forms may have different chemical, physical, and/or pharmacological properties, including but not limited to, melting point, X-ray crystal and diffraction pattern, chemical reactivity, solubility, dissolution rate, vapor pressure, density, hygroscopicity, flowability, stability, compactability, and bioavailability. Polymorphic forms may spontaneously convert from a metastable form (unstable form) to the stable form at a particular temperature. According to Ostwald's rule, in general it is not the most stable but the least stable polymorph that crystallizes first. Thus, quality, efficacy, safety, processability and/or manufacture of a chemical compound, such as a compound of the present invention, can be affected by polymorphism. Often, the most stable polymorph of a compound (such as a compound of the present invention) is chosen due to the minimal potential for conversion to another polymorph. However, a polymorphic form which is not the most stable polymorphic form may be chosen due to reasons other than stability, e.g. solubility, dissolution rate, and/or bioavailability.

The term "crystalline form" of a material as used herein means that the smallest components (i.e., atoms, molecule or ions) of said material form crystal structures. A "crystal structure" as referred to herein means a unique three-dimensional arrangement of atoms or molecules in a crystalline liquid or solid and is characterized by a pattern, a set of atoms arranged in a particular manner, and a lattice exhibiting long-range order and symmetry. A lattice is an array of points repeating periodically in three dimensions and patterns are located upon the points of a lattice. The subunit of the lattice is the unit cell. The lattice parameters are the lengths of the edges of a unit cell and the angles between them. The symmetry properties of the crystal are embodied in its space group. In order to describe a crystal structure the following parameters are required: chemical formula, lattice parameters, space group, the coordinates of the atoms and occupation number of the point positions.

The term "amorphous form" of a material as used herein means that the smallest components (i.e., atoms, molecule or ions) of said material are not arranged in a lattice but are arranged randomly. Thus, unlike crystals in which a short-range order (constant distances to the next neighbor atoms) and a long-range order (periodical repetition of a basic lattice) exist, only a short-range order exists in an amorphous form.

The term "solvate" as used herein refers to an addition complex of a dissolved material in a solvent (such as an organic solvent (e.g., an aliphatic alcohol (such as methanol, ethanol, n-propanol, isopropanol), acetone, acetonitrile, ether, and the like), water or a mixture of two or more of these liquids), wherein the addition complex exists in the form of a crystal or mixed crystal. The amount of solvent contained in the addition complex may be stoichiometric or non-stoichiometric. A "hydrate" is a solvate wherein the solvent is water.

In isotopically labeled compounds one or more atoms are replaced by a corresponding atom having the same number of protons but differing in the number of neutrons. For example, a hydrogen atom may be replaced by a deuterium atom. Exemplary isotopes which can be used in the compounds of the present invention include deuterium, ¹¹C, ¹³C, ¹⁴C, ¹⁵N, ¹⁸F, ³²P, ³²S, ³⁵S, ³⁶Cl, and ¹²⁵I.

The compounds which are used and/or synthesized in the process of the invention and which contain a basic functionality may form salts, in particular pharmaceutically acceptable salts, with a variety of inorganic or organic acids. The compounds which are used and/or synthesized in the process of the invention and which contain an acidic functionality may form salts, in particular pharmaceutically acceptable salts, with a variety of inorganic or organic bases. Exemplary inorganic and organic acids/bases as well as exemplary acid/base addition salts of these compounds are given below. The compounds which are used and/or synthesized in the process of the invention and which contain both basic and acidic functionalities may be converted into either base or acid addition salt. The neutral forms of the compounds which are used and/or synthesized in the process of the invention may be regenerated by contacting the salt with a base or acid and isolating the parent compound in the conventional manner. The salts are preferably pharmaceutically acceptable salts.

The term "pharmaceutically acceptable" refers to the non-toxicity of a material which does not interact with the (e.g., therapeutic) action of the active component of a pharmaceutical composition.

"Pharmaceutically acceptable salts" comprise, for example, acid addition salts which may, for example, be formed by mixing a solution of compounds with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compound carries an acidic moiety, suitable pharmaceutically acceptable salts thereof may include alkali metal salts (e.g., sodium or potassium salts); alkaline earth metal salts (e.g., calcium or magnesium salts); and salts formed with suitable organic ligands (e.g., ammonium, quaternary ammonium and amine cations formed using counteranions such as halide, hydroxide, carboxylate, sulfate, phosphate, nitrate, alkyl sulfonate and aryl sulfonate). Illustrative examples of pharmaceutically acceptable salts include, but are not limited to, acetate, adipate, alginate, arginate, ascorbate, aspartate, benzenesulfonate, benzoate, bicarbonate, bisulfate, bitartrate, borate, bromide, butyrate, calcium edetate, camphorate, camphorsulfonate, camsylate, carbonate, chloride, citrate, clavulanate, cyclopentanepropionate, digluconate, dihydrochloride, dodecylsulfate, edetate, edisylate, estolate, esylate, ethanesulfonate, formate, fumarate, galactate, galacturonate, gluceptate, glucoheptonate, gluconate, glutamate, glycerophosphate, glycolylarsanilate, hemisulfate, heptanoate, hexanoate, hexylresorcinate, hydrabamine, hydrobromide, hydrochloride, hydroiodide, 2-hydroxyethanesulfonate, hydroxynaphthoate, iodide, isobutyrate, isothionate, lactate, lactobionate, laurate, lauryl sulfate, malate, maleate, malonate, mandelate, mesylate, methanesulfonate, methylsulfate, mucate, 2-naphthalenesulfonate, napsylate, nicotinate, nitrate, N-methylglucamine ammonium salt, oleate, oxalate, pamoate (embonate), palmitate, pantothenate, pectinate, persulfate, 3-phenylpropionate, phosphate/diphosphate, phthalate, picrate, pivalate, polygalacturonate, propionate, salicylate, stearate, sulfate, suberate, succinate, tannate, tartrate, teoclate, tosylate, triethiodide, undecanoate, valerate, and the like (see, for example, Berge et al., "Pharmaceutical Salts", J. Pharm. Sci., 66, pp. 1-19 (1977)).

The expression "reflux temperature" as used herein in conjunction with a reaction mixture refers to the boiling point of the liquid contained in the reaction mixture which has the lowest boiling point of all liquids contained in the reaction mixture.

In a preferred embodiment, the product obtained by the process of the invention (in particular the compound of formula (I)) is substantially free of contaminants.

The term "substantially free of contaminants" as used herein in conjunction with product obtained by the process of the invention means that the amount of contaminants in the product obtained by the process of the invention is at most 5% by weight (preferably at most 4% by weight, at most 3% by weight, at most 2% by weight, at most 1% by weight, at most 0.5% by weight, at most 0.1% by weight, at most 0.05% by weight, at most 0.01% by weight, at most 0.005% by weight, at most 0.001% by weight), based on the total weight of said product.

The term "chromatography-free manner" as used herein in the context of the synthesis of a compound of interest (such as a compound of formula (1) or a solvate or salt thereof or an intermediate of any one of formulas (3), (4), and (5)) means that in at least one of the steps of said synthesis (preferably in all steps of said synthesis) the separation of the compound of interest from other compounds (such as contaminants) is achieved by means other than chromatography, preferably by means of precipitating the compound of interest, optionally washing the precipitated compound of interest with a suitable solvent (in particular, a solvent in which the compound of interest is poorly soluble), and optionally drying the precipitated compound of interest. However, the term "chromatography-free manner" as used herein in the context of the synthesis of a compound of interest does not mean that the analysis of a sample obtained during the synthesis is performed using chromatography (i.e., a process for synthesizing a compound of interest which is performed in a chromatography-free manner merely excludes preparative steps using chromatography, but does not exclude analytical steps using chromatography).

The term "equivalent" as used herein means the amount of a compound containing 1 mole of hydrogen (i.e., 1.008 g of hydrogen) or 1 mole of a different element (e.g., 12.011 g for carbon or 15.999 g for oxygen). For example, a solution of NaOH having a concentration of 40 g/l is equivalent to (i) a solution of KOH having a concentration of 56.5 g/l, (ii) a solution of HCl having a concentration of 36.5 g; or (iv) a solution of H₂SO₄ having a concentration of 49 g/l. Furthermore, in the context of, for example, a condensation reaction of a first compound with a second compound, wherein the first and second compounds react in a molar ratio of 1:1, the term "1 equivalent" means that x moles (e.g., x = 2) of the first compound and x moles of the second compound are used, i.e., the molar amount of the first compound is the same as the molar amount of the second compound.

The terms "poorly soluble" compound and "insoluble" compound are used herein interchangeably in the context with a solvent and mean that under standard conditions less than 0.5 parts by weight of the compound (preferably, less than 0.4 parts by weight, such as less than 0.3 parts by weight, less than 0.2 parts by weight, less than 0.1 parts by weight, less than 0.09 parts by weight, less than 0.08 parts by weight, less than 0.07 parts by weight, less than 0.06 parts by weight, less than 0.05 parts by weight, less than 0.04 parts by weight, less than 0.03 parts by weight, less than 0.02 parts by weight, less than 0.01 parts by weight, less than 0.009 parts by weight, less than 0.005 parts by weight, or less than 0.004 parts by weight) dissolve in 100 parts by weight of the solvent.

The term "standard conditions" as used herein refers to a temperature of 25°C and an absolute pressure of 101.325 kPa.

In a first aspect, the present invention is directed to a process for synthesizing N-(4-(4-amino-2-(2-methoxyethyl)-1H-imidazo[4,5-c]quinolin-1-yl)butyl)-N-(tetrahydro-2H-pyran-4-yl)acetamide, i.e. a compound of formula (I), a solvate or salt thereof. Within the context of the present invention, novel intermediates are generated as part of the novel synthesis process. Together, the present invention provides an improved, efficient process for the synthesis of N-(4-(4-amino-2-(2-methoxyethyl)-1H-imidazo[4,5-c]quinolin-1-yl)butyl)-N-(tetrahydro-2H-pyran-4-yl)acetamide, a solvate or salt thereof.

More specifically, the present invention relates to a process for synthesizing N-(4-(4-amino-2-(2-methoxyethyl)-1H-imidazo[4,5-c]quinolin-1-yl)butyl)-N-(tetrahydro-2H-pyran-4-yl)acetamide, a solvate or salt thereof, the process comprising the following steps:
a) providing a compound of formula (3): 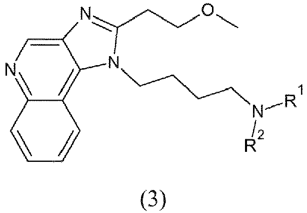 or a solvate or salt thereof, wherein each of R¹ and R² is independently selected from the group consisting of -H and an amino protecting group, and at least one of R¹ and R² is an amino protecting group;
b) reacting the compound of formula (3) or a solvate or salt thereof with one or more reagents to remove the amino protecting group(s) at position R¹ and R² to give a compound of formula (4): 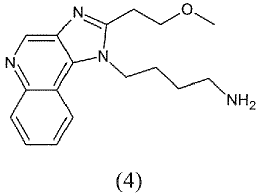 or a solvate or salt thereof;
c) reacting the compound of formula (4) or a solvate or salt thereof with one or more reagents for introducing (i) a tetrahydropyranyl group and (ii) an acetyl group to give a compound of formula (5): 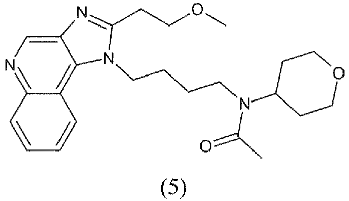 or a s solvate or salt thereof; and
d) subjecting the compound of formula (5) or a solvate or salt thereof to an oxidative amination reaction to give the compound of formula (1) or a solvate or salt thereof.

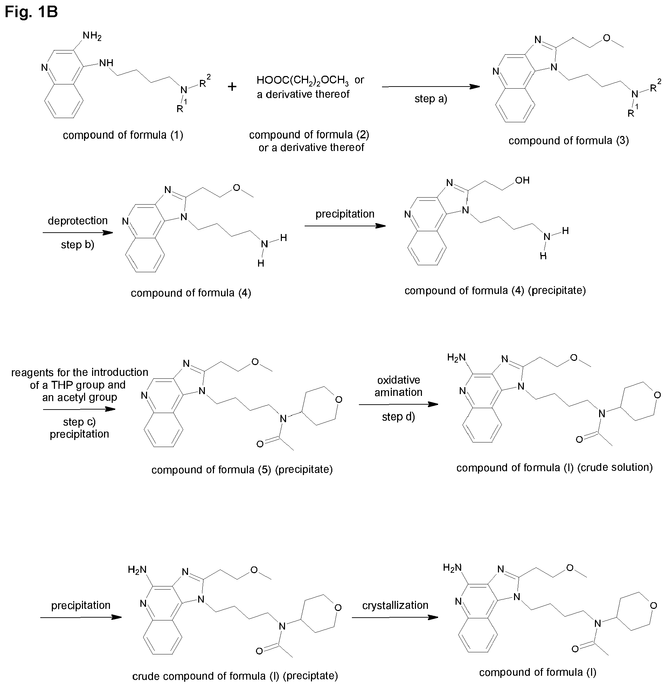

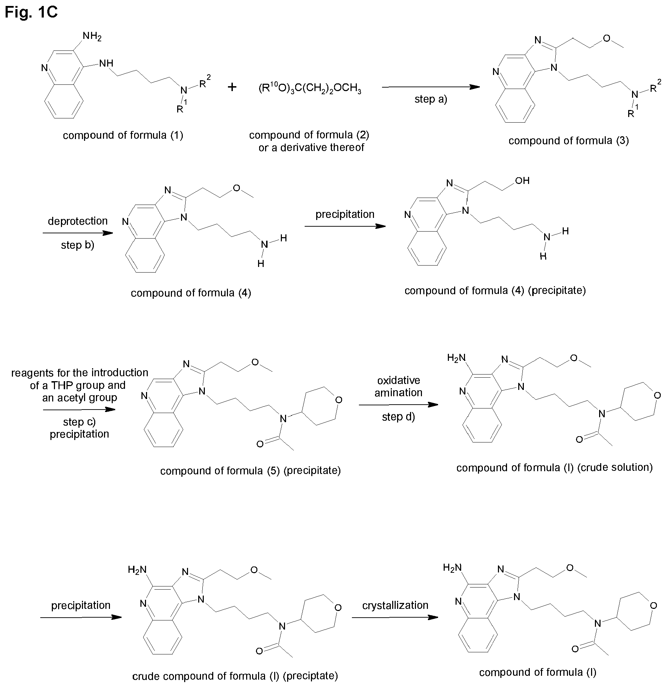

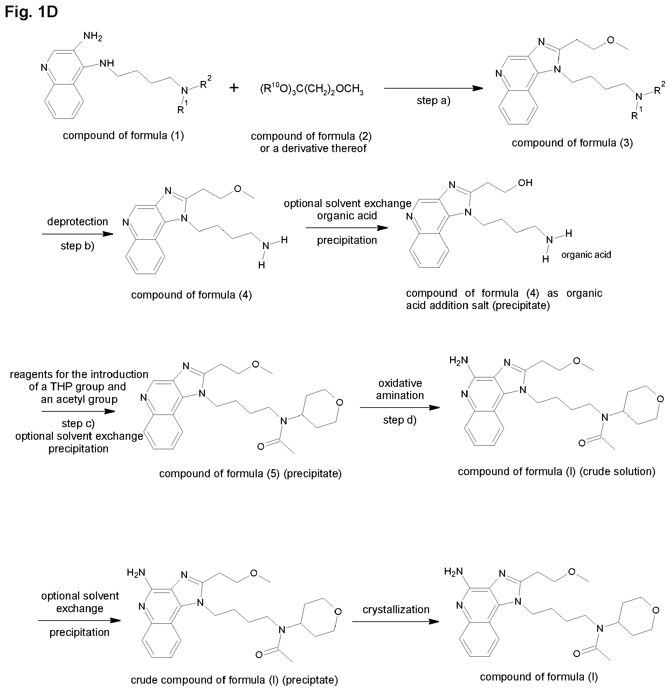

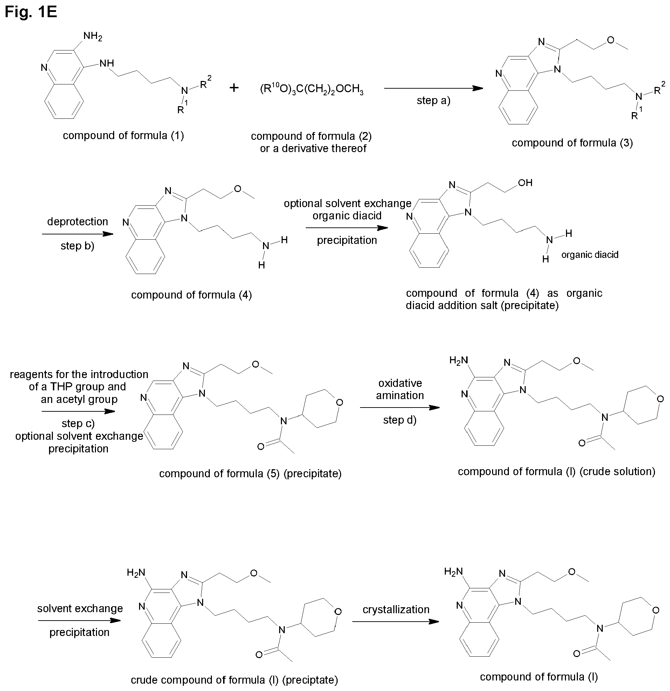

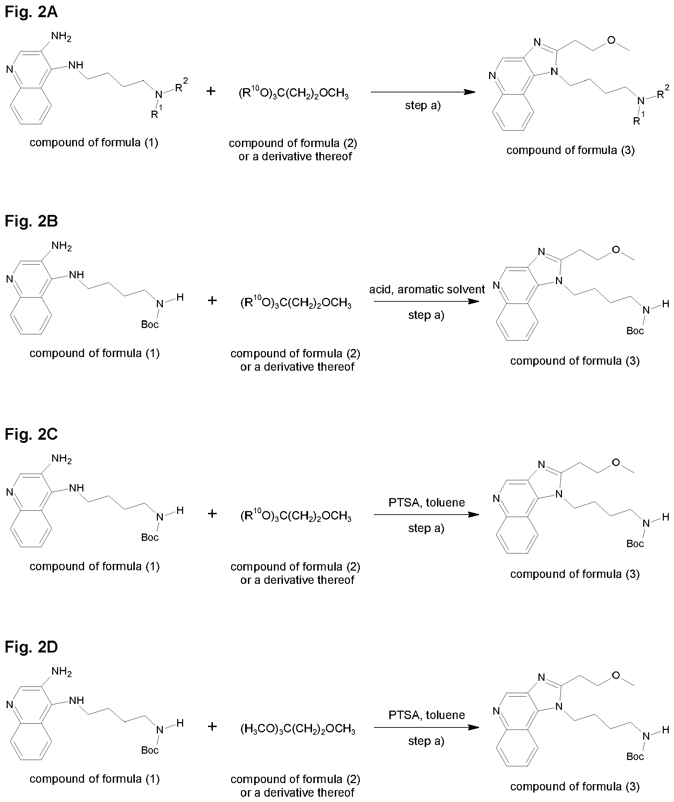

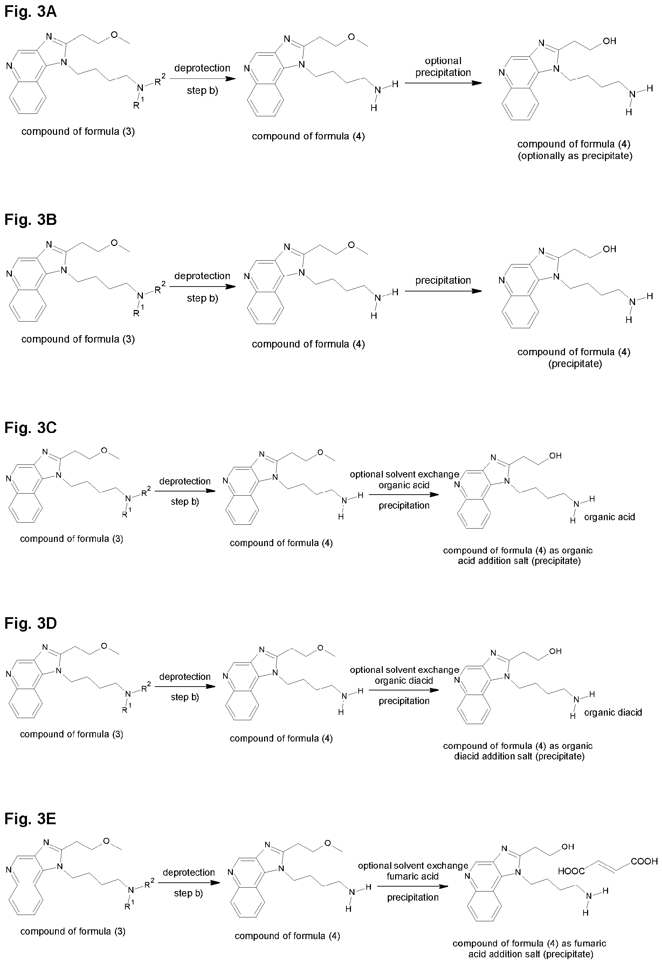

In one embodiment of the process of the first aspect (in particular of the process of the invention as specified in any one of Figures 1A, 1B, 1C, 1D, and 1E or of the process of the invention comprising (1) step a) as specified in Figure 2A; and/or (2) step b) as specified in any one of Figures 3A, 3B, 3C, 3D, and 3E) each of R¹ and R² is independently selected from the group consisting of -H, *tert-*butyloxycarbonyl (Boc), 9-fluorenylmethyloxy carbonyl (Fmoc), benzyloxycarbonyl (Cbz), tosylate (Ts), benzyl (Bn), allyloxycarbonyl (alloc), trityl (Trt), dimethoxytrityl (DMT), and monomethoxytrityl (MMT). Preferably, one of R¹ and R² is H and the other is an amino protecting group, e.g., selected from the group consisting of tert-butyloxycarbonyl (Boc), 9-fluorenylmethyloxy carbonyl (Fmoc), benzyloxycarbonyl (Cbz), tosylate (Ts), benzyl (Bn), allyloxycarbonyl (alloc), trityl (Trt), dimethoxytrityl (DMT), and monomethoxytrityl (MMT), more preferably, from the group consisting of tert-butyloxycarbonyl (Boc), 9-fluorenylmethyloxy carbonyl (Fmoc), and allyloxycarbonyl (alloc). In one particularly preferred embodiment of the process of the first aspect (in particular of the process of the invention as specified in any one of Figures 1A, 1B, 1C, 1D, and 1E or of the process of the invention comprising (1) step a) as specified in Figure 2A; and/or (2) step b) as specified in any one of Figures 3A, 3B, 3C, 3D, and 3E), one of R¹ and R² is H and the other is *tert-*butyloxycarbonyl (Boc).

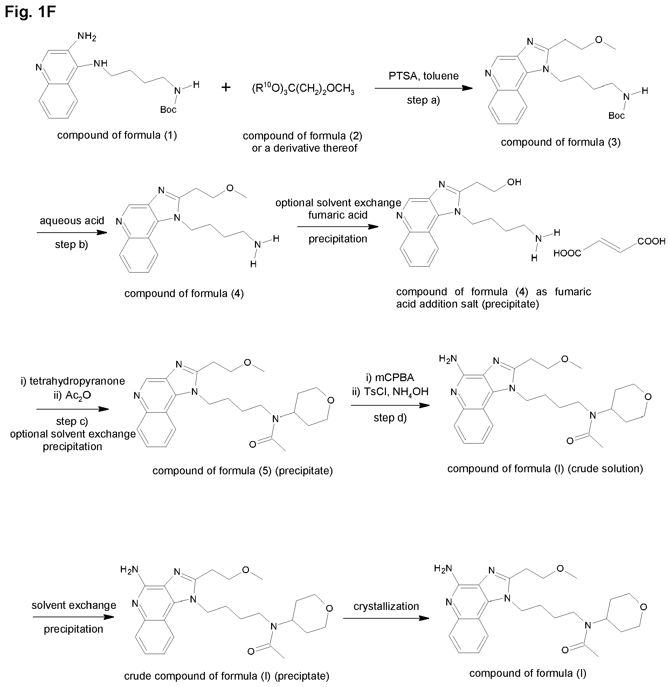

In any of the above embodiments of the process of the first aspect, step a) may comprise reacting a compound of formula (1): 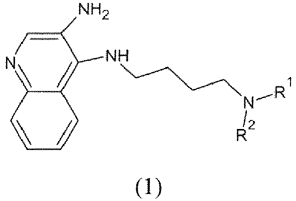 or a solvate or salt thereof with HOOC(CH₂)₂OCH₃ (formula (2)) or a derivative thereof to give a compound of formula (3) or a solvate or salt thereof. In this embodiment (such as in the process as specified in any one of Figures 1A and 1B), any derivative of formula 2 can be used as long as the condensation of said derivative with the compound of formula (1) or a solvate or salt thereof gives the compound of formula (3). Exemplary derivatives of the compound of formula (2) include, but are not limited to, carboxylic acid halides, orthoesters, and 1,1-dialkoxyalkyl alkanoates. In a preferred embodiment of the process of the first aspect, the derivative of formula (2) is an orthoester, preferably an orthoester having the formula (R¹⁰O)₃C(CH₂)₂OCH₃ (cf., e.g., any one of Figures 1C, 1D, 1E, 1F, 2A, 2B, and 2C), wherein each R¹⁰ is independently selected from the group consisting of alkyl, alkenyl, alkynyl, aryl, heteroaryl, cycloalkyl, and heterocycloalkyl, any of which is optionally substituted with one or more (such as 1 to the maximum number of hydrogen atoms bound to the alkyl, alkenyl, alkynyl, aryl, heteroaryl, cycloalkyl, or heterocycloalkyl group, e.g., 1, 2, 3, 4, 5, 6, 7, 8, 9, or up to 10, such as between 1 to 5, 1 to 4, or 1 to 3, or 1 or 2) independently selected R²⁰, the R²⁰ preferably being a 1^{st} level substituent, a 2^{nd} level substituent, or a 3^{rd} level substituent as specified herein. In one embodiment, each R¹⁰ is independently selected from the group consisting of C₁₋₁₂ alkyl, e.g., C₁₋₈ alkyl, such as methyl, ethyl, or propyl. In one embodiment, the amount of the compound of formula (2) or a derivative thereof used in the reaction with the compound of formula (1) may be 1 to 1.5 equivalents (such as 1.0 to 1.4 equivalents, 1.1 to 1.3 equivalents, 1.2 to 1.3 equivalents) relative to 1 equivalent of the compound of formula (1).

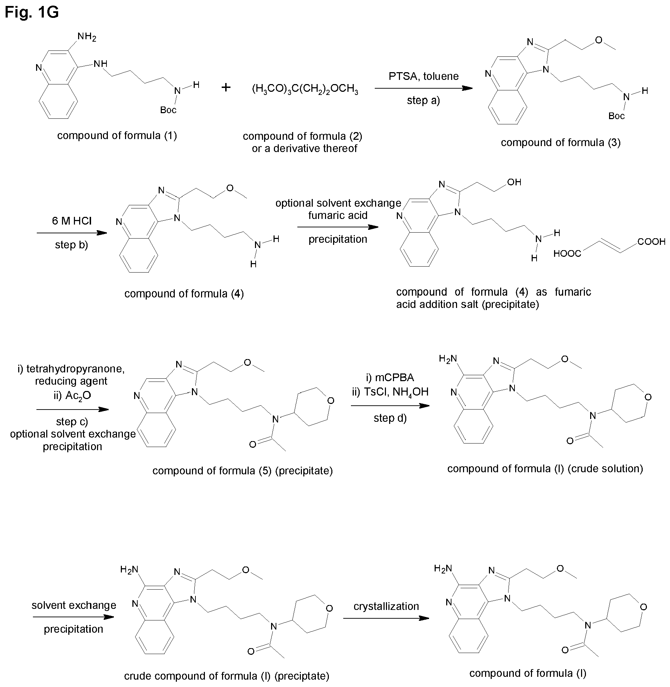

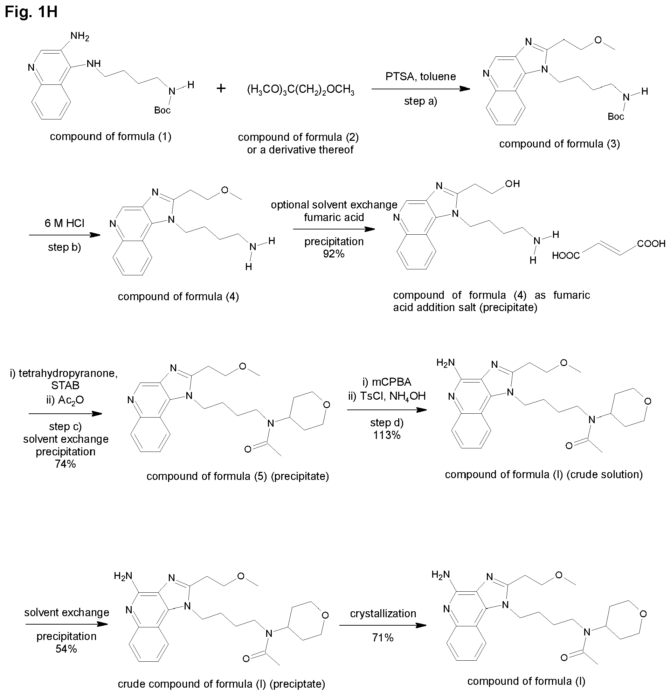

In any of the above embodiments of the process of the first aspect (in particular of the process of the invention as specified in any one of Figures 1A, 1B, 1C, 1D, 1E, 1F, 1G, and 1H or of the process of the invention comprising step a) as specified in any one of Figures 2A, 2B, 2C, and 2D), the reaction of the compound of formula (1) with the compound of formula (2) may be performed (i) in a solvent; (ii) in the presence of an acid; (iii) at a suitable temperature; and/or (iv) for a sufficient amount of time.

In any of the above embodiments of the process of the first aspect (in particular of the process of the invention as specified in any one of Figures 1A, 1B, 1C, 1D, and 1E or of the process of the invention comprising step a) as specified in Figure 2A), the reaction of the compound of formula (1) with the compound of formula (2) may be performed in a solvent, such as an aromatic solvent, preferably toluene. The amount of solvent used in this reaction may be 2 to 20 parts by volume (such as 3 to 18 parts by volume, 4 to 16 parts by volume, 6 to 14 parts by volume, 8 to 12 parts by volume, 9 to 10 parts by volume) relative to 1 part by weight of the compound of formula (1).

In any of the above embodiments of the process of the first aspect (in particular of the process of the invention as specified in any one of Figures 1A, 1B, 1C, 1D, and 1E or of the process of the invention comprising step a) as specified in Figure 2A), the reaction of the compound of formula (1) with the compound of formula (2) may be performed in the presence of an acid, such as a mineral acid or organic acid, e.g., p-toluenesulfonic acid. The amount of acid used in this reaction may be 0.01 to 0.1 equivalents (such as 0.02 to 0.09 equivalents, 0.02 to 0.08 equivalents, 0.03 to 0.07 equivalents, 0.03 to 0.06 equivalents, 0.04 to 0.05 equivalents) relative to 1 equivalent of the compound of formula (1).

In any of the above embodiments of the process of the first aspect (in particular of the process of the invention as specified in any one of Figures 1A, 1B, 1C, 1D, 1E, 1F, 1G, and 1H or of the process of the invention comprising step a) as specified in any one of Figures 2A, 2B, 2C, and 2D), the reaction of the compound of formula (1) with the compound of formula (2) may be performed at any suitable temperature, e.g., at standard conditions (e.g., at 25°C), at reduced temperature (such as between - 10°C and 10°C) or at elevated temperature (e.g., at 50°C or higher, such as at the reflux temperature of the reaction mixture containing compound of formula (1), the compound of formula (2), and/or the compound of formula (3)). An exemplary temperature range is between 0°C and 120°C, e.g., between 25°C and the reflux temperature of the solvent, if present.

In any of the above embodiments of the process of the first aspect, the reaction of the compound of formula (1) with the compound of formula (2) may be performed for a sufficient amount of time (in particular of the process of the invention as specified in any one of Figures 1A, 1B, 1C, 1D, 1E, 1F, 1G, and 1H or of the process of the invention comprising step a) as specified in any one of Figures 2A, 2B, 2C, and 2D), such as in the range of 0.5 to 10 h, preferably 1 to 9 h, more preferably 1.5 to 8 h, more preferably 2 to 7 h, more preferably 2.5 to 6 h, more preferably 3 to 5 h. Alternatively, the reaction of the compound of formula (1) with the compound of formula (2) may be performed until the reaction is substantially complete, i.e., until substantially the entire initial amount (e.g., ≥ 99.0% of the initial amount) of the compound of formula (1) is consumed.

In any of the above embodiments of the process of the first aspect (in particular of the process of the invention as specified in any one of Figures 1A, 1B, 1C, 1D, 1E, 1F, 1G, and 1H or of the process of the invention comprising step a) as specified in any one of Figures 2A, 2B, 2C, and 2D), after the reaction of the compound of formula (1) with the compound of formula (2) has been performed for a sufficient amount of time (e.g., until the reaction is substantially complete, i.e., until substantially the entire initial amount of the compound of formula (1) has been consumed), the reaction mixture may be cooled to any temperature lower than the highest temperature used in the reaction of the compound of formula (1) with the compound of formula (2), preferably to the temperature or temperature range at which the next step is to be performed.

In step b) of the process of the first aspect, the compound of formula (3) or a solvate or salt thereof is reacted with one or more reagents to remove the amino protecting group(s) at position R¹ and R² to give a compound of formula (4): 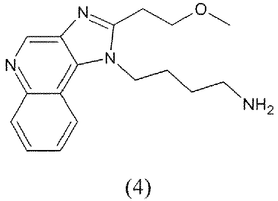 or a solvate or salt thereof. Thus, in other words, in step b) the protected primary amino group of the compound of formula (3) is deprotected in order to give the compound of formula (4) having a free primary amino group.

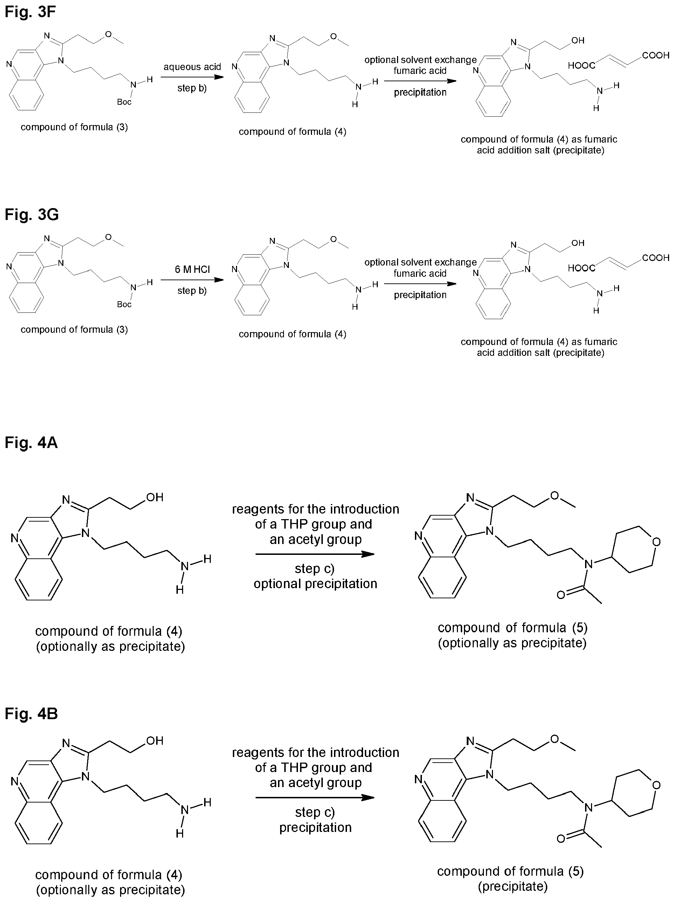

In any of the above embodiments of the process of the first aspect (in particular of the process of the invention as specified in any one of Figures 1A, 1B, 1C, 1D, 1E, 1F, 1G, and 1H or of the process of the invention comprising step b) as specified in any one of Figures 3A, 3B, 3C, 3D, 3E, 3F, and 3G), step b) may be performed (i) in a solvent; (ii) at a suitable temperature; and/or (iii) for a sufficient amount of time. The reaction conditions and the one or more reagents used for the deprotection reaction in step b) vary and mainly depend on the specific amino protecting group(s) used at position R¹ and/or R². However, the skilled person is familiar with protecting technology and, thus, knows suitable methods (including conditions (e.g., temperature, reaction time, solvent, etc.) and reagents) for the deprotection of a protected primary amino group; cf., e.g., "Greene's Protective Groups in Organic Synthesis", Peter G. M. Wuts (Ed.), 2014, John Wiley & Sons, Inc. Exemplary conditions and/or reagents for the removal of the common amino protecting groups Boc, Trt, Fmoc, Cbz, and Alloc, are as follows:
(1) Boc: addition of a strong acid (e.g., selected from the group consisting of trifluoroacetic acid (TFA; e.g., 25-50% in DCM), HCl (e.g., 4-6 M in an organic solvent), CH₃SO₃H (e.g., 2 M in dioxane), and (H₃C)₃SiCl (TMSCl; e.g., 1 M TMSCI-phenol in DCM));
(2) Trt: addition of a strong acid (e.g., TFA (e.g., 1% in DCM or 0.2%, 1% H₂O in DCM), HOBt (e.g., 0.1 M in 2,2,2-trifluoroethanol), or trichloroacetic acid (TCA; e.g., 3% in DCM));
(3) Fmoc: addition of a base, in particular a secondary amine (e.g., NH₃ (e.g., as liquid; about 10 h), morpholine or piperidine (within minutes), diethylamine (DEA; e.g., 10%), dimethylacetamide (DMA; e.g., 2 h), or polymeric (silica gel or polystyrene) secondary amines (i.e., piperazine or piperidine) in organic solvents;
(4) Cbz: catalytical hydrogenolysis or addition of a strong acid (e.g., HBr (e.g., in in acetic acid), TFA (e.g., at high temperature; TFA-thioanisole), HF (e.g., liquid), or BBr₃);
(5) Alloc: Pd-catalyzed transfer of the allyl group to a nucleophile or scavenger (e.g., Pd(PPh)₃; scavengers: H₃N·BH₃, Me₂NH·BH₃ or PhSiH₃ in organic solvents)

In any of the above embodiments of the process of the first aspect (in particular of the process of the invention as specified in any one of Figures 1A, 1B, 1C, 1D, and I E or of the process of the invention comprising step b) as specified in any one of Figures 3A, 3B, 3C, 3D, and 3E), in step b), each of R¹ and R² may be independently selected from the group consisting of -H and tert-butyloxycarbonyl (Boc) and the one or more reagents to remove the amino protecting group(s) in step b) may be selected from the group consisting of trifluoroacetic acid (TFA), HCl, CH₃SO₃H, and (H₃C)₃SiCl (TMSCl).

In any of the above embodiments of the process of the first aspect (in particular of the process of the invention as specified in any one of Figures 1A, 1B, 1C, 1D, 1E, 1F, 1G, and 1H or of the process of the invention comprising step b) as specified in any one of Figures 3A, 3B, 3C, 3D, 3E, 3F, and 3G), step b) may be performed in a solvent, such as an aromatic solvent, e.g., the solvent used in step a), preferably toluene.

In any of the above embodiments of the process of the first aspect (in particular of the process of the invention as specified in any one of Figures 1A, 1B, 1C, 1D, 1E, 1F, 1G, and 1H or of the process of the invention comprising step b) as specified in any one of Figures 3A, 3B, 3C, 3D, 3E, 3F, and 3G), step b) may be performed at any suitable temperature, e.g., at standard conditions (e.g., at 25°C), at reduced temperature (such as between -10°C and 10°C) or at elevated temperature (e.g., at 50°C or higher, such as at the reflux temperature of the reaction mixture containing the compound of formula (4), the one or more reagents to remove the amino protecting group(s) at position R¹ and R², and/or the compound of formula (4)), depending on the specific deprotection reaction used in step b), such as between 0°C and 40°C. If, for example, each of R¹ and R² is independently selected from the group consisting of -H and tert-butyloxycarbonyl (Boc) (cf., e.g., the process of the invention as specified in any one of Figures 1F, 1G, and 1H or of the process of the invention comprising step b) as specified in any one of Figures 3F and 3G), an exemplary temperature range for the deprotection reaction in step b) is between 0°C and 30°C, e.g., between 10°C and 25°C. In one embodiment of the process of the first aspect (in particular of the process of the invention as specified in any one of Figures 1A, 1B, 1C, 1D, 1E, 1F, 1G, and 1H or of the process of the invention comprising step b) as specified in any one of Figures 3A, 3B, 3C, 3D, 3E, 3F, and 3G), the amount of the one or more reagents used in step b) to remove the amino protecting group(s) at position R¹ and R² may be 1.5 to 10 equivalents (such as 2.0 to 9 equivalents, 2.5 to 8 equivalents, 3 to 7 equivalents, 3.5 to 7 equivalents, 4 to 6 equivalents) relative to 1 equivalent of the compound of formula (3).

In any of the above embodiments of the process of the first aspect (in particular of the process of the invention as specified in any one of Figures 1A, 1B, 1C, 1D, 1E, 1F, 1G, and 1H or of the process of the invention comprising step b) as specified in any one of Figures 3A, 3B, 3C, 3D, 3E, 3F, and 3G), step b) is preferably performed for a sufficient amount of time, such as in the range of 0.5 to 5 h, preferably 1 to 4.5 h, more preferably 1.5 to 4 h, more preferably 2 to 3.5 h, more preferably 2.5 to 3 h. Alternatively, step b) is performed until the reaction is substantially complete, i.e., until substantially the entire initial amount (e.g., ≥ 99.0% of the initial amount) of the compound of formula (3) is consumed.

In any of the above embodiments of the process of the first aspect, in which in step b) an acid is added (in particular in the embodiments of the process of the invention as specified in any one of Figures 1F, 1G, and 1H or in the embodiments of the process of the first aspect comprising step b) as specified in any one of Figures 3F and 3G), it is preferred that after the reaction of the compound of formula (3) with the one or more reagents to remove the amino protecting group(s) at position R¹ and R² has been performed for a sufficient amount of time (e.g., until the reaction is substantially complete, i.e., until substantially the entire initial amount of the compound of formula (3) has been consumed), a base is added, preferably in order to neutralize the acid. E.g., the base is added in such an amount to yield the compound of formula (4) having a deprotonated primary amino group (i.e., the group -NH₂ instead of the protonated group -NH₃⁺ in the presence of an acid). The base may be any base which is suitable to deprotonate a primary protonated amino group, and illustrative bases include, but are not limited to, NaOH, KOH, a carbonate salt (such as K₂CO₃ or Cs₂CO₃), an organic amine (such as a cyclic amine (e.g., 1,8-diazabicycloundec-7-ene (DBU))), an alkoxide (such sodium tert-butoxide or potassium *tert-*butoxide), an amine salt (such as lithium diisopropylamide (LDA) or lithium tetramethylpiperidide (LiTMP)), or a silicon-based amide (such as sodium bis(trimethylsilyl)amide (NaHMDS) or potassium bis(trimethylsilyl)amide (KHMDS)). It is preferred that the reaction with the base is performed at a suitable temperature (e.g., at standard conditions (e.g., at 25°C), at reduced temperature (such as between -10°C and 10°C) or at elevated temperature (e.g., at 50°C or higher), such as between 0°C and 40°C (e.g., between 0°C and 35°C or 10°C and 30°C); and/or for a sufficient amount of time (e.g., in the range of 0.5 to 5 h, preferably 1 to 4.5 h, more preferably 1.5 to 4 h, more preferably 2 to 3.5 h, more preferably 2.5 to 3 h. Alternatively, the addition of the base is continued until the deprotonation of the protonated primary amino group -NH₃⁺ of the compound of formula (4) is substantially complete, e.g., until the pH of the reaction mixture containing the compound of formula (4) is ≥ 12.

In any of the above embodiments of the process of the first aspect (in particular of the process of the invention as specified in any one of Figures 1A, 1B, 1C, 1D, 1E, 1F, 1G, and 1H or of the process of the invention comprising step b) as specified in any one of Figures 3A, 3B, 3C, 3D, 3E, 3F, and 3G), after step b) and before adding a base, the process of the present invention may further comprise the step of performing a solvent exchange. The performance of a solvent exchange before adding a base is particularly preferable for the embodiment of the process of the present invention, wherein the compound of formula (4) having a deprotonated primary amine group dissolves better in the solvent after the solvent exchange than in the solvent used in step b) (i.e., the solvent before the solvent exchange). In a preferred embodiment the solvent after the solvent exchange comprises a halogenated organic solvent (e.g., DCM), optionally in a mixture with water (e.g., a mixture of 5 parts by volume of halogenated organic solvent (e.g., DCM) and 2 parts by volume of water). In one embodiment, after the solvent exchange the reaction mixture is substantially free of the solvent used in step b) (i.e., before the solvent exchange), e.g., after the solvent exchange the reaction mixture contains ≤ 1.0 % by weight of the solvent used in step b) (i.e., before the solvent exchange).

In any of the above embodiments of the process of the first aspect (in particular of the process of the invention as specified in Figure 1A or of the process of the invention comprising step b) as specified in Figure 3A), after step b) and before step c), the process of the first aspect may further comprise the step of precipitating the compound of formula (4) or a solvate or salt thereof. In this embodiment (in particular of the process of the invention as specified in any one of Figures 1B, 1C, 1D, 1E, 1F, 1G, and 1H or of the process of the invention comprising step b) as specified in any one of Figures 3B, 3C, 3D, 3E, 3F, and 3G), the step of precipitating the compound of formula (4) or a solvate or salt thereof may be achieved by adding an acid to the compound of formula (4) or a solvate or salt thereof so that the acid addition salt of the compound of formula (4) precipitates. Optionally, a solvent exchange is performed before or after the addition of the acid in order to enhance the precipitation of the acid addition salt of the compound of formula (4). It is preferred that the solvent after the solvent exchange is one in which the compound of formula (4) having a deprotonated primary amine group is soluble. It is also preferred that after the solvent exchange the reaction mixture is substantially free of the solvent present before the solvent exchange took place, e.g., after the solvent exchange the reaction mixture contains ≤ 1.0 % by weight of the solvent present before the solvent exchange took place. In one embodiment, the acid to be added to the compound of formula (4) or a solvate or salt thereof so that the acid addition salt of the compound of formula (4) precipitates is an organic acid, e.g., an organic diacid, such as oxalic acid, malonic acid, succinic acid, glutaric acid, adipic acid, maleic acid, fumaric acid, or glutaconic acid, preferably fumaric acid. In one embodiment (in particular of the process of the invention as specified in any one of Figures 1D, 1E, 1F, 1G, and 1H or of the process of the invention comprising step b) as specified in any one of Figures 3C, 3D, 3E, 3F, and 3G), the solvent after the solvent exchange is an alcoholic solvent, e.g., methanol, ethanol, 1-propanol, or 2-propanol, preferably ethanol. It is preferred that (in particular in the embodiments of the process of the invention as specified in any one of Figures ID, 1E, 1F, 1G, and 1H or of the process of the invention comprising step b) as specified in any one of Figures 3C, 3D, 3E, 3F, and 3G) the precipitation of the acid addition salt of the compound of formula (4) is performed at a suitable temperature (e.g., between 0°C and 40°C, such as between 10°C and 30°C or between 15°C and 25°C) and/or for a sufficient amount of time (e.g., in the range of 2 to 20 h, preferably 4 to 18 h, more preferably 6 to 16 h, more preferably 8 to 14 h, more preferably 10 to 13 h). In one embodiment (in particular in the embodiments of the process of the invention as specified in any one of Figures 1D, 1E, 1F, 1G, and 1H or of the process of the invention comprising step b) as specified in any one of Figures 3C, 3D, 3E, 3F, and 3G), the precipitated acid addition salt of the compound of formula (4) is collected (e.g., by filtration), optionally washed (e.g., with a solvent in which acid addition salt of the compound of formula (4) is poorly soluble or insoluble (e.g., an ether solvent such as tert-butyl methyl ether)) and optionally dried (e.g., at a suitable temperature (such as between 30°C and 50°C, preferably between 40°C and 45°C) and/or for a sufficient amount of time (such as in the range of 4 to 20 h, e.g., 6 to 18 h, 8 to 16 h, 8 to 15 h, or 10 to 13 h)), wherein the optional washing and/or drying may be performed under an inert atmosphere (such as nitrogen).

In step c) of the process of the first aspect, the compound of formula (4) or a solvate or salt thereof is reacted with one or more reagents for introducing (i) a tetrahydropyranyl group and (ii) an acetyl group to give a compound of formula (5): 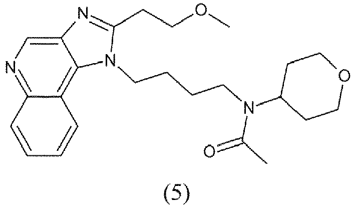 or a s solvate or salt thereof. Thus, in other words, in step c) the primary amino group is converted into a tertiary amino group bearing a tetrahydropyranyl group and an acetyl group.

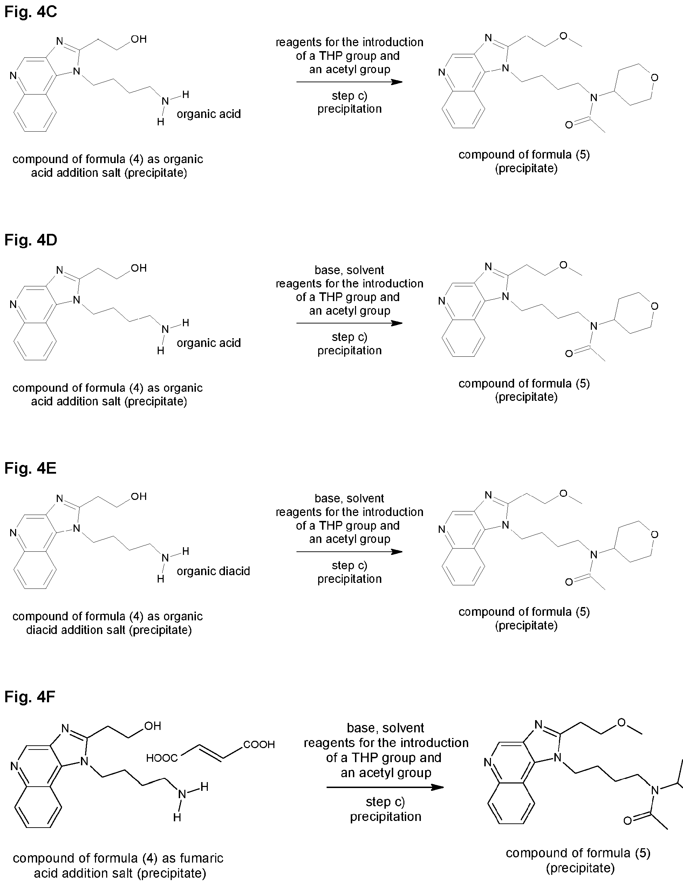

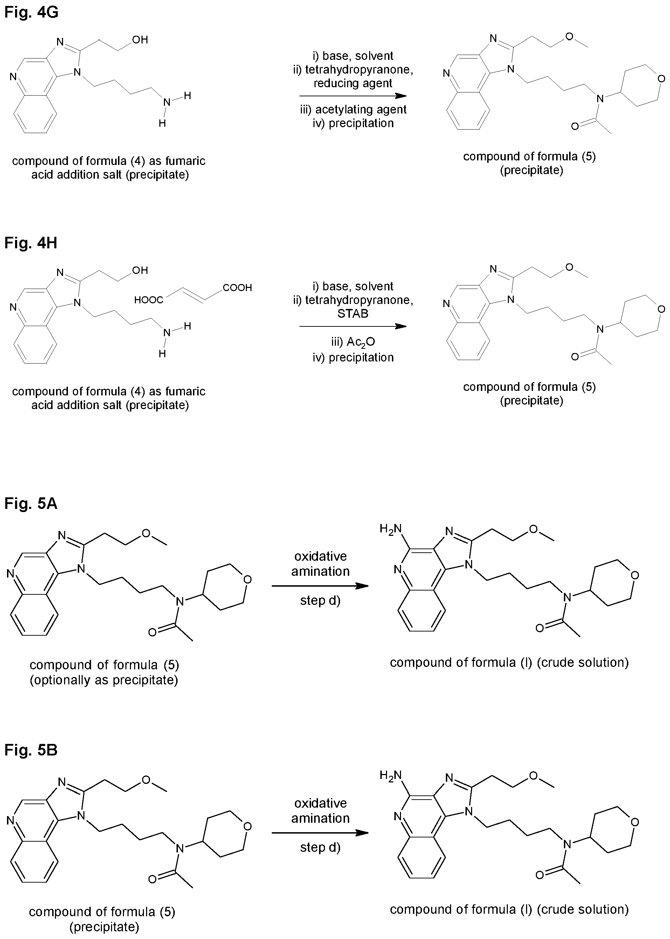

In any of the above embodiments of the process of the first aspect (in particular of the process of the invention as specified in any one of Figures 1D, 1E, 1F, 1G, and 1H or of the process of the invention comprising step c) as specified in any one of Figures 4C, 4D, 4E, 4F, 4G, and 4H), wherein in step c) the compound of formula (4) is the form of an acid addition salt, it is preferred that before the reaction with one or more reagents for introducing a tetrahydropyranyl group and an acetyl group, the acid addition salt the compound of formula (4) is converted into the compound of formula (4) having a deprotonated primary amine group. To this end, the compound of formula (4) in the form of an acid addition salt may be dissolved in a solvent in which the acid addition salt is soluble (e.g., a halogenated organic solvent, such as DCM), an aqueous base (e.g., an aqueous base) is added, and the organic phase containing the compound of formula (4) having a deprotonated primary amine group is collected. The base may be any base which is suitable to deprotonate a primary protonated amino group, and illustrative bases include, but are not limited to, NaOH, KOH, a carbonate salt (such as K₂CO₃ or Cs₂CO₃), an organic amine (such as a cyclic amine (e.g., 1,8-diazabicycloundec-7-ene (DBU))), an alkoxide (such sodium tert-butoxide or potassium tert-butoxide), an amine salt (such as lithium diisopropylamide (LDA) or lithium tetramethylpiperidide (LiTMP)), or a silicon-based amide (such as sodium bis(trimethylsilyl)amide (NaHMDS) or potassium bis(trimethylsilyl)amide (KHMDS)). It is preferred that the reaction with the base is performed at a suitable temperature (e.g., at standard conditions (e.g., at 25°C), at reduced temperature (such as between -10°C and 10°C) or at elevated temperature (e.g., at 50°C or higher), such as between 0°C and 40°C (e.g., between 0°C and 35°C or 10°C and 30°C); and/or for a sufficient amount of time (e.g., in the range of 0.5 to 5 h, preferably 1 to 4.5 h, more preferably 1.5 to 4 h, more preferably 2 to 3.5 h, more preferably 2.5 to 3 h). Alternatively, the addition of the base is continued until the deprotonation of the protonated primary amino group -NH₃⁺ of the compound of formula (4) is substantially complete, e.g., until the pH of the reaction mixture containing the compound of formula (4) is ≥ 12.

In any of the above embodiments of the process of the first aspect (in particular of the process of the invention as specified in any one of Figures 1A, 1B, 1C, 1D, and 1E or of the process of the invention comprising step c) as specified in any one of Figures 4A, 4B, 4C, 4D, 4E, and 4F), the one or more reagents for introducing a tetrahydropyranyl group in step c) may be tetrahydropyranone and a reducing agent. The reducing agent can be any reducing agent suitable for performing a reductive amination reaction, e.g., a borohydride such as sodium triacetoxyborohydride (STAB). In one embodiment of the process of the first aspect (in particular of the process of the invention as specified in any one of Figures 1A, 1B, 1C, 1D, 1E, 1F, 1G, and 1H or of the process of the invention comprising step c) as specified in any one of Figures 4A, 4B, 4C, 4D, 4E, 4F, 4G, and 4H), the amount of tetrahydropyranone is 0.90 to 1.5 equivalents (such as 0.95 to 1.4 equivalents, 1.00 to 1.3 equivalents, 1.05 to 1.25 equivalents, 1.1 to 1.20 equivalents) relative to 1 equivalent of the compound of formula (4). It is preferred that the amount of reducing agent is in the range of 1.0 to 2.5 equivalents (such as 1.1 to 2.4 equivalents, 1.2 to 2.3 equivalents, 1.3 to 2.2 equivalents, 1.4 to 2.1 equivalents, 1.5 to 2.0 equivalents, 1.6 to 1.9 equivalents, 1.7 to 1.8 equivalents) relative to 1 equivalent of the compound of formula (4).

In any of the above embodiments of the process of the first aspect (in particular of the process of the invention as specified in any one of Figures 1A, 1B, 1C, 1D, 1E, 1F, 1G, and 1H or of the process of the invention comprising step c) as specified in any one of Figures 4A, 4B, 4C, 4D, 4E, 4F, 4G, and 4H), in step c) the reaction of the compound of formula (4) or a solvate or salt thereof with one or more reagents for introducing a tetrahydropyranyl group may be performed (i) in a solvent; (ii) in the presence of an acid; (iii) at a suitable temperature; and/or (iv) for a sufficient amount of time.

In any of the above embodiments of the process of the first aspect (in particular of the process of the invention as specified in any one of Figures 1A, 1B, 1C, 1D, 1E, 1F, 1G, and 1H or of the process of the invention comprising step c) as specified in any one of Figures 4A, 4B, 4C, 4D, 4E, 4F, 4G, and 4H), in step c) the reaction of the compound of formula (4) or a solvate or salt thereof with one or more reagents for introducing a tetrahydropyranyl group may be performed in a solvent, in particular in a solvent in which the compound of formula (4) or a solvate or salt thereof is soluble (e.g., a halogenated organic solvent, such as DCM). The amount of solvent used in this reaction may be 2 to 20 parts by volume (such as 3 to 18 parts by volume, 4 to 16 parts by volume, 6 to 14 parts by volume, 8 to 12 parts by volume, 9 to 10 parts by volume) relative to 1 part by weight of the compound of formula (4).

In any of the above embodiments of the process of the first aspect (in particular of the process of the invention as specified in any one of Figures 1A, 1B, 1C, 1D, 1E, IF, 1G, and 1H or of the process of the invention comprising step c) as specified in any one of Figures 4A, 4B, 4C, 4D, 4E, 4F, 4G, and 4H), in step c) the reaction of the compound of formula (4) or a solvate or salt thereof with one or more reagents for introducing a tetrahydropyranyl group may be performed in the presence of an acid, such as a mineral acid or organic acid, e.g., acetic acid. The amount of acid used in this reaction may be 0.85 to 1.4 equivalents (such as 0.90 to 1.3 equivalents, 0.95 to 1.2 equivalents, 1.00 to 1.1 equivalents) relative to 1 equivalent of the compound of formula (4).

In any of the above embodiments of the process of the first aspect (in particular of the process of the invention as specified in any one of Figures 1A, 1B, 1C, 1D, 1E, 1F, 1G, and 1H or of the process of the invention comprising step c) as specified in any one of Figures 4A, 4B, 4C, 4D, 4E, 4F, 4G, and 4H), in step c) the reaction of the compound of formula (4) or a solvate or salt thereof with one or more reagents for introducing a tetrahydropyranyl group may be performed at a suitable temperature, e.g., at standard conditions (e.g., at 25°C), at reduced temperature (such as between -10°C and 10°C) or at elevated temperature (e.g., at 50°C or higher). An exemplary temperature range is between -20°C and 40°C (e.g., between -15°C and 30°C or between -10°C and 25°C).

In any of the above embodiments of the process of the first aspect (in particular of the process of the invention as specified in any one of Figures 1A, 1B, 1C, 1D, 1E, 1F, 1G, and 1H or of the process of the invention comprising step c) as specified in any one of Figures 4A, 4B, 4C, 4D, 4E, 4F, 4G, and 4H), in step c) the reaction of the compound of formula (4) or a solvate or salt thereof with one or more reagents for introducing a tetrahydropyranyl group may be performed for a sufficient amount of time, such as in the range of 5 h to 24 h, e.g., 7 h to 23 h, 9 h to 21 h, 11 h to 20 h, 13 h to 19 h, or 15 h to 18 h. Alternatively, the reaction of the compound of formula (4) or a solvate or salt thereof with one or more reagents for introducing a tetrahydropyranyl group may be performed until the reaction is substantially complete, i.e., until substantially the entire initial amount (e.g., ≥ 99.5 % of the initial amount) of the compound of formula (4) is consumed.

In any of the above embodiments of the process of the first aspect (in particular of the process of the invention as specified in any one of Figures 1A, 1B, 1C, 1D, 1E, 1F, 1G, and 1H or of the process of the invention comprising step c) as specified in any one of Figures 4A, 4B, 4C, 4D, 4E, 4F, 4G, and 4H), it is preferred that after the compound of formula (4) or a solvate or salt thereof has been reacted with one or more reagents for introducing a tetrahydropyranyl group (e.g., after said reaction is substantially complete), a base (e.g., an aqueous base (such as aqueous NaOH) is added to quench the reaction. Thereafter, the organic phase may be separated from the aqueous phase, optionally washed, dried using a solid drying agent (such as solid MgSO₄), and/or concentrated (e.g., under reduced pressure).

In any of the above embodiments of the process of the first aspect (in particular of the process of the invention as specified in any one of Figures 1A, 1B, 1C, 1D, and 1E, or of the process of the invention comprising step c) as specified in any one of Figures 4A, 4B, 4C, 4D, 4E, 4F, and 4G), the one or more reagents for introducing an acetyl group in step c) may be selected from acetic anhydride and acetic halides. In one embodiment, the amount of the one or more reagents for introducing an acetyl group is 0.80 to 1.5 equivalents (such as 0.85 to 1.4 equivalents, 0.90 to 1.3 equivalents, 0.95 to 1.2 equivalents, 1.00 to 1.1 equivalents) relative to 1 equivalent of the compound of formula (4).

In any of the above embodiments of the process of the first aspect (in particular of the process of the invention as specified in any one of Figures 1A, 1B, 1C, 1D, 1E, 1F, 1G, and 1H or of the process of the invention comprising step c) as specified in any one of Figures 4A, 4B, 4C, 4D, 4E, 4F, 4G, and 4H), in step c) the reaction of the compound of formula (4) or a solvate or salt thereof with one or more reagents for introducing an acetyl group may be performed (i) in a solvent; (ii) at a suitable temperature; and/or (iii) for a sufficient amount of time.

In any of the above embodiments of the process of the first aspect (in particular of the process of the invention as specified in any one of Figures 1A, 1B, 1C, 1D, 1E, 1F, 1G, and 1H or of the process of the invention comprising step c) as specified in any one of Figures 4A, 4B, 4C, 4D, 4E, 4F, 4G, and 4H), in step c) the reaction of the compound of formula (4) or a solvate or salt thereof with one or more reagents for introducing an acetyl group may be performed in a solvent, in particular in a solvent in which the compound of formula (4) or a solvate or salt thereof is soluble (e.g., the solvent used in the reaction for introducing a tetrahydropyranyl group, such as a halogenated organic solvent, e.g., DCM). The amount of solvent used in this reaction may be 2 to 20 parts by volume (such as 3 to 18 parts by volume, 4 to 16 parts by volume, 6 to 14 parts by volume, 8 to 12 parts by volume, 9 to 10 parts by volume) relative to 1 part by weight of the compound of formula (4).

In any of the above embodiments of the process of the first aspect (in particular of the process of the invention as specified in any one of Figures 1A, 1B, 1C, 1D, 1E, 1F, 1G, and 1H or of the process of the invention comprising step c) as specified in any one of Figures 4A, 4B, 4C, 4D, 4E, 4F, 4G, and 4H), in step c) the reaction of the compound of formula (4) or a solvate or salt thereof with one or more reagents for introducing an acetyl group may be performed at a suitable temperature, e.g., at standard conditions (e.g., at 25°C), at reduced temperature (such as between -10°C and 10°C) or at elevated temperature (e.g., at 50°C or higher). An exemplary temperature range is between 0°C and 50°C (e.g., between 10°C and 40°C or between 20°C and 30°C).

In any of the above embodiments of the process of the first aspect (in particular of the process of the invention as specified in any one of Figures 1A, 1B, 1C, 1D, 1E, 1F, 1G, and 1H or of the process of the invention comprising step c) as specified in any one of Figures 4A, 4B, 4C, 4D, 4E, 4F, 4G, and 4H), in step c) the reaction of the compound of formula (4) or a solvate or salt thereof with one or more reagents for introducing an acetyl group may be performed for a sufficient amount of time, such as in the range of 6 h to 48 h, e.g., 8 h to 46 h, 10 h to 44 h, 12 h to 42 h, 14 h to 40 h, 16 h to 38 h, 18 h to 36 h, 20 h to 34, 22 h to 32, or 24 h to 30 h. Alternatively, the reaction of the compound of formula (4) or a solvate or salt thereof with one or more reagents for introducing an acetyl group may be performed until the reaction is substantially complete, i.e., until substantially the entire initial amount (e.g., ≥ 99.5 % of the initial amount) of the compound of formula (4) is consumed.

It is to be understood that in any of the above embodiments of the process of the first aspect (in particular of the process of the invention as specified in any one of Figures 1A, 1B, 1C, 1D, 1E, 1F, 1G, and 1H or of the process of the invention comprising step c) as specified in any one of Figures 4A, 4B, 4C, 4D, 4E, 4F, 4G, and 4H), the compound of formula (4) or a solvate or salt thereof may be reacted first with one or more reagents for introducing a tetrahydropyranyl group and then with one or more reagents for introducing an acetyl group. Alternatively, in any of the above embodiments of the process of the first aspect (in particular of the process of the invention as specified in any one of Figures 1A, 1B, 1C, 1D, 1E, 1F, 1G, and 1H or of the process of the invention comprising step c) as specified in any one of Figures 4A, 4B, 4C, 4D, 4E, 4F, 4G, and 4H), the compound of formula (4) or a solvate or salt thereof may be reacted first with one or more reagents for introducing an acetyl group and then with one or more reagents for introducing a tetrahydropyranyl group. Thus, in one mode of any of the above embodiments of the process of the first aspect (in particular of the process of the invention as specified in any one of Figures 1A, 1B, 1C, 1D, 1E, 1F, 1G, and 1H or of the process of the invention comprising step c) as specified in any one of Figures 4A, 4B, 4C, 4D, 4E, 4F, 4G, and 4H), the compound of formula (4) or a solvate or salt thereof is reacted first with one or more reagents for introducing an acetyl group and with one or more reagents for introducing a tetrahydropyranyl group. However, in an alternative and preferred mode of any of the above embodiments of the process of the first aspect (in particular of the process of the invention as specified in any one of Figures 1A, 1B, 1C, 1D, 1E, 1F, 1G, and 1H or of the process of the invention comprising step c) as specified in any one of Figures 4A, 4B, 4C, 4D, 4E, 4F, 4G, and 4H), the compound of formula (4) or a solvate or salt thereof is reacted first with one or more reagents for introducing a tetrahydropyranyl group and then with one or more reagents for introducing an acetyl group.

In any of the above embodiments of the process of the first aspect (in particular of the process of the invention as specified in any one of Figures 1A, 1B, 1C, 1D, 1E, 1F, 1G, and 1H or of the process of the invention comprising step c) as specified in any one of Figures 4A, 4B, 4C, 4D, 4E, 4F, 4G, and 4H), it is preferred that after step c), a base (preferably, an aqueous base, such an aqueous solution of a carbonate (e.g., Na₂CO₃, K₂CO₃, NaHCO₃, or KHCO₃)) is added, the organic phase may be separated from the aqueous phase, optionally washed, dried using a solid drying agent (such as solid MgSO₄), and/or concentrated (e.g., under reduced pressure and at a temperature of ≤ 30°C).

In any of the above embodiments of the process of the first aspect (in particular of the process of the invention as specified in Figure 1A or of the process of the invention comprising step c) as specified in Figure 4A), after step c) and before step d), the process of the first aspect may further comprise the step of precipitating the compound of formula (5) or a solvate or salt thereof. In this embodiment (in particular of the process of the invention as specified in any one of Figures 1B, 1C, 1D, 1E, 1F, 1G, and 1H or of the process of the invention comprising step c) as specified in any one of Figures 4B, 4C, 4D, 4E, 4F, 4G, and 4H), the step of precipitating the compound of formula (5) or a solvate or salt thereof may be achieved by performing a solvent exchange. It is preferred that the solvent after the solvent exchange is one in which the compound of formula (5) or a solvate or salt is poorly soluble or insoluble. It is also preferred that after the solvent exchange the reaction mixture is substantially free of the solvent present before the solvent exchange took place, e.g., after the solvent exchange the reaction mixture contains ≤ 10% by weight (preferably ≤ 1.0% by weight) of the solvent present before the solvent exchange took place. In one embodiment (in particular of the process of the invention as specified in any one of Figures 1B, 1C, 1D, 1E, 1F, 1G, and 1H or of the process of the invention comprising step c) as specified in any one of Figures 4B, 4C, 4D, 4E, 4F, 4G, and 4H), the solvent after the solvent exchange is an ether solvent, such as *tert*-butyl methyl ether. It is preferred (in particular in the embodiments of the process of the invention as specified in any one of Figures 1B, 1C, 1D, 1E, 1F, 1G, and 1H or in the embodiments of the process of the invention comprising step c) as specified in any one of Figures 4B, 4C, 4D, 4E, 4F, 4G, and 4H)) that the precipitation of the compound of formula (5) or a solvate or salt is performed at a suitable temperature (e.g., between 0°C and 40°C, preferably between 10°C and 30°C or between 15°C and 25°C) and/or for a sufficient amount of time (e.g., in the range of 0.5 h to 5 h, preferably 1 to 4 h, more preferably 2 to 3 h). In one embodiment (in particular of the process of the invention as specified in any one of Figures 1B, 1C, 1D, 1E, 1F, 1G, and 1H or of the process of the invention comprising step c) as specified in any one of Figures 4B, 4C, 4D, 4E, 4F, 4G, and 4H), the precipitated compound of formula (5) or a solvate or salt is collected (e.g., by filtration), optionally washed (e.g., with a solvent in which acid addition salt of the compound of formula (5) or a solvate or salt is poorly soluble or insoluble (e.g., an ether solvent such as *tert*-butyl methyl ether)) and optionally dried (e.g., at a suitable temperature (such as between 30°C and 50°C, preferably between 40°C and 45°C) and/or for a sufficient amount of time (such as in the range of 4 to 20 h, e.g., 6 to 18 h, 8 to 16 h, 8 to 15 h, or 10 to 13 h)), wherein the optional washing and/or drying may be performed under an inert atmosphere (such as nitrogen).

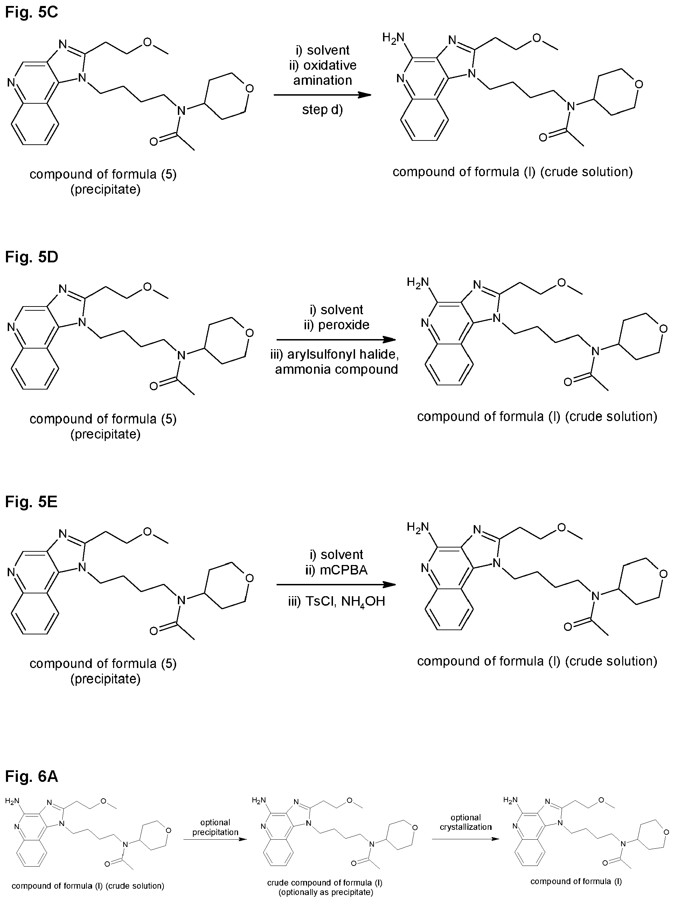

In step d) of the process of the first aspect, the compound of formula (5) or a solvate or salt thereof is subjected to an oxidative amination reaction to give the compound of formula (1) or a solvate or salt thereof. In any of the above embodiments of the process of the first aspect (in particular of the process of the invention as specified in any one of Figures 1A, 1B, 1C, 1D, 1E, 1F, 1G, and 1H or of the process of the invention comprising step d) as specified in any one of Figures 5A, 5B, 5C, 5D, and 5E), step d) may comprise reacting the compound of formula (5) or a solvate or salt thereof with first an oxidation agent (in order to prepare an N-oxide intermediate), optionally adding a base (preferably in order to deprotonate the N-oxide intermediate) and then reacting the N-oxide intermediate with an acylating agent and an aminating agent. In one embodiment, the oxidation agent is selected from oxidation agents (such as peroxides) capable of forming N-oxides. One suitable oxidation agent is 3-chloroperoxybenzoic acid (also called mCPBA). In one embodiment (in particular of the process of the invention as specified in any one of Figures 1A, 1B, 1C, 1D, 1E, 1F, 1G, and 1H or of the process of the invention comprising step d) as specified in any one of Figures 5A, 5B, 5C, 5D, and 5E), the amount of the oxidation agent is in the range of 1.0 to 2.0 equivalents (such as 1.1 to 1.9 equivalents, 1.2 to 1.8 equivalents, 1.3 to 1.7 equivalents, 1.4 to 1.6 equivalents) relative to 1 equivalent of the compound of formula (5).

In any of the above embodiments of the process of the first aspect (in particular of the process of the invention as specified in any one of Figures 1A, 1B, 1C, 1D, 1E, 1F, 1G, and 1H or of the process of the invention comprising step d) as specified in any one of Figures 5A, 5B, 5C, 5D, and 5E), the oxidation reaction of the compound of formula (5) or a solvate or salt thereof with an oxidation agent may be performed (i) in a solvent; (ii) at a suitable temperature; and/or (iii) for a sufficient amount of time.

In any of the above embodiments of the process of the first aspect (in particular of the process of the invention as specified in any one of Figures 1A, 1B, 1C, 1D, 1E, 1F, 1G, and 1H or of the process of the invention comprising step d) as specified in any one of Figures 5A, 5B, 5C, 5D, and 5E), in step d) the oxidation reaction of the compound of formula (5) or a solvate or salt thereof with an oxidation agent may be performed in a solvent, in particular in a solvent in which the compound of formula (5) or a solvate or salt thereof is soluble (e.g., a halogenated organic solvent, such as DCM). The amount of solvent used in this reaction may be 2 to 20 parts by volume (such as 3 to 18 parts by volume, 4 to 16 parts by volume, 6 to 14 parts by volume, 8 to 12 parts by volume, 9 to 10 parts by volume) relative to 1 part by weight of the compound of formula (5).

In any of the above embodiments of the process of the first aspect (in particular of the process of the invention as specified in any one of Figures 1A, 1B, 1C, 1D, 1E, 1F, 1G, and 1H or of the process of the invention comprising step d) as specified in any one of Figures 5A, 5B, 5C, 5D, and 5E), in step d) the oxidation reaction of the compound of formula (5) or a solvate or salt thereof with an oxidation agent may be performed at a suitable temperature, e.g., at standard conditions (e.g., at 25°C), at reduced temperature (such as between -20°C and 10°C) or at elevated temperature (e.g., at 50°C or higher). An exemplary temperature range is between -10°C and 40°C (e.g., between 0°C and 30°C or between 10°C and 25°C).

In any of the above embodiments of the process of the first aspect (in particular of the process of the invention as specified in any one of Figures 1A, 1B, 1C, 1D, 1E, 1F, 1G, and 1H or of the process of the invention comprising step d) as specified in any one of Figures 5A, 5B, 5C, 5D, and 5E), in step d) the oxidation reaction of the compound of formula (5) or a solvate or salt thereof with an oxidation agent may be performed for a sufficient amount of time, e.g., for a amount of time in the range of 0.5 h to 12 h, e.g., 1 h to 11 h, 2 h to 10 h, 3 h to 9 h, 4 h to 8 h, or 5 h to 7 h. Alternatively, the oxidation reaction of the compound of formula (5) or a solvate or salt thereof with an oxidation agent may be performed until the reaction is substantially complete, i.e., until substantially the entire initial amount (e.g., > 96% of the initial amount) of the compound of formula (5) is consumed.

In any of the above embodiments of the process of the first aspect (in particular of the process of the invention as specified in any one of Figures 1A, 1B, 1C, 1D, and 1E or of the process of the invention comprising step d) as specified in any one of Figures 5A, 5B, and 5C), in step d) the acylating agent may be selected from the group consisting of alkysulfonyl halides and arylsulfonyl halides. Exemplary acylating agents which can be used in this respect include methanesulfonyl chloride, benzenesulfonyl chloride, and p-toluenesulfonyl chloride. In one embodiment (in particular of the process of the invention as specified in any one of Figures 1A, 1B, 1C, 1D, 1E, 1F, 1G, and 1H or of the process of the invention comprising step d) as specified in any one of Figures 5A, 5B, 5C, 5D, and 5E), the amount of acylating agent used is in the range of 1.0 to 2.0 equivalents (such as 1.1 to 1.9 equivalents, 1.2 to 1.8 equivalents, 1.3 to 1.7 equivalents, 1.4 to 1.6 equivalents) relative to 1 equivalent of the N-oxide intermediate of the compound of formula (5).

In any of the above embodiments of the process of the first aspect (in particular of the process of the invention as specified in any one of Figures 1A, 1B, 1C, 1D, and 1E or of the process of the invention comprising step d) as specified in any one of Figures 5A, 5B, and 5C), in step d) the aminating agent may be selected from the group consisting of ammonia and its salts, such as aqueous NH₃, ammonium hydroxide, ammonium carbonate, ammonium bicarbonate, or ammonium phosphate. In one embodiment (in particular of the process of the invention as specified in any one of Figures 1A, 1B, 1C, 1D, 1E, 1F, 1G, and 1H or of the process of the invention comprising step d) as specified in any one of Figures 5A, 5B, 5C, 5D, and 5E), the amount of aminating agent used is in the range of 1.0 to 4.0 equivalents (such as 1.5 to 3.5 equivalents, 2.0 to 3.0 equivalents) relative to 1 equivalent of the N-oxide intermediate of the compound of formula (5).

In any of the above embodiments of the process of the first aspect (in particular of the process of the invention as specified in any one of Figures 1A, 1B, 1C, 1D, 1E, 1F, 1G, and 1H or of the process of the invention comprising step d) as specified in any one of Figures 5A, 5B, 5C, 5D, and 5E), in step d) the reaction of the N-oxide intermediate of the compound of formula (5) with an acylating agent and an aminating agent may be performed (i) in a solvent; (ii) at a suitable temperature; and/or (iii) for a sufficient amount of time.

In any of the above embodiments of the process of the first aspect (in particular of the process of the invention as specified in any one of Figures 1A, 1B, 1C, 1D, 1E, 1F, 1G, and 1H or of the process of the invention comprising step d) as specified in any one of Figures 5A, 5B, 5C, 5D, and 5E), in step d) the reaction of the N-oxide intermediate of the compound of formula (5) with an acylating agent and an aminating agent may be performed in a solvent, in particular in a solvent in which the N-oxide intermediate of the compound of formula (5) is soluble (e.g., a halogenated organic solvent, such as DCM). The amount of solvent used in this reaction may be 2 to 20 parts by volume (such as 3 to 18 parts by volume, 4 to 16 parts by volume, 6 to 14 parts by volume, 8 to 12 parts by volume, 9 to 10 parts by volume) relative to 1 part by weight of the compound of formula (5) or of the N-oxide intermediate of the compound of formula (5).

In any of the above embodiments of the process of the first aspect (in particular of the process of the invention as specified in any one of Figures 1A, 1B, 1C, 1D, 1E, 1F, 1G, and 1H or of the process of the invention comprising step d) as specified in any one of Figures 5A, 5B, 5C, 5D, and 5E), in step d) the reaction of the N-oxide intermediate of the compound of formula (5) with an acylating agent and an aminating agent may be performed at a suitable temperature, e.g., at standard conditions (e.g., at 25°C), at reduced temperature (such as between -20°C and 10°C) or at elevated temperature (e.g., at 50°C or higher). An exemplary temperature range is between -10°C and 40°C (e.g., between 0°C and 30°C or between 10°C and 25°C).

In any of the above embodiments of the process of the first aspect (in particular of the process of the invention as specified in any one of Figures 1A, 1B, 1C, 1D, 1E, 1F, 1G, and 1H or of the process of the invention comprising step d) as specified in any one of Figures 5A, 5B, 5C, 5D, and 5E), in step d) the reaction of the N-oxide intermediate of the compound of formula (5) with an acylating agent and an aminating agent may be performed for a sufficient amount of time, e.g., for an amount of time in the range of 0.5 h to 12 h, e.g., 1 h to 11 h, 2 h to 10 h, 3 h to 9 h, 4 h to 8 h, or 5 h to 7 h. Alternatively, the reaction of the N-oxide intermediate of the compound of formula (5) with an acylating agent and an aminating agent may be performed until the reaction is substantially complete, i.e., until substantially the entire initial amount (e.g., ≥ 99.5% of the initial amount) of the compound of formula (5) or of the N-oxide intermediate of the compound of formula (5) is consumed.

In any of the above embodiments of the process of the first aspect (in particular of the process of the invention as specified in any one of Figures 1A, 1B, 1C, 1D, 1E, 1F, 1G, and 1H or of the process of the invention comprising step d) as specified in any one of Figures 5A, 5B, 5C, 5D, and 5E), after step d), i.e., after the reaction of the N-oxide intermediate of the compound of formula (5) with an acylating agent and an aminating agent has taken place, the organic phase containing the compound of formula (I) may be separated from the aqueous phase, optionally washed, dried using a solid drying agent (such as solid MgSOft, and/or concentrated (e.g., under reduced pressure).

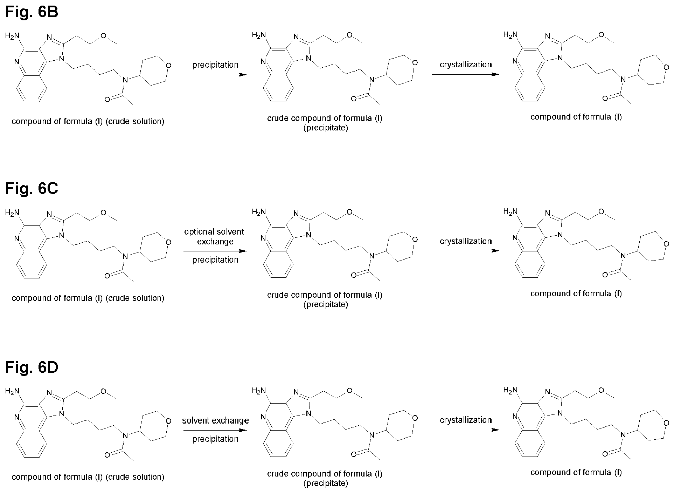

In any of the above embodiments of the process of the first aspect (in particular of the process of the invention as specified in Figure 1A or of the process of the invention comprising steps as specified in Figure 6A), after step d) the process of the first aspect may further comprise the step of precipitating the compound of formula (1) or a solvate or salt thereof. In this embodiment (in particular of the process of the invention as specified in any one of Figures 1B, 1C, and 1D or of the process of the invention comprising the steps as specified in any one of Figures 6B and 6C), the step of precipitating the compound of formula (5) or a solvate or salt thereof may be achieved by performing a solvent exchange. It is preferred (in particular in the embodiments of the process of the invention as specified in any one of Figures 1E, 1F, 1G, and 1H or in the embodiments of the process of the invention comprising the steps as specified in Figure 6D) that the solvent after the solvent exchange is one in which the compound of formula (I) or a solvate or salt is poorly soluble or insoluble. It is also preferred (in particular in the embodiments of the process of the invention as specified in any one of Figures 1E, 1F, 1G, and 1H or in the embodiments of the process of the invention comprising the steps as specified in Figure 6D) that after the solvent exchange the reaction mixture is substantially free of the solvent present before the solvent exchange took place, e.g., after the solvent exchange the reaction mixture contains ≤ 1.0% by weight of the solvent present before the solvent exchange took place. In one embodiment (in particular of the process of the invention as specified in any one of Figures 1E, 1F, 1G, and 1H or of the process of the invention comprising the steps as specified in Figure 6D), the solvent after the solvent exchange is an alcoholic solvent, such as 2-propanol. It is preferred (in particular in the embodiments of the process of the invention as specified in any one of Figures 1B, 1C, 1D, 1E, 1F, 1G, and 1H or in the embodiments of the process of the invention comprising the steps as specified in any one of Figures 6B, 6C, and 6D) that the precipitation of the compound of formula (1) or a solvate or salt is performed at a suitable temperature (e.g., between 0°C and 40°C, such as between 10°C and 30°C or between 15°C and 25°C) and/or for a sufficient amount of time (e.g., in the range of 0.5 h to 5 h, preferably 1 to 4 h, more preferably 2 to 3 h). In one embodiment (in particular of the process of the invention as specified in any one of Figures 1B, 1C, 1D, 1E, 1F, 1G, and 1H or of the process of the invention comprising the steps as specified in any one of Figures 6B, 6C, and 6D), the precipitated compound of formula (1) or a solvate or salt is collected (e.g., by filtration), optionally washed (e.g., with a solvent in which the precipitated compound of formula (I) or a solvate or salt is poorly soluble or insoluble (e.g., an ether solvent such as tert-butyl methyl ether)) and optionally dried (e.g., at a suitable temperature (such as between 30°C and 50°C, preferably between 40°C and 45°C) and/or for a sufficient amount of time (such as in the range of 4 to 20 h, e.g., 6 to 18 h, 8 to 16 h, 8 to 15 h, or 10 to 13 h)), wherein the optional washing and/or drying may be performed under an inert atmosphere (such as nitrogen).

In any of the above embodiments of the process of the first aspect (in particular of the process of the invention as specified in Figures 1A or in the embodiments of the process of the invention comprising the steps as specified in Figures 6A), after step d) or after the precipitation of the compound of formula (1) or a solvate or salt thereof, the process of the first aspect may further comprise the step of crystallizing the compound of formula (1) or a solvate or salt thereof, e.g., from an alcoholic solvent, preferably an aqueous alcoholic solvent. Preferably, the alcoholic solvent, preferably aqueous alcoholic solvent, used in this crystallizing step is one in which the compound of formula (I) or a solvate or salt thereof is soluble, at least at elevated temperature (e.g., between 60°C and 80°C, such as between 65°C and 70°C). An exemplary alcoholic solvent, preferably aqueous alcoholic solvent, for this purpose is selected from the group consisting of methanol, ethanol, 1-propanol, 2-propanol, and their mixtures with water, such as 5% water in ethanol.

In one mode (in particular of the embodiments of the process of the invention as specified in any one of Figures 1B, 1C, 1D, 1E, 1F, 1G, and 1H or in the embodiments of the process of the invention comprising the steps as specified in any one of Figures 6B, 6C, and 6D), the crystallizing of the compound of formula (1) or a solvate or salt thereof comprises the dissolving the compound of formula (I) or a solvate or salt thereof obtained from step d) in the alcoholic solvent, preferably the aqueous alcoholic solvent, at a suitable temperature (e.g., between 60°C and 80°C, such as between 65°C and 70°C); decreasing the temperature (e.g., by about 20°C, preferably to a range of between 40°C and 60°C, such as between 45°C and 50°C) over a suitable period of time (e.g., 2 h to 5 h, such as 2.5 h to 3.5 h); optionally adding a seed crystal of the compound of formula (1) or a solvate or salt thereof; optionally further decreasing the temperature (e.g., to a range of between -5°C and 10°C, such as 0°C to 5°C); collecting the solids (i.e., the compound of formula (1) or a solvate or salt thereof); optionally washing the solids with a suitable solvent (e.g., one in which the compound of formula (I) or a solvate or salt thereof is poorly soluble or insoluble, such as an ether solvent, e.g., tert-butyl methyl ether), wherein the optional washing is preferably performed without using a nitrogen blanket; optionally drying (e.g., at a suitable temperature (such as between 30°C and 50°C, preferably between 35°C and 40°C) and/or for a sufficient amount of time (such as in the range of 4 to 70 h, e.g., 6 to 60 h, 8 h to 50 h, 10 h to 40 h, or 12 h to 30 h)); and optionally repeating the crystallizing of the compound of formula (I) or a solvate or salt thereof.

In any of the above embodiments of the process of the first aspect (in particular of the process of the invention as specified in any one of Figures 1A, 1B, 1C, 1D, 1E, 1F, 1G, and 1H or of the process of the invention comprising (1) step a) as specified in any one of Figures 2A, 2B, 2C, and 2D; (2) step b) as specified in any one of Figures 3A, 3B, 3C, 3D, 3F, and 3G; (3) step c) as specified in any one of Figures 4A, 4B, 4C, 4D, 4E, 4F, 4G, and 4H; (4) step d) as specified in any one of Figures 5A, 5B, 5C, 5D, and 5E; and/or (5) the steps as specified in any one of Figures 6A, 6B, 6C, and 6D), it is preferred that one or more of steps a) to d) (e.g., step a), step b), step c) or step d); or steps a) and b), steps a) and c), steps a) and d), steps b) and c), steps b) and d), or steps c) and d); or steps a), b), and c), steps a), b), and d), or steps b), c) and d); or steps a), b), c), and d)) or all steps (i.e., the entire process of the first aspect) are performed in a chromatography-free manner. In other words, it is preferred that in one or more of steps a) to d) of the process of the first aspect (in particular of the process of the invention as specified in any one of Figures 1A, 1B, 1C, 1D, 1E, 1F, 1G, and 1H or of the process of the invention comprising (1) step a) as specified in any one of Figures 2A, 2B, 2C, and 2D; (2) step b) as specified in any one of Figures 3A, 3B, 3C, 3D, 3F, and 3G; (3) step c) as specified in any one of Figures 4A, 4B, 4C, 4D, 4E, 4F, 4G, and 4H; (4) step d) as specified in any one of Figures 5A, 5B, 5C, 5D, and 5E; and/or (5) the steps as specified in any one of Figures 6A, 6B, 6C, and 6D), the separation of the compound of interest (i.e., a compound of formula (I) or a solvate or salt thereof or an intermediate of any one of formulas (3), (4), and (5)) from other compounds (such as contaminants) is achieved by means other than chromatography, preferably by means of precipitating the compound of interest, optionally washing the precipitated compound of interest with a suitable solvent (in particular, a solvent in which the compound of interest is poorly soluble), and optionally drying the precipitated compound of interest. Thus, it is preferred that those steps of the process of the first aspect which are directed to the preparation of a compound of interest (i.e., preparative steps) are performed without using chromatography, whereas optional steps of the process of the first aspect which are directed to the analysis of a compound of interest and/or its contaminants (i.e., analytical steps) may be performed using chromatography.

In particular, the present inventors found that it is possible to avoid chromatography in the preparative steps of the process of the first aspect and that by using alternative means, especially precipitating the compound of interest (i.e., a compound of formula (1) or a solvate or salt thereof or an intermediate of any one of formulas (3), (4), and (5)), the compound of formula (I) or a solvate or salt thereof can be obtained in a much larger scale, in higher yield and in a purity similar to that achieved by a comparative process for synthesizing N-(4-(4-amino-2-(2-methoxyethyl)-1H-imidazo[4,5-c]quinolin-1-yl)butyl)-N-(tetrahydro-2H-pyran-4-yl)acetamide which uses several chromatographic purification steps.

In a second aspect, the present invention provides a compound selected from the group consisting of: 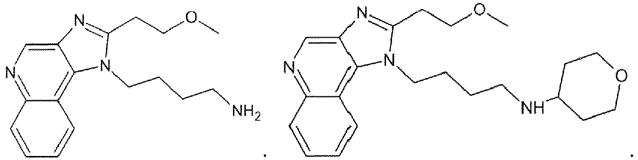 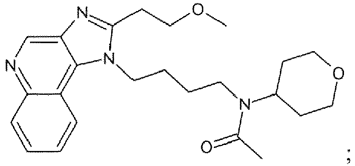 and a solvate or salt thereof. These compounds are intermediates useful in the process of the first aspect.

In a third aspect, the present invention provides a use of a compound of the second aspect for synthesizing a compound of formula (I). In this respect, it is noted that the embodiments specified above for the first aspect equally apply to the third aspect. Thus, for example, in one embodiment of the third aspect, a compound of formula (5) or a solvate or salt thereof is subjected to an oxidative amination reaction to give the compound of formula (1) or a solvate or salt thereof and, optionally, the compound of formula (I) or a solvate or salt is crystallized, preferably from an alcoholic solvent.

### Examples

### Abbreviations

The following abbreviations are used throughout description: SM: starting material for the respective synthetic step; IPC: In-Process Control; DCM: dichloromethane; TBME: tert-butyl methyl ether; MeOH: methanol; EtOH: ethanol; HCl: hydrochloric acid; NaOH: sodium hydroxide; Ac₂O: acetic anhydride; HPLC: High Performance Liquid Chromatography; LOD: Loss On Drying; IPA: 2-propanol; pTSCI or TsCl: p-toluenesulfonyl chloride; PTSA: p-toluenesulfonic acid; STAB: sodium triacetoxyborohydride; eq.: equivalent(s); vol.: volume(s); NMR: Nuclear Magnetic Resonance; ppm: parts per million; THP: tetrahydropyranyl; Ac₂O: acetic anhydride; mCPBA: 3-chloroperoxybenzoic acid.

### Example 1 - Synthesis of N-(4-(4-amino-2-(2-methoxyethyl)-1H-imidazo[4,5-c]quinolin-1-yl)butyl)-N-(tetrahydro-2H-pyran-4-yl)acetamide using the process of the present invention

### Step a): Providing a compound of formula (3)

Compound 1 (1 eq.), p-toluenesulfonic acid monohydrate (0.05 eq.) and toluene (10 vol.) were charged to a reactor. After addition of 1,1,1,3-tetramethoxypropane (1.2 eq.) the contents of reactor were adjusted to reflux at approx. 95°C and the content of compound 1 was monitored using HPLC.

### Step b): Preparing a compound of formula (4)

When the content of compound 1 dropped to ≤ 1.0% area of the initial area (determined by HPLC), the contents of reactor were adjusted to 10 to 15°C, 6 N HCl (5 vol.) were added to reactor over approx. 1 h, whereby the temperature was maintained ≤25°C. The contents of the reactor were agitated at 20°C to 25°C for at least 2 hours and the reaction completion was monitored by HPLC. The lower aqueous layer containing the desired product was collected, the upper organic layer was discharged, the collected aqueous layer containing the desired product was transferred into the reactor, DCM (5 vol.) was added, the reaction mixture was agitated for 15 min and allowed to settle for 15 min. The lower organic layer was discharged, water (2 vol.) was added to reactor, and the contents of reactor were adjusted to 0°C to 5°C. NaOH (11 eq.) was added to the reactor portion-wise while maintaining the temperature ≤30°C. After the pH of the reaction mixture was adjusted to ≥ 12, the temperature of the reaction mixture was increased to 20°C to 25°C, DCM (5 vol.) was added, and the reaction mixture was agitated for 15 min and allowed to settle for 15 min. The lower organic layer containing the desired product was collected, DCM (5 vol.) was added, and the reaction mixture was agitated for 15 min and allowed to settle for 15 min. The lower organic layer containing the product was collected, the upper aqueous layer was discharged, and the 2 collected organic layers were combined in the reactor. A solvent exchange to ethanol was performed until the amount of DCM in the reaction mixture was ≤1%. The amount of compound 4 was determined, fumaric acid (1 eq. based on the determined amount of compound 4) was added, and the reaction mixture was agitated at 20°C to 25°C for at least 12 hours to precipitate the compound of formula (4) as fumaric acid addition salt. TBME (10 vol.) was added over at least 30 min and the reaction mixture was agitated at 20°C to 25°C for 1 to 2 hours. The precipitate was collected using filtration under nitrogen, the reactor was washed with TBME (2 × 3 vol.) and this washing solution was used to wash the precipitate under nitrogen. The collected precipitate was dried at 40°C to 45°C for at least 12 hours.

**Table 1**

| *Input* | *Output* | *Yield* | *Purity by HPLC* |
|---|---|---|---|
| 7.30Kg | 8.50Kg | 92% | 95.428% area |

### Step c): Preparing a compound of formula (5)

Compound 4 as fumaric acid addition salt (1 eq.) obtained from step b), DCM (5 vol.), and 2 M NaOH (10 vol.) were added to a reactor, and the reaction mixture was agitated for 15 min and allowed to settle for 15 min. The lower organic layer containing compound 4 as free base was collected, the aqueous phase was extracted with DCM (5 vol.), and the lower organic layer was collected. After discharging the upper aqueous layer, the 2 organic layers were charged into the reactor, MgSO₄ (0.2 wt.-%) were added to the reactor, the reaction mixture was agitated for 5 min and the contents of the reactor filtered to remove MgSO₄. The filtrate was charged into a new clean reactor, tetrahydropyranone (1.1 eq.) and acetic acid (1 eq.) were added, and the temperature of the reaction mixture was adjusted to -10°C to 0°C. STAB (1.8 eq.) was added portion-wise while maintaining the temperature ≤0°C. After completion of the addition, the temperature was increased to 20°C to 25°C, the reaction mixture was agitated for at least 18 hours, and the reaction completion was monitored by HPLC. Once the content of compound 4 Target was ≤0.50% of the initial area, the reaction mixture was cooled to 10°C to 15°C and slowly quenched by the addition of 2 M NaOH (10 vol.) while maintaining the temperature ≤ 25°C. After completion of the NaOH addition, the reaction mixture was agitated for 15 min and allowed to settle for 15 min. The lower organic layer containing the desired product was collected, the upper aqueous layer was extracted with DCM (10 vol.), the lower organic layer containing the desired product was collected, and the upper aqueous layer was discharged. MgSO₄ (0.2 wt.%) was added to the reactor and the 2 organic layers were charged into the reactor and the reaction mixture was agitated for at least 15 min. MgSO₄ was removed by filtration and the MgSO₄ cake was washed with DCM (1 vol.). The filtrates were combined in a clean reactor and reduced to approx. 10 to 12 vol. under reduced pressure at 35°C to 40°C. The temperature of the reaction mixture was adjusted to 20°C to 25°C, acetic anhydride (1 eq.) was added slowly while maintaining the temperature between 20°C and 30°C. After completion of the addition, the temperature of the reaction mixture was adjusted to 20°C to 25°C, the reaction mixture was agitated for at least 24 hours, and the content of the tetrahydropyranyl adduct and STAB was monitored using HPLC. Once the content of each the tetrahydropyranyl adduct and STAB was ≤ 0.50% of the initial area, the reaction was slowly quenched by the addition of 5% NaHCO₃ solution (10 vol.), and the reaction was agitated for 15 min and allowed to settle for 15 min. The lower organic layer containing the desired product was collected and charged into a clean reactor. MgSO₄ (0.2 wt.-%) was added, the reaction mixture was agitated for at least 15 min and then filtered to remove MgSO₄. The reactor was washed with DCM (1 vol.) and this washing solution was used to rinse the MgSO₄ cake. The filtrates were recharged into a clean reactor and reduced to 6-7 vol. under reduced pressure at ≤ 30°C. A solvent exchange to TBME was performed until the content of DCM in the reaction mixture was ≤10%. Then, TBME (3 vol.) was added, the temperature of the reaction mixture was adjusted to 20°C to 25°C and the reaction mixture was agitated for at least 1 hour. The suspension was filtered under nitrogen and the reactor and filter cake were washed with TBME (2 × 2 vol.). The collected precipitate was dried at 35°C to 40°C for at least 12 hours.

**Table 2**

| *Input* | *Output* | *Yield* | *Purity by HPLC* |
|---|---|---|---|
| 8.40Kg | 6.41 Kg | 74% | 94.88% area |

### Step d): Preparing a compound of formula (I)

Compound 5 (1 eq.) obtained from step c) was charged in a reactor, DCM (10 vol.) was added, and the temperature of the reaction mixture was adjusted to -10°C to 0°C. 70% mCPBA (1.5 eq.) was added to the reactor portion-wise while maintaining the temperature ≤ 0°C. After completion of the addition the temperature of the reaction mixture was adjusted to 20°C to 25°C, the reaction mixture was agitated for at least 2 hours, and the content of compound 5 in the reaction mixture was monitored using HPLC. Once the content of compound 5 in the reaction mixture was ≤ 4% of the initial amount, the temperature of the reaction mixture was adjusted to 0°C to 5°C and ammonium hydroxide (10 vol.) was added while maintaining the temperature ≤ 20°C. After completion of the addition, the temperature of the reaction mixture was adjusted to 0°C to 5°C. pTsCl (1.5 eq.) dissolved in DCM (3 vol.) was slowly added to the reactor while maintaining the temperature ≤ 10°C. After completion of the addition, the temperature of the reaction mixture was adjusted to 20°C to 25°C, the reaction mixture was agitated for at least 2 hours, and the content of the N-oxide intermediate in the reaction mixture was monitored using HPLC. Once the content of the N-oxide intermediate in the reaction mixture was ≤0.5% of the initial amount, the temperature of the reaction mixture was adjusted to 30°C to 35°C and the reaction mixture was agitated for at least 2 hours. The temperature of the reaction mixture was adjusted to 20°C to 25°C, the lower organic layer containing the desired product was collected, charged into a clean reactor, MgSO₄ (0.2 wt.-%) was added, and the reaction mixture was agitated for at least 15 min. The reaction mixture was filtered to remove MgSO₄, the reactor was washed with DCM (1 vol.) and this washing solution was used to rinse the MgSO₄ cake. The filtrates were combined in a clean reactor and reduced to approx. 10 vol. under reduced pressure at 35°C to 40°C, and the content of the compound of formula (I) in the resulting solution was determined.

**Table 3**

| *Input* | *Output* | *Yield* |
|---|---|---|
| 6.35 Kg | 7.7 Kg* | 113% |

### Step e): Precipitating the compound of formula (I)

The crude solution of the compound of formula (1) (1 eq.) obtained from step d) was charged into a reactor. A solvent exchange to IPA was performed until the content of DCM in the reaction mixture was ≤1%. Then, the temperature of the reaction mixture was adjusted to 5°C to 10°C and the reaction mixture was agitated for at least 1 h. The suspension was filtered, the reactor was washed with IPA (2 vol.), the washing solution was cooled to 5°C to 10 °C and then used to rinse the filter cake. The reactor was washed with TBME (2 × 5 vol.) and each washing solution was used to rinse the filter cake. The collected precipitate was dried at 35°C to 40°C for at least 12 hours

**Table 4**

| *Input* | *Output* | *Yield* | *Purity by HPLC* |
|---|---|---|---|
| 3.00 Kg | 1.62 Kg | 54%* | 96.18% area |
| 3.00 Kg | 1.70 Kg | 57%* | Not proceeded to step 4 |

| | | | |
|---|---|---|---|
| *Yield typical. Expected yield = 45 to 60% theoretical as input is crude product. | | | |

### Step f): Crystallization of the compound of formula (I)

5% water in ethanol (10 vol.) was charged into a reactor and the compound of formula (I) (1 eq.) obtained from step c) was added. The temperature of the reaction mixture was adjusted to 65°C to 70°C and held for at 30 min to 1 hour. Once all of the compound of formula (I) was dissolved, the solution was filtered and the filtrate was charged into a clean reactor preheated to 65°C to 70°C. The temperature of the solution was adjusted to 45°C to 50°C over 3 h ± 30 mins. A seed crystal of the compound of formula (I) was added, temperature of the reaction mixture was adjusted to 0°C to 5°C over at least 7 h, and the reaction mixture was agitated at 0°C to 5°C for at least 2 hours. The suspension was filtered, the reactor was washed with 5% water in ethanol (2 vol.), the temperature of the washing solution was adjusted to 0°C to 5°C and used to rinse the filter cake. The reactor was washed with TBME (2 vol.) and the washing solution was used to rinse the filter cake. Optionally, the crystallization step was repeated.

**Table 5**

| *Input* | *Output* | *Yield* | *Purity by HPLC* |
|---|---|---|---|
| 1.60Kg | 1.19Kg | 71% | 99.0% area |

The overall yield of the process of the present invention, the amount of compound of formula (I) produced by said process, and the time required for the synthesis are given in Table 6, below.

### Example 2 - Comparative synthesis of N-(4-(4-amino-2-(2-methoxyethyl)-1H-imidazo[4,5-c]quinolin-1-yl)butyl)-N-(tetrahydro-2H-pyran-4-yl)acetamide using chromatographic purification steps (comparative process B)

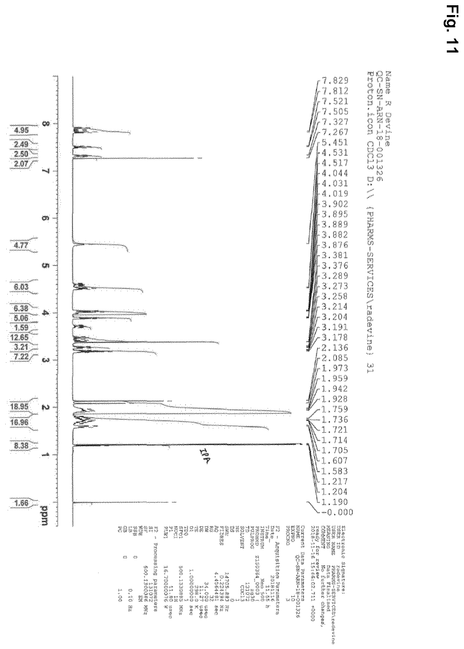

N-(4-(4-amino-2-(2-methoxyethyl)-1H-imidazo[4,5-c]quinolin-1-yl)butyl)-N-(tetrahydro-2H-pyran-4-yl)acetamide was prepared according to the process set forth in Fig. 11, which is similar to the process of the present invention but which used 3 chromatographic purification steps (one after the removal of the Boc protecting group; one after the reaction with tetrahydropyranone; and one after the oxidative amination). The overall yield of the comparative process B, the amount of compound of formula (I) produced by said process, and the time required for the synthesis are given in Table 6, below.

**Table 6**

| | *Process of the present invention* | *Comparative process B* |
|---|---|---|
| Overall yield | 31% | 11% |
| Chromatographic purifications | 0 | 3 |
| Amount produced | 1.19 kg | 10.42 g |
| Purity | 99.0% | 99.5% |
| Time | 7.5 weeks | 5 weeks |

As can be seen from the results presented in Table 6, the process of the present invention provides much higher yields than the comparative process B and is capable of providing the compound of formula (I) in much higher amounts (about 1.2 kg vs. about 10 g, i.e., a factor of more than 10²) than the comparative process B and in a purity comparable to that achieved by using the comparative process B.

## Claims

1. A process for synthesizing a compound having the formula (1): 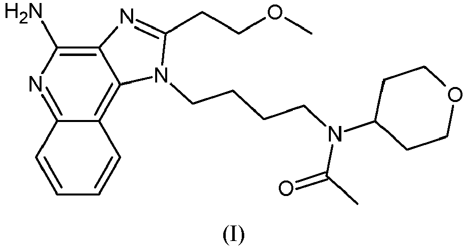 or a solvate or salt thereof, the process comprising the following steps:
a) providing a compound of formula (3): 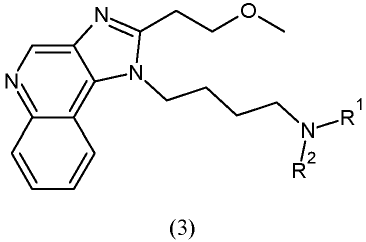 or a solvate or salt thereof, wherein each of R¹ and R² is independently selected from the group consisting of -H and an amino protecting group, and at least one of R¹ and R² is an amino protecting group;
b) reacting the compound of formula (3) or a solvate or salt thereof with one or more reagents to remove the amino protecting group(s) at position R¹ and R² to give a compound of formula (4): 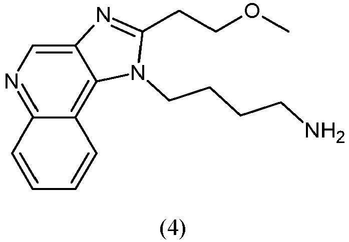 or a solvate or salt thereof;
c) reacting the compound of formula (4) or a solvate or salt thereof with one or more reagents for introducing (i) a tetrahydropyranyl group and (ii) an acetyl group to give a compound of formula (5): 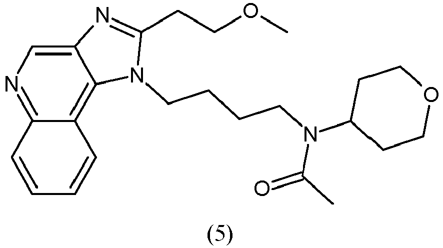 or a solvate or salt thereof; and
d) subjecting the compound of formula (5) or a solvate or salt thereof to an oxidative amination reaction to give the compound of formula (I) or a solvate or salt thereof.

2. The process of claim 1, further comprising the step of (i) precipitating the compound of formula (4) or a solvate or salt thereof before step c); (ii) precipitating the compound of formula (5) or a solvate or salt thereof before step d); and/or (iii) precipitating the compound of formula (I) or a solvate or salt thereof after step d).

3. The process of claim 1 or 2, wherein each of R¹ and R² is independently selected from the group consisting of -H, tert-butyloxycarbonyl (Boc), 9-fluorenylmethyloxy carbonyl (Fmoc), benzyloxycarbonyl (Cbz), tosylate (Ts), benzyl (Bn), allyloxycarbonyl (Alloc), trityl (Trt), dimethoxytrityl (DMT), and monomethoxytrityl (MMT).

4. The process of any one of claims 1 to 3, wherein step a) comprises reacting a compound of formula (1): 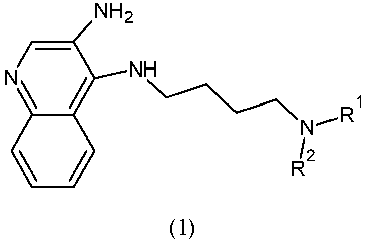 or a solvate or salt thereof with HOOC(CH₂)₂OCH₃ (formula (2)) or a derivative thereof to give a compound of formula (3) or a solvate or salt thereof, wherein, preferably, the derivative of formula (2) is (i) selected from the group consisting of carboxylic acid halides, orthoesters, and 1,1-dialkoxyalkyl alkanoates; and/or (ii) an orthoester, preferably an orthoester having the formula (R¹⁰O)₃C(CH₂)₂OCH₃, wherein each R¹⁰ is independently selected from the group consisting of alkyl, alkenyl, alkynyl, aryl, heteroaryl, cycloalkyl, and heterocycloalkyl.

5. The process of claim 4, wherein the reaction of the compound of formula (1) with the compound of formula (2) is performed (i) in a solvent, such as an aromatic solvent; (ii) in the presence of an acid; (iii) at a suitable temperature, such as between 0°C and 120°C, e.g., at the reflux temperature of the solvent, if present; and/or (iv) for a sufficient amount of time, such as 30 min to 10 h.

6. The process of any one of claims 1 to 5, wherein step b) is performed (i) in a solvent, such as an aromatic solvent, e.g., the solvent used in step a); (ii) at a suitable temperature, such as between 0°C and 40°C; and/or (iii) for a sufficient amount of time, such as 30 min to 5 h.

7. The process of any one of claims 1 to 6, wherein:
(A) each of R¹ and R² is independently selected from the group consisting of -H and tert-butyloxycarbonyl (Boc) and the one or more reagents to remove the amino protecting group(s) in step b) are selected from the group consisting of trifluoroacetic acid (TFA), HCl, CH₃SO₃H, and (H₃C)₃SiCl (TMSCl), wherein preferably, after the reaction of the compound of formula (3) or a solvate or salt thereof with one or more reagents to remove the amino protecting group(s), step b) further comprises adding a base; and/or
(B) the one or more reagents for introducing a tetrahydropyranyl group in step c) are tetrahydropyranone and a reducing agent, such as tetrahydropyranone and a borohydride; and/or
(C) in step c) the reaction of the compound of formula (4) or a solvate or salt thereof with one or more reagents for introducing a tetrahydropyranyl group is performed (i) in a solvent, such as a halogenated organic solvent; (ii) in the presence of an acid; (iii) at a suitable temperature, such as between -20°C and 40°C; and/or (iv) for a sufficient amount of time, such as 5 h to 24 h; and/or
(D) the one or more reagents for introducing an acetyl group in step c) are selected from acetic anhydride and acetic halides; and/or
(E) in step c) the reaction of the compound of formula (4) or a solvate or salt thereof with one or more reagents for introducing an acetyl group is performed (i) in a solvent, such as a halogenated organic solvent; (ii) at a suitable temperature, such as between 0°C and 50°C; and/or (iii) for a sufficient amount of time, such as 6 h to 48 h.

8. The process of any one of claims 1 to 7, wherein step d) comprises reacting the compound of formula (5) or a solvate or salt thereof with first (i) an oxidation agent, and then with (ii) an acylating agent and an aminating agent.

9. The process of claim 8, wherein the oxidation agent is selected from oxidation agents capable of forming N-oxides, such as 3-chloroperoxybenzoic acid, and the oxidation reaction is preferably performed (i) in a solvent, such as a halogenated organic solvent; (ii) at a suitable temperature, such as between -10°C and 40°C; and/or (iii) for a sufficient amount of time, such as 30 min to 12 h.

10. The process of claim 8 or 9, wherein the acylating agent is selected from the group consisting of alkysulfonyl halides and arylsulfonyl halides, such as methanesulfonyl chloride, benzenesulfonyl chloride, or p-toluenesulfonyl chloride, and the reaction with the acylating agent is preferably performed (i) in a solvent, such as a halogenated organic solvent; (ii) at a suitable temperature, such as between -10°C and 40°C; and/or (iii) for a sufficient amount of time, such as 30 min to 12 h.

11. The process of any one of claims 8 to 10, wherein the aminating agent is selected from the group consisting of ammonia and its salts, such as aqueous NH₃, ammonium hydroxide, ammonium carbonate, ammonium bicarbonate, or ammonium phosphate, and the reaction with the aminating agent is preferably performed (i) in a solvent, such as a halogenated organic solvent; (ii) at a suitable temperature, such as between -10°C and 40°C; and/or (iii) for a sufficient amount of time, such as 30 min to 12 h.

12. The process of any one of claims 1 to 11, wherein after step d) the process further comprises crystallizing the compound of formula (I) or a solvate or salt thereof, preferably from an alcoholic solvent.

13. The process of any one of claims 1 to 12, wherein one or more of steps a) to d) are performed in a chromatography-free manner.

14. A compound selected from the group consisting of: 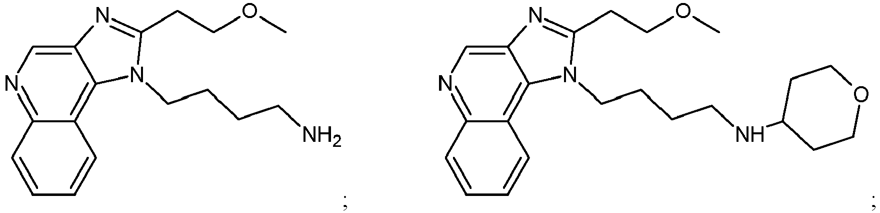 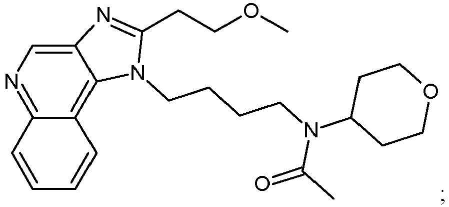 and a solvate or salt thereof.

15. Use of a compound of claim 14 for synthesizing a compound of formula (I) as defined in claim 1.

## Patentansprüche

1. Verfahren zum Synthetisieren einer Verbindung der Formel (I): 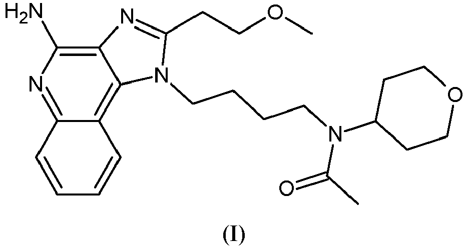 oder eines Solvats oder Salzes davon, wobei das Verfahren die folgenden Schritte umfasst:
a) Bereitstellen einer Verbindung der Formel (3): 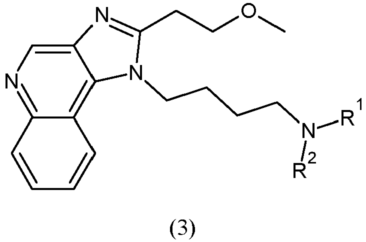 oder eines Solvats oder Salzes davon, wobei jedes R¹ und R² unabhängig ausgewählt ist aus der Gruppe, bestehend aus -H und einer Aminoschutzgruppe, und mindestens eines von R¹ und R² eine Aminoschutzgruppe ist;
b) Reagieren der Verbindung der Formel (3) oder eines Solvats oder Salzes davon mit einem oder mehreren Reagenzien zum Entfernen der Aminoschutzgruppe(n) an Position R¹ und R², um eine Verbindung der Formel (4): 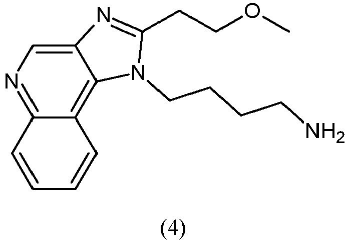 oder ein Solvat oder Salz davon zu ergeben;
c) Reagieren der Verbindung der Formel (4) oder eines Solvats oder Salzes davon mit einem oder mehreren Reagenzien zum Einbringen (i) einer Tetrahydropyranylgruppe und (ii) einer Acetylgruppe, um eine Verbindung der Formel (5): 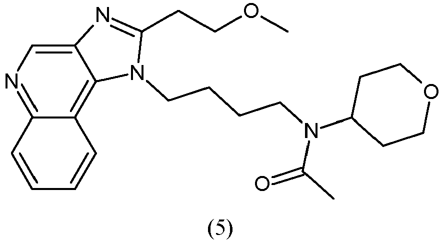 oder ein Solvat oder Salz davon zu ergeben; und
d) Unterziehen der Verbindung der Formel (5) oder eines Solvats oder Salzes davon einer oxidativen Aminierungsreaktion, um die Verbindung der Formel (I) oder ein Solvat oder Salz davon zu ergeben.

2. Verfahren nach Anspruch 1, das ferner den Schritt eines (i) Präzipitierens der Verbindung der Formel (4) oder eines Solvats oder Salzes davon vor Schritt c); (ii) Präzipitierens der Verbindung der Formel (5) oder eines Solvats oder Salzes davon vor Schritt d); und/oder (iii) Präzipitierens der Verbindung der Formel (I) oder eines Solvats oder Salzes davon nach Schritt d) umfasst.

3. Verfahren nach Anspruch 1 oder 2, wobei jedes R¹ und R² unabhängig ausgewählt ist aus der Gruppe, bestehend aus -H, tert-Butyloxycarbonyl- (Boc), 9-Fluorenylmethyloxycarbonyl-(Fmoc), Benzyloxycarbonyl- (Cbz), Tosylat- (Ts), Benzyl- (Bn), Allyloxycarbonyl- (Alloc), Trityl- (Trt), Dimethoxytrityl- (DMT) und Monomethoxytritylgruppe (MMT).

4. Verfahren nach einem jeglichen der Ansprüche 1 bis 3, wobei Schritt a) ein Reagieren einer Verbindung der Formel (1): 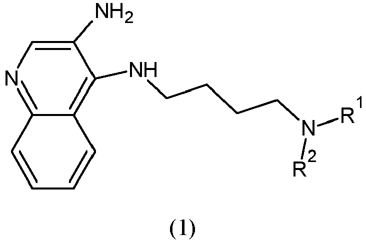 oder eines Solvats oder Salzes davon mit HOOC(CH₂)₂OCH₃ (Formel (2)) oder einem Derivat davon, um eine Verbindung der Formel (3) oder ein Solvat oder Salz davon zu ergeben, umfasst, wobei vorzugsweise das Derivat der Formel (2) (i) ausgewählt ist aus der Gruppe, bestehend aus Carbonsäurehalogeniden, Orthoestern und 1,1-Dialkoxyalkylalkanoaten; und/oder (ii) ein Orthoester, vorzugsweise ein Orthoester der Formel (R¹⁰O)₃C(CH₂)₂OCH₃ ist, worin jedes R¹⁰ unabhängig ausgewählt ist aus der Gruppe, bestehend aus Alkyl-, Alkenyl-, Alkinyl-, Aryl-, Heteroaryl-, Cycloalkyl- und Heterocycloalkylgruppe.

5. Verfahren nach Anspruch 4, wobei die Reaktion der Verbindung der Formel (1) mit der Verbindung der Formel (2) (i) in einem Lösungsmittel, wie einem aromatischen Lösungsmittel; (ii) in der Gegenwart einer Säure; (iii) bei einer geeigneten Temperatur, wie zwischen 0°C und 120°C, z.B. bei der Rückflusstemperatur des Lösungsmittels, falls präsent; und/oder (iv) für eine ausreichende Zeitspanne, wie 30 min bis 10 h, durchgeführt wird.

6. Verfahren nach einem jeglichen der Ansprüche 1 bis 5, wobei Schritt b) (i) in einem Lösungsmittel, wie einem aromatischen Lösungsmittel, z.B. dem in Schritt a) verwendeten Lösungsmittel; (ii) bei einer geeigneten Temperatur, wie zwischen 0°C und 40°C; und/oder (iii) für eine ausreichende Zeitspanne, wie 30 min bis 5 h, durchgeführt wird.

7. Verfahren nach einem jeglichen der Ansprüche 1 bis 6, wobei:
(A) jedes R¹ und R² unabhängig ausgewählt ist aus der Gruppe, bestehend aus -H und tert-Butyloxycarbonylgruppe (Boc), und das eine oder die mehreren Reagenzien zum Entfernen der Aminoschutzgruppe(n) in Schritt b) ausgewählt sind aus der Gruppe, bestehend aus Trifluoressigsäure (TFA), HCl, CH₃SO₃H und (H₃C)₃SiCl (TMSCl), wobei vorzugsweise nach der Reaktion der Verbindung der Formel (3) oder eines Solvats oder Salzes davon mit einem oder mehreren Reagenzien zum Entfernen der Aminoschutzgruppe(n) Schritt b) ferner ein Zusetzen einer Base umfasst; und/oder
(B) das eine oder die mehreren Reagenzien zum Einbringen einer Tetrahydropyranylgruppe in Schritt c) Tetrahydropyranon und ein Reduktionsmittel, wie Tetrahydropyranon und ein Borhydrid, sind; und/oder
(C) in Schritt c) die Reaktion der Verbindung der Formel (4) oder eines Solvats oder Salzes davon mit einem oder mehreren Reagenzien zum Einbringen einer Tetrahydropyranylgruppe (i) in einem Lösungsmittel, wie einem halogenierten organischen Lösungsmittel; (ii) in der Gegenwart einer Säure; (iii) bei einer geeigneten Temperatur, wie zwischen -20°C und 40°C; und/oder (iv) für eine ausreichende Zeitspanne, wie 5 h bis 24 h, durchgeführt wird; und/oder (D) das eine oder die mehreren Reagenzien zum Einbringen einer Acetylgruppe in Schritt c) ausgewählt sind aus Essigsäureanhydrid und Essigsäurehalogeniden; und/oder (E) in Schritt c) die Reaktion der Verbindung der Formel (4) oder eines Solvats oder Salzes davon mit einem oder mehreren Reagenzien zum Einbringen einer Acetylgruppe (i) in einem Lösungsmittel, wie einem halogenierten organischen Lösungsmittel; (ii) bei einer geeigneten Temperatur, wie zwischen 0°C und 50°C; und/oder (iii) für eine ausreichende Zeitspanne, wie 6 h bis 48 h, durchgeführt wird.

8. Verfahren nach einem jeglichen der Ansprüche 1 bis 7, wobei Schritt d) ein Reagieren der Verbindung der Formel (5) oder eines Solvats oder Salzes davon mit zuerst (i) einem Oxidationsmittel und dann mit (ii) einem Acylierungsmittel und einem Aminierungsmittel umfasst.

9. Verfahren nach Anspruch 8, wobei das Oxidationsmittel ausgewählt ist aus Oxidationsmitteln, die fähig sind, N-Oxide zu bilden, wie 3-Chlorperoxybenzoesäure, und die Oxidationsreaktion vorzugsweise (i) in einem Lösungsmittel, wie einem halogenierten organischen Lösungsmittel; (ii) bei einer geeigneten Temperatur, wie zwischen -10°C und 40°C; und/oder (iii) für eine ausreichende Zeitspanne, wie 30 min bis 12 h, durchgeführt wird.

10. Verfahren nach Anspruch 8 oder 9, wobei das Acylierungsmittel ausgewählt ist aus der Gruppe, bestehend aus Alkylsulfonylhalogeniden und Arylsulfonylhalogeniden, wie Methansulfonylchlorid, Benzolsulfonylchlorid oder p-Toluolsulfonylchlorid, und die Reaktion mit dem Acylierungsmittel vorzugsweise (i) in einem Lösungsmittel, wie einem halogenierten organischen Lösungsmittel; (ii) bei einer geeigneten Temperatur, wie zwischen -10°C und 40°C; und/oder (iii) für eine ausreichende Zeitspanne, wie 30 min bis 12 h, durchgeführt wird.

11. Verfahren nach einem jeglichen der Ansprüche 8 bis 10, wobei das Aminierungsmittel ausgewählt ist aus der Gruppe, bestehend aus Ammoniak und seinen Salzen, wie wässriges NH₃, Ammoniumhydroxid, Ammoniumcarbonat, Ammoniumbicarbonat oder Ammoniumphosphat, und die Reaktion mit dem Aminierungsmittel vorzugsweise (i) in einem Lösungsmittel, wie einem halogenierten organischen Lösungsmittel; (ii) bei einer geeigneten Temperatur, wie zwischen -10°C und 40°C; und/oder (iii) für eine ausreichende Zeitspanne, wie 30 min bis 12 h, durchgeführt wird.

12. Verfahren nach einem jeglichen der Ansprüche 1 bis 11, wobei nach Schritt d) das Verfahren ferner ein Kristallisieren der Verbindung der Formel (I) oder eines Solvats oder Salzes davon, vorzugsweise aus einem alkoholischen Lösungsmittel, umfasst.

13. Verfahren nach einem jeglichen der Ansprüche 1 bis 12, wobei einer oder mehrere der Schritte a) bis d) in einer Chromatographie-freien Weise durchgeführt werden.

14. Verbindung, die aus der Gruppe ausgewählt ist, bestehend aus: 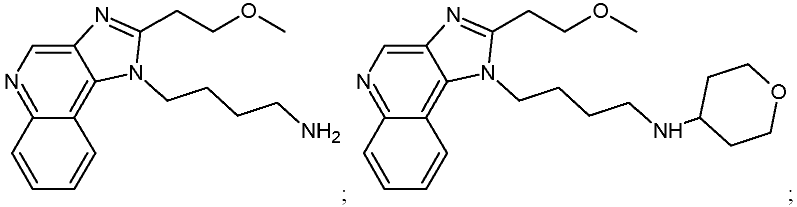 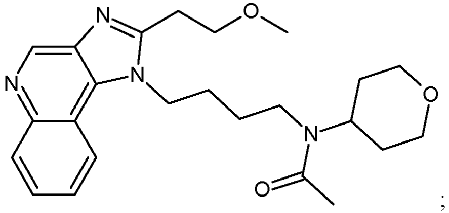 und einem Solvat oder Salz davon.

15. Verwendung einer Verbindung nach Anspruch 14 zum Synthetisieren einer wie in Anspruch 1 definierten Verbindung der Formel (I).

## Revendications

1. Procédé permettant la synthèse d'un composé comportant la formule (I) : 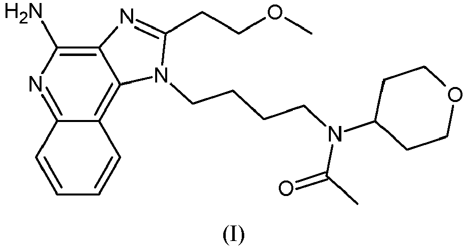 ou un solvate ou sel de celui-ci, le procédé comprenant les étapes suivantes :
a) fourniture d'un composé de formule (3) : 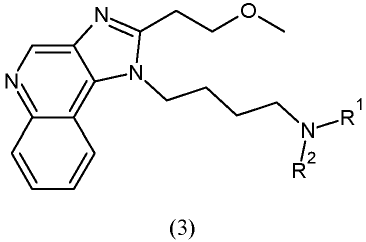 ou un solvate ou sel de celui-ci, dans lequel chacun des R¹ et R² est choisi indépendamment parmi le groupe constitué de -H et d'un groupe amino protecteur, et au moins un parmi R¹ et R² est un groupe amino protecteur ;
b) mise en réaction du composé de formule (3) ou d'un solvate ou sel de celui-ci avec un ou plusieurs réactifs pour éliminer le ou les groupes amino protecteurs à la position R¹ et R² pour donner un composé de formule (4) : 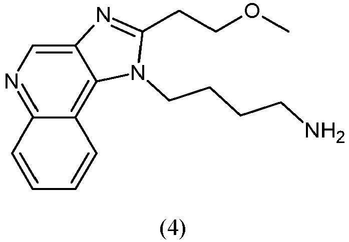 ou un solvate ou sel de celui-ci ;
c) mise en réaction du composé de formule (4) ou d'un solvate ou sel de celui-ci avec un ou plusieurs réactifs pour introduire (i) un groupe tétrahydropyranyle et (ii) un groupe acétyle pour donner un composé de formule (5) : 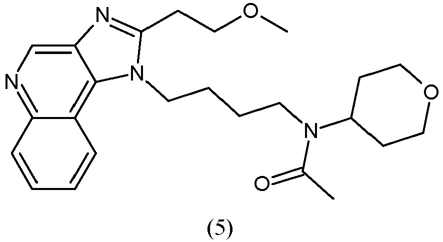 ou un solvate ou sel de celui-ci ; et
d) exposition du composé de la formule (5) ou d'un solvate ou sel de celui-ci à une réaction d'amination oxydative pour donner le composé de formule (I) ou un solvate ou sel de celui-ci.

2. Procédé de la revendication 1, comprenant en outre l'étape de (i) précipitation du composé de la formule (4) ou d'un solvate ou sel de celui-ci avant l'étape c) ; (ii) précipitation du composé de la formule (5) ou d'un solvate ou sel de celui-ci avant l'étape d) ; et/ou (iii) précipitation du composé de formule (I) ou d'un solvate ou sel de celui-ci après l'étape d).

3. Procédé de la revendication 1 ou 2, dans lequel chacun des R¹ et R² est choisi indépendamment parmi le groupe constitué de -H, tert-butyloxycarbonyle (Boc), 9-fluorénylméthyloxycarbonyle (Fmoc), benzyloxycarbonyle (Cbz), tosylate (Ts), benzyle (Bn), allyloxycarbonyle (Alloc), trityle (Trt), diméthoxytrityle (DMT), et monométhoxytrityle (MMT).

4. Procédé de l'une quelconque des revendications 1 à 3, dans lequel l'étape a) comprend la mise en réaction d'un composé de formule (1) : 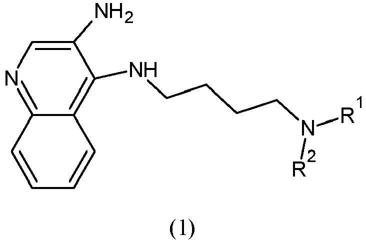 ou d'un solvate ou sel de celui-ci avec HOOC(CH₂)₂OCH₃ (formule (2)) ou d'un dérivé de celui-ci pour donner un composé de formule (3) ou un solvate ou sel de celui-ci, dans lequel, de préférence, le dérivé de formule (2) est (i) choisi parmi le groupe constitué d'halogénures d'acides carboxyliques, orthoesters, et d'alcanoates de 1,1-dialcoxyalkyle ; et/ou (ii) un orthoester, de préférence un orthoester comportant la formule (R¹⁰O)₃C(CH₂)₂OCH₃, dans laquelle chaque R¹⁰ est choisi indépendamment parmi le groupe constitué de alkyle, alcényle, alcynyle, aryle, hétéroaryle, cycloalkyle, et hétérocycloalkyle.

5. Procédé de la revendication 4, dans lequel la réaction du composé de formule (1) avec le composé de formule (2) est effectuée (i) dans un solvant, tel qu'un solvant aromatique ; (ii) en présence d'un acide ; (iii) à une température appropriée, tel qu'entre 0 °C et 120 °C, par exemple, à la température de reflux du solvant, si présent ; et/ou (iv) pendant une période suffisante, tel que 30 minutes à 10 heures.

6. Procédé de l'une quelconque des revendications 1 à 5, dans lequel l'étape b) est effectuée (i) dans un solvant, tel qu'un solvant aromatique, par exemple, le solvant utilisé à l'étape a) ; (ii) à une température appropriée, telle qu'entre 0 °C et 40 °C ; et/ou (iii) pendant une durée suffisante, telle que 30 minutes à 5 heures.

7. Procédé de l'une quelconque des revendications 1 à 6, dans lequel :
(A) chacun des R¹ et R² est choisi indépendamment parmi le groupe constitué de -H et de tert-butyloxycarbonyl (Boc) et le ou les réactifs pour éliminer le ou les groupes amino protecteurs à l'étape b) sont choisis parmi le groupe constitué d'acide trifluoroacétique (TFA), HCl, CH₃SO₃H, et (H₃C)₃SiCl (TMSC1), dans lequel, de préférence, après la réaction du composé de formule (3) ou d'un solvate ou sel de celui-ci avec un ou plusieurs réactifs pour éliminer le ou les groupes amino protecteurs, l'étape b) comprend en outre l'ajout d'une base ; et/ou
(B) le ou les réactifs pour introduire un groupe tétrahydropyranyle à l'étape c) sont la tétrahydropyranone et un agent réducteur, tel que la tétrahydropyranone et le borohydrure ; et/ou
(C) à l'étape c) la réaction du composé de formule (4) ou d'un solvate ou sel de celui-ci avec un ou plusieurs réactifs pour introduire un groupe tétrahydropyranyle est effectuée (i) dans un solvant, tel qu'un solvant organique halogéné ; (ii) en présence d'un acide ; (iii) à une température appropriée, tel qu'entre -20 °C et 40 °C; et/ou (iv) pendant une période suffisante, tel que 5 heures à 24 heures ; et/ou
(D) le ou les réactifs pour introduire un groupe acétyle à l'étape c) sont choisis parmi l'anhydride acétique et des halogénures acétiques ; et/ou
(E) à l'étape c) la réaction du composé de formule (4) ou d'un solvate ou sel de celui-ci avec un ou plusieurs réactifs pour introduire un groupe acétyle est effectuée (i) dans un solvant, tel qu'un solvant organique halogéné ; (ii) à une température appropriée, tel qu'entre 0 °C et 50 °C ; et/ou (iii) pendant une période suffisante, tel que 6 heures à 48 heures.

8. Procédé de l'une quelconque des revendications 1 à 7, dans lequel l'étape d) comprend la réaction du composé de formule (5) ou d'un solvate ou sel de celui-ci avec d'abord (i) un agent d'oxydation, et ensuite (ii) un agent acylant et un agent aminant.

9. Procédé de la revendication 8, dans lequel l'agent d'oxydation est choisi parmi des agents d'oxydation capables de former des oxydes de N, tels que l'acide 3-chloroperoxybenzoïque, et la réaction d'oxydation est de préférence effectuée (i) dans un solvant, tel qu'un solvant organique halogéné ; (ii) à une température appropriée, tel qu'entre -10 °C et 40 °C ; et/ou (iii) pendant une durée suffisante, tel que 30 minutes à 12 heures.

10. Procédé de la revendication 8 ou 9, dans lequel l'agent acylant est choisi parmi le groupe constitué d'halogénures d'alkysulfonyles et d'halogénures d'arylsulfonyles, tels que le chlorure de méthanesulfonyle, le chlorure de benzènesulfonyle ou le chlorure de p-toluènesulfonyle, et la réaction avec l'agent acylant est de préférence effectuée (i) dans un solvant, tel qu'un solvant organique halogéné ; (ii) à une température appropriée, tel qu'entre -10 °C et 40 °C ; et/ou (iii) pendant une durée suffisante, tel que 30 minutes à 12 heures.

11. Procédé de l'une quelconque des revendications 8 à 10, dans lequel l'agent aminant est choisi parmi le groupe constitué de l'ammoniac et ses sels, tels que NH₃ aqueux, hydroxyde d'ammonium, carbonate d'ammonium, bicarbonate d'ammonium ou phosphate d'ammonium, et la réaction avec l'agent aminant est de préférence effectuée (i) dans un solvant, tel qu'un solvant organique halogéné ; (ii) à une température appropriée, tel qu'entre -10 °C et 40 °C ; et/ou (iii) pendant une durée suffisante, tel que 30 minutes à 12 heures.

12. Procédé de l'une quelconque des revendications 1 à 11, après l'étape d) le procédé comprenant en outre la cristallisation du composé de formule (I) ou d'un solvate ou sel de celui-ci, de préférence à partir d'un solvant alcoolique.

13. Procédé de l'une quelconque des revendications 1 à 12, dans lequel une ou plusieurs des étapes a) à d) sont effectuées sans chromatographie.

14. Composé choisi parmi le groupe constitué de : 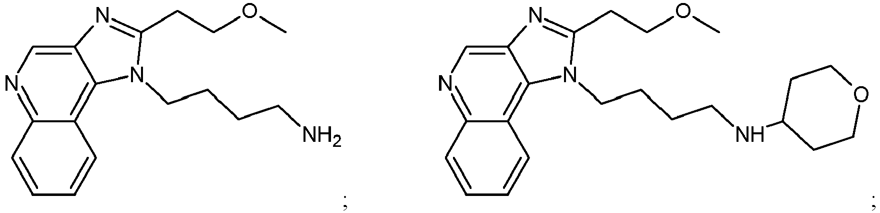 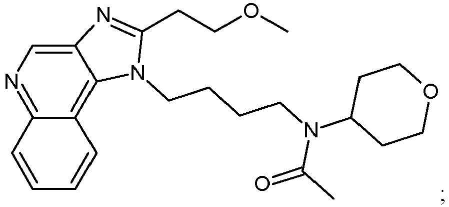 et un solvate ou sel de celui-ci.

15. Utilisation d'un composé de la revendication 14 pour synthétiser un composé de formule (I) tel que défini dans la revendication 1.
